# EUROPEAN PATENT SPECIFICATION

(11) **EP 3 609 535 B1**
(45) Date of publication and mention of the grant of the patent: **25.06.2025**
(21) Application number: 18719248.9
(22) Date of filing: 10.04.2018
(51) Int. Cl.: A61K 39/29, A61K 39/00

(54) **HBV VACCINE**
HBV-IMPFSTOFF
VACCIN CONTRE VHB

(30) Priority: 10.04.2017 GB 201705765
(43) Date of publication of application: 19.02.2020
(73) Proprietor: Oxford University Innovation Ltd., Oxford, Oxfordshire OX2 0JB (GB)
(72) Inventor: BARNES, Eleanor, Oxford Oxfordshire OX2 0JB (GB); CHINNAKANNAN, Senthil, Oxford Oxfordshire OX2 0JB (GB)
(74) Representative: Barker Brettell LLP
(86) International application number: PCT/GB2018/050948
(87) International publication number: WO 2018/189522

(56) References cited:
- WO-A1-2008/020656
- WO-A1-2012/109404
- WO-A1-2013/007772
- WO-A2-2011/015656
- PERRINE MARTIN ET AL: "TG1050, an immunotherapeutic to treat chronic hepatitis B, induces robust T cells and exerts an antiviral effect in HBV-persistent mice", GUT, vol. 64, no. 12, 26 November 2014 (2014-11-26), UK, pages 1961 - 1971, XP055453477, ISSN: 0017-5749, DOI: 10.1136/gutjnl-2014-308041
- SARAH KUTSCHER ET AL: "Design of therapeutic vaccines: hepatitis B as an example", MICROBIAL BIOTECHNOLOGY, vol. 5, no. 2, 29 September 2011 (2011-09-29), pages 270 - 282, XP055217521, ISSN: 1751-7915, DOI: 10.1111/j.1751-7915.2011.00303.x

## Description

This invention relates to multi-antigen HBV immunogen for viral vectored vaccines for therapeutic vaccination of chronic hepatitis B.

Hepatitis B is a viral infection that attacks the liver and can cause both acute and chronic disease. The Hepatitis B virus (HBV) is transmitted through contact with the blood or other body fluids of an infected person. There is no specific treatment for acute hepatitis B. Therefore, care is aimed at maintaining comfort and adequate nutritional balance, including replacement of fluids lost from vomiting and diarrhoea. However, the mainstay of tackling acute Hepatitis B is by prevention using vaccination. Once established, chronic hepatitis B infection can be treated with drugs, including oral antiviral agents. Treatment can slow the progression of cirrhosis, reduce incidence of liver cancer and improve long-term survival. However, once chronic infection is established spontaneous viral control is rare. A major goal therefore is to develop immunotherapeutic HBV vaccines to induce viral control or cure.

Currently, there are few different T-cell inducing vaccines in Phase I and Phase II clinical trials, all of which are based on either one or two full length HBV-antigens or multiple truncated or full length chimeric HBV-antigens or synthetic peptides based on the HBV-proteome. GS-4774, by GlobeImmune is currently in Phase II clinical trial (GS-4774 has heat-inactivated yeast cells expressing a chimera of HBx HBsAg and HBcAg. TG1050, by Transgene is currently in Phase I clinical trial (TG1050 is a human adenovirus serotype 5 based vaccine encoding a chimera of truncated version of three HBV antigens, core-Polymerase-Envelope). HepTcellTM, by Altimmune is currently in Phase I clinical trial (HepTcell is a completely synthetic peptide product, based on 9 synthetic 32-40 mer peptides derived from conserved regions of HBV protein). INO-1800, by Inovio is currently in Phase I clinical trial (INO-1800 is a multi-antigen SynCon^{®} DNA immunotherapy targeting hepatitis B virus clades A & C surface antigens & HBV core antigens). Currently available prophylactic HBV vaccines (generally based on HBV protein vaccines) have no therapeutic effect on chronically infected HBV individuals and hence they fail to control chronic HBV infection.

Therefore, an aim of the present invention is to provide an improved vaccine for HBV infection.

According to a first aspect of the invention, there is provided a multi-HBV immunogen viral vector vaccine in accordance with claim 1 herein.

Described is a multi-HBV immunogen viral vector vaccine comprising:
a viral vector comprising an immunogen expression cassette, wherein the expression of a protein encoded by the expression cassette is arranged to be driven by a promoter, wherein the immunogen expression cassette encodes:
   a) HBV Core;
   b) a modified HBV polymerase (Pₘᵤₜ), wherein the modification is a mutation to wild-type HBV polymerase to substantially remove polymerase function;
   c) HBV surface antigen (HbsAg); and
   d) an intergenic sequence that is arranged to cause expression of at least the HBV surface antigen (HbsAg) as a separate protein from the HBV core and the modified HBV polymerase (Pₘᵤₜ),
wherein the intergenic sequence is downstream (3') of the sequences encoding the HBV core and the modified HBV polymerase (Pₘᵤₜ) and upstream (5') of the sequence encoding the HBV surface antigen (HbsAg).

The intergenic sequence may comprise a cleavage domain, an IRES (Internal Ribosomal Entry Site), a splicing signal, or a secondary promoter. In one embodiment, the intergenic sequence comprises a cleavage domain comprising a sequence arranged to cause ribosome skipping. In another embodiment, the intergenic sequence comprises a secondary promoter to promote expression of at least the surface antigen (HbsAg).

Induction of a strong, multi-antigen specific T cell response against different HBV proteins is thought to play a major role in viral clearance of a resolving chronic HBV infection. For induction of a multi-antigen T-cell response, it can be important to have maximal number of T-cell epitopes, which requires encoding full-length HBV proteins within a T-cell inducing HBV vaccine. Some of the current HBV vaccines under development do not encode full-length HBV antigens. The described vaccine can encode 3 full-length proteins of HBV (namely the Core, optionally with Pre-Core region, Polymerase and Surface proteins). In addition, the vaccine design utilizes an at least two-protein expression, for example using an F2A peptide cleavage strategy to encode a separate surface protein, which in addition to the induction of T-cell response could also induce an antibody response that could possibly have a role in potentiating the therapeutic effect of T-cell vaccine, with possible help in clearance of the virus within a chronically infected individual. Inducing antibodies based on a mammalian system (compared to other systems, for e.g. Yeast) has a selective advantage of providing the mammalian-type glycosylation that could induce antibodies appropriate for the natural host.

The description herein advantageously provides a single vaccine encoding full-length multiple-HBV antigens that could induce broad T-cell response and in-addition could also induce antibodies to the surface protein. The new HBV immunogen at least encompasses three full length HBV-antigens (namely the Core, Polymerase and Surface) and encoded them into a viral vector, such as the Chimpanzee adeno and MVA viral vectors. In addition, the HBV-polymerase protein in the immunogen is provided with mutations that abolish its function, which avoids its participation in HBV genome replication and improves safety for the vaccine.

By using a peptide cleavage strategy, for example by using a Furin-2A cleavage sequence, or a secondary promoter, the transgene cassette can generate at least two proteins, a fused core and polymerase protein and a separate surface protein, both of which generates a T-cell immune response. The encoded surface protein, in addition to the generation of T-cell immune response can also generate an antibody response.

### Immunogen expression cassette

The skilled person will recognize that the cleavage domain may not lead to cleavage on all occasions, for example F2A based ribosomal skipping events may occur in a small proportion of expressions, such that a small proportion of proteins made will be a fusion of all the HBV proteins/antigens that would otherwise be separated by the cleavage. Therefore, in one embodiment, the immunogen expression cassette may further encode a fusion protein comprising at least the HBV Core, modified HBV polymerase (Pmut) and HBV surface antigen.

In one embodiment, the immunogen expression cassette further encodes HBV Pre-Core (PreC). In another embodiment, the immunogen expression cassette further encodes HBV PreS1, and/or a truncated form thereof. In another embodiment, the immunogen expression cassette further encodes HBV PreS2.

In one embodiment, the immunogen expression cassette further encodes HBV Pre-Core (PreC) and HBV PreS1, or a truncated form of HBV PreS1. In another embodiment, the immunogen expression cassette further encodes HBV Pre-Core (PreC) and HBV PreS2. In one embodiment, the immunogen expression cassette further encodes HBV Pre-Core (PreC) and HBV PreS1, and a truncated form of PreS1. In one embodiment, the immunogen expression cassette further encodes HBV Pre-Core (PreC), HBV PreS1, and/or a truncated form thereof, and HBV PreS2.

In an embodiment wherein the HBV Pre-core and Core are encoded, the immunogen expression cassette may be capable of expressing the HBV e-Antigen. The skilled person in the art will recognise that HBV e-Antigen comprises or consists of the 10 C-terminal amino acids of Pre-Core and the 149 N-terminal amino acids of the Core. For the expression of HBV e-Antigen, the Pre-Core and Core are expressed together and this whole expressed protein undergoes N-terminal and C-terminal cleavage.

In one embodiment, the HBV Core and modified polymerase (Pmut) are arranged to be expressed as a fusion protein. In an embodiment wherein the immunogen expression cassette encodes HBV Pre-core, the HBV Pre-core, HBV Core and modified polymerase (Pmut) are arranged to be expressed as a fusion protein.

In one embodiment the immunogen expression cassette encodes at least two proteins, comprising a first fusion protein comprising at least the HBV Core and the modified HBV polymerase (Pmut), and a second protein comprising at least the HBV surface antigen (HbsAg). In another embodiment the immunogen expression cassette encodes only two proteins, comprising a first fusion protein comprising at least the HBV Core and the modified HBV polymerase (Pmut), and a second protein comprising at least the HBV surface antigen (HbsAg).

In one embodiment, the immunogen expression cassette does not encode HBV X protein.

In one embodiment, the immunogen expression cassette may comprise a nucleic acid sequence encoding any one of:
SIi-HBV-CPmutS;
SIi-HBV-SCPmut;
HBV-CPmutS
SIi-HBV-CPmutPreS-S(sh);
SIi-HBV-CPmutPreS-TPA-S(sh);
SIi-HBV-PreS-Pmut-C;
MVA-SIi-HBV-PreS-Pmut-C-S(sh); or
MVA-SIi-HBV-PreS-Pmut-C-TPA-S(sh), as described herein.

In one embodiment, the immunogen expression cassette comprises SEQ ID NO: 46 (SIi-HBV-CPmutS) or a variant thereof. A variant of SEQ ID NO: 46 may comprise a sequence having at least 70%, 75%, 80%, 85%, 90%, 95%, 98%, 99%, or 99.5% identity with SEQ ID NO: 46. The variant of SEQ ID NO: 46 may encode protein that substantially retains the immunogenicity of equivalent protein encoded by SEQ ID NO: 46.

In one embodiment, the immunogen expression cassette comprises SEQ ID NO: 47 (SIi-HBV-SCPmut) or a variant thereof. A variant of SEQ ID NO: 47 may comprise a sequence having at least 70%, 75%, 80%, 85%, 90%, 95%, 98%, 99%, or 99.5% identity with SEQ ID NO: 47. The variant of SEQ ID NO: 47 may encode protein that substantially retains the immunogenicity of equivalent protein encoded by SEQ ID NO: 47.

In another embodiment, the immunogen expression cassette comprises SEQ ID NO: 48 (SIi-HBV-CPmutPreS-S(sh)) or a variant thereof. A variant of SEQ ID NO: 48 may comprise a sequence having at least 70%, 75%, 80%, 85%, 90%, 95%, 98%, 99%, or 99.5% identity with SEQ ID NO: 48. The variant of SEQ ID NO: 48 may encode protein that substantially retains the immunogenicity of equivalent protein encoded by SEQ ID NO: 48.

In another embodiment, the immunogen expression cassette comprises SEQ ID NO: 49 (SIi-HBV-CPmutPreS-TPA-S(sh)) or a variant thereof. A variant of SEQ ID NO: 49 may comprise a sequence having at least 70%, 75%, 80%, 85%, 90%, 95%, 98%, 99%, or 99.5% identity with SEQ ID NO: 49. The variant of SEQ ID NO: 49 may encode protein that substantially retains the immunogenicity of equivalent protein encoded by SEQ ID NO: 49.

In one embodiment, the immunogen expression cassette comprises SEQ ID NO: 59 (SIi-HBV-PreS-Pmut-C) or a variant thereof. A variant of SEQ ID NO: 59 may comprise a sequence having at least 70%, 75%, 80%, 85%, 90%, 95%, 98%, 99%, or 99.5% identity with SEQ ID NO: 59. The variant of SEQ ID NO: 59 may encode protein that substantially retains the immunogenicity of equivalent protein encoded by SEQ ID NO: 59.

In one embodiment, the immunogen expression cassette comprises SEQ ID NO: 24 (MVA-SIi-HBV-PreS-Pmut-C-S(sh)) or a variant thereof. A variant of SEQ ID NO: 24 may comprise a sequence having at least 70%, 75%, 80%, 85%, 90%, 95%, 98%, 99%, or 99.5% identity with SEQ ID NO: 24. The variant of SEQ ID NO: 24 may encode protein that substantially retains the immunogenicity of equivalent protein encoded by SEQ ID NO: 24.

In one embodiment, the immunogen expression cassette comprises SEQ ID NO: 27 (MVA-SIi-HBV-PreS-Pmut-C-TPA-S(sh)) or a variant thereof. A variant of SEQ ID NO: 27 may comprise a sequence having at least 70%, 75%, 80%, 85%, 90%, 95%, 98%, 99%, or 99.5% identity with SEQ ID NO: 27. The variant of SEQ ID NO: 27 may encode protein that substantially retains the immunogenicity of equivalent protein encoded by SEQ ID NO: 27. In one embodiment, the immunogen expression cassette comprises SEQ ID NO: 58 (MVA-SIi-HBV-PreS-Pmut-C-TPA-S(sh)) or a variant thereof. A variant of SEQ ID NO: 58 may comprise a sequence having at least 70%, 75%, 80%, 85%, 90%, 95%, 98%, 99%, or 99.5% identity with SEQ ID NO: 58. The variant of SEQ ID NO: 58 may encode protein that substantially retains the immunogenicity of equivalent protein encoded by SEQ ID NO: 58.

### Viral vector

The viral vector may comprise a virus. The immunogen expression cassette sequence of the description may be cloned into any suitable viral vector that is known to elicit good immune response. Suitable viral vectors have been described in Dicks et al (Vaccine. 2015 Feb 25;33(9):1121-8. doi: 10.1016/j.vaccine.2015.01.042. Epub 2015 Jan 25), Antrobus et al (Mol Ther. 2014 Mar;22(3):668-74. doi: 10.1038/mt.2013.284. Epub 2013 Dec 30.), and (Warimwe et al. (Virol J. 2013 Dec 5;10:349. doi: 10.1186/1743-422X-10-349).

The viral vector may be an attenuated viral vector. The viral vector may comprise an adenovirus, such as a human or simian adenovirus. In one embodiment, the viral vector comprises an adenovirus, such as a group E simian adenovirus, when used in a prime vaccine of a prime boost regime. The viral vector may comprise a group E simian adenovirus. The viral vector may comprise ChAdOx1 (a group E simian adenovirus, like the AdCh63 vector used safely in malaria trials) or ChAdOx2. The skilled person will be familiar with ChAdOx1 based viral vectors, for example from patent publication WO2012172277. The viral vector may comprise AdCh63. The viral vector may comprise AdC3 or AdH6. In one embodiment, the viral vector is a human serotype. In another embodiment, the viral vector comprises Modified Vaccinia Ankara (MVA). The viral vector may comprise MVA when used as a vaccine boost in a prime boost regime. In one embodiment, the viral vector comprises an adenovirus, such as a group E simian adenovirus, when used in a prime vaccine of a prime boost regime, and may comprise MVA when used as a vaccine boost in a prime boost regime. The skilled person will be familiar with MVA based viral vectors, for example from US patent publication US9273327.

MVA advantageously allows expression of more than one protein using different pox viral promoters.

In an alternative embodiment, the viral vector may comprise Adeno-associated virus (AAV) or *Lentivirus.* In another embodiment, the viral vector may comprise any of Vaccinia virus, fowlpox virus or canarypox virus (e.g. members of *Poxviridae* and the genus *Avipoxvirus*), or New York attenuated vaccinia virus (Tartaglia et al. Virology. 1992 May;188(1):217-32). In another embodiment, the viral vector may comprise any of Herpes simplex virus, Human Cytomegalo virus, Measles virus (MeV), Sendai virus (SeV), *Flavivirus* (e.g. Yellow Fever Virus - 17D), or *alphavirus* vectors, such as Sindibis virus (SINV), Venezuelan equine encephalitis virus, or Semliki forest virus.

In one embodiment, the viral vector may comprise nucleic acid comprising the sequence of SEQ ID NO: 39 and 40 (ChAdOx1) or a variant thereof. A variant of SEQ ID NO: 39 and 40 may comprise a sequence having at least 70%, 75%, 80%, 85%, 90%, 95%, 98%, 99%, or 99.5% identity with SEQ ID NO: 39 and 40. The variant of SEQ ID NO: 39 and 40 may encode a viral vector that substantially retains the function of the viral vector of SEQ ID NO: 39 and 40 (ChAdOx1).

In one embodiment, the viral vector may comprise nucleic acid comprising the sequence of SEQ ID NO: 41 and 42 (ChAdOx2) or a variant thereof. A variant of SEQ ID NO: 41 and 42 may comprise a sequence having at least 70%, 75%, 80%, 85%, 90%, 95%, 98%, 99%, or 99.5% identity with SEQ ID NO: 41 and 42. The variant of SEQ ID NO: 41 and 42 may encode a viral vector that substantially retains the function of the viral vector of SEQ ID NO: 41 and 42 (ChAdOx2).

In one embodiment, the viral vector may comprise nucleic acid comprising the sequence of SEQ ID NO: 44 and 45 (MVA) or a variant thereof. A variant of SEQ ID NO: 44 and 45 may comprise a sequence having at least 70%, 75%, 80%, 85%, 90%, 95%, 98%, 99%, or 99.5% identity with SEQ ID NO: 44 and 45. The variant of SEQ ID NO: 44 and 45 may encode a viral vector that substantially retains the function of the viral vector of SEQ ID NO: 44 and 45 (MVA).

In one embodiment, the viral vector encodes any one of:
SIi-HBV-CPmutS;
SIi-HBV-SCPmut;
SIi-HBV-CPmutPreS-S(sh);
SIi-HBV-CPmutPreS-TPA-S(sh);
MVA-SIi-HBV-PreS-Pmut-C-S(sh); or
MVA-SIi-HBV-PreS-Pmut-C-TPA-S(sh), as described herein.

In one embodiment, the viral vector encodes SEQ ID NO: 3 (SIi-HBV-CPmutS) or a variant thereof. A variant of SEQ ID NO: 3 may comprise a sequence having at least 70%, 75%, 80%, 85%, 90%, 95%, 98%, 99%, or 99.5% identity with SEQ ID NO: 3. The variant of SEQ ID NO: 3 may substantially retain the immunogenicity of SEQ ID NO: 3.

In one embodiment, the viral vector encodes SEQ ID NO: 11 (SIi-HBV-SCPmut) or a variant thereof. A variant of SEQ ID NO: 11 may comprise a sequence having at least 70%, 75%, 80%, 85%, 90%, 95%, 98%, 99%, or 99.5% identity with SEQ ID NO: 11. The variant of SEQ ID NO: 11 may substantially retain the immunogenicity of SEQ ID NO: 11.

In one embodiment, the viral vector encodes SEQ ID NO: 13 (SIi-HBV-CPmutPreS-S(sh)) or a variant thereof. A variant of SEQ ID NO: 13 may comprise a sequence having at least 70%, 75%, 80%, 85%, 90%, 95%, 98%, 99%, or 99.5% identity with SEQ ID NO: 13. The variant of SEQ ID NO: 13 may substantially retain the immunogenicity of SEQ ID NO: 13.

In one embodiment, the viral vector encodes SEQ ID NO: 25 (SIi-HBV-CPmutPreS-TPA-S(sh)) or a variant thereof. A variant of SEQ ID NO: 25 may comprise a sequence having at least 70%, 75%, 80%, 85%, 90%, 95%, 98%, 99%, or 99.5% identity with SEQ ID NO: 25. The variant of SEQ ID NO: 25 may substantially retain the immunogenicity of SEQ ID NO: 25.

In one embodiment, the viral vector encodes SEQ ID NO: 23 (MVA-SIi-HBV-PreS-Pmut-C-S(sh)) or a variant thereof. A variant of SEQ ID NO: 23 may comprise a sequence having at least 70%, 75%, 80%, 85%, 90%, 95%, 98%, 99%, or 99.5% identity with SEQ ID NO: 23. The variant of SEQ ID NO: 23 may substantially retain the immunogenicity of SEQ ID NO: 23.

In one embodiment, the viral vector encodes SEQ ID NO: 26 (MVA-SIi-HBV-PreS-Pmut-C-TPA-S(sh)) or a variant thereof. A variant of SEQ ID NO: 26 may comprise a sequence having at least 70%, 75%, 80%, 85%, 90%, 95%, 98%, 99%, or 99.5% identity with SEQ ID NO: 26. The variant of SEQ ID NO: 26 may substantially retain the immunogenicity of SEQ ID NO: 26.

In one embodiment, the viral vector comprises the nucleic acid sequence of SEQ ID NO: 46 (SIi-HBV-CPmutS) or a variant thereof. A variant of SEQ ID NO: 46 may comprise a sequence having at least 70%, 75%, 80%, 85%, 90%, 95%, 98%, 99%, or 99.5% identity with SEQ ID NO: 46.

In one embodiment, the viral vector comprises the nucleic acid sequence of SEQ ID NO: 47 (SIi-HBV-SCPmut) or a variant thereof. A variant of SEQ ID NO: 47 may comprise a sequence having at least 70%, 75%, 80%, 85%, 90%, 95%, 98%, 99%, or 99.5% identity with SEQ ID NO: 47.

In one embodiment, the viral vector comprises the nucleic acid sequence of SEQ ID NO: 48 (SIi-HBV-CPmutPreS-S(sh)) or a variant thereof. A variant of SEQ ID NO: 48 may comprise a sequence having at least 70%, 75%, 80%, 85%, 90%, 95%, 98%, 99%, or 99.5% identity with SEQ ID NO: 48.

In one embodiment, the viral vector comprises the nucleic acid sequence of SEQ ID NO: 49 (SIi-HBV-CPmutPreS-TPA-S(sh)) or a variant thereof. A variant of SEQ ID NO: 49 may comprise a sequence having at least 70%, 75%, 80%, 85%, 90%, 95%, 98%, 99%, or 99.5% identity with SEQ ID NO: 49.

In one embodiment, the viral vector comprises the nucleic acid sequence of SEQ ID NO: 24 (MVA-SIi-HBV-PreS-Pmut-C-S(sh)) or a variant thereof. A variant of SEQ ID NO: 24 may comprise a sequence having at least 70%, 75%, 80%, 85%, 90%, 95%, 98%, 99%, or 99.5% identity with SEQ ID NO: 24.

In one embodiment, the viral vector comprises the nucleic acid sequence of SEQ ID NO: 27 (MVA-SIi-HBV-PreS-Pmut-C-TPA-S(sh)) or a variant thereof. A variant of SEQ ID NO: 27 may comprise a sequence having at least 70%, 75%, 80%, 85%, 90%, 95%, 98%, 99%, or 99.5% identity with SEQ ID NO: 27. In one embodiment, the viral vector comprises the nucleic acid sequence of SEQ ID NO: 58 (MVA-SIi-HBV-PreS-Pmut-C-TPA-S(sh)) or a variant thereof. A variant of SEQ ID NO: 58 may comprise a sequence having at least 70%, 75%, 80%, 85%, 90%, 95%, 98%, 99%, or 99.5% identity with SEQ ID NO: 58.

### Promoter

In one embodiment, the promoter is encoded in the immunogen expression cassette, for example the promoter may be encoded at, or adjacent to, the 5' end of the immunogen expression cassette. Alternatively, the promoter may be encoded as part of the viral vector nucleic acid outside of the immunogen expression cassette. For example the promoter may be encoded upstream (5') of the immunogen expression cassette.

The promoter may promote the expression of all the encoded protein of the immunogen expression cassette. In an alternative embodiment wherein the immunogen expression cassette comprises a secondary promoter, the promoter may be a primary promoter that is arranged to promote expression of at least the HBV core and modified polymerase (Pmut), and optionally not the HBV surface antigen (HbsAg) which may be arranged to be promoted separately by the secondary promoter.

The skilled person will recognise that any suitable promoter may be used as the primary and/or secondary promoter as appropriate for the host for expression. In one embodiment, the promoter comprises a CMV promoter. The CMV promoter may comprise the long or short CMV promoter. In an embodiment wherein the viral vector comprises an Adenoviral vector, such as ChAdOx1 or 2, the promoter may comprise a CMV promoter, SV40 promoter, or EFla promoter. In an embodiment wherein the viral vector comprises an Adenoviral vector, such as ChAdOx1 or 2, the promoter may comprise a CMV promoter.

The promoter element(s) used in the vector, for example adenoviral vector, (such as ChAdOx1 or 2) may comprise a tetracycline operator (*tetO*) sequence. A tetracycline operator (*tetO*) sequence is helpful for generation of viral vectors that can express foreign proteins that are toxic to cells within which they are generated.

In another embodiment, the promoter comprises a pox viral promoter. In an embodiment wherein the viral vector comprises MVA, the promoter may comprise a pox viral promoter, such as F11. In one embodiment, the promoter comprises early F11 promoter. Alternatively, the pox viral promotor may comprise an early promoter, for example selected from any of B8R, K6L, A44L, C11R, and B2R, a promoter with early and late activity, for example selected from mH5, p7.5, and SSP, or a late promoter, for example FP4b.

Early promoter based transgene expression is can be useful for an immunogen intended primarily for T-cell response (there is a higher magnitude of T-cell induction upon usage of these early promoters). However promoters with both early and late activity can be used for an immunogen intended for inducing antibody response. However, these early and late activity promoters can also be used for an immunogen intended for T-cell induction.

In one embodiment, the promoter comprises or consists of the nucleic acid sequence of SEQ ID NO: 50 or 52 (CMV long or short promoter) or variants thereof. A variant of SEQ ID NO: 50 or 52 may comprise a sequence having at least 70%, 75%, 80%, 85%, 90%, 95%, 98%, 99%, or 99.5% identity with SEQ ID NO: 50 or 52 respectively. The variant may substantially retain the promoter function of SEQ ID NO: 50 or 52).

In one embodiment, the promoter comprises or consists of a sequence located in the F11 Left flank sequence (SEQ ID NO: 35) or a variant thereof. A variant may comprise a sequence having at least 70%, 75%, 80%, 85%, 90%, 95%, 98%, 99%, or 99.5% identity with the promoter sequence located in the F11 Left flank sequence (SEQ ID NO: 35). The variant may substantially retain the promoter function of the promoter located in the F11 Left flank sequence (SEQ ID NO: 35).

### Secondary Promoter

In one embodiment, the immunogen expression cassette encodes a secondary promoter. In an embodiment wherein a secondary promoter is provided in addition to the first/primary promoter, the secondary promoter may be encoded by/within the immunogen expression cassette. The secondary promoter may be encoded 3' of the first/primary promoter. The secondary promoter may be encoded 3' of the first protein/antigen to be expressed, for example downstream of the HBV core and HBV modified polymerase and upstream of the HBV surface antigen. The secondary promoter may promote the expression of at least the HBV surface antigen.

The secondary promoter may comprise a pox viral promoter. The secondary promoter may comprise pox viral early promoters, such as any of B8R, K6L, A44L, C11R, and B2R, or pox viral promoters with early and late activity, such as mH5, p7.5 or SSP, or a late promoter such as FP4b.

In one embodiment, the secondary promoter comprises early/late promoter mH5. In one embodiment, the secondary promoter comprises or consists of the nucleic acid sequence of SEQ ID NO: 28 (mH5 promoter) or a variant thereof. A variant of SEQ ID NO: 28 may comprise a sequence having at least 70%, 75%, 80%, 85%, 90%, 95%, 98%, 99%, or 99.5% identity with SEQ ID NO: 28. The variant may substantially retain the secondary promoter function of SEQ ID NO: 28).

In another embodiment, the secondary promoter may comprise a SV40 promoter or EF1a promoter.

### Cleavage domain

In one embodiment, the immunogen expression cassette encodes a cleavage domain. In one embodiment, the cleavage domain is a post-translation proteolytic cleavage domain, which allows for the cleavage of a translated protein, for example by a proteolytic cleavage enzyme. The cleavage enzyme may be provided by the host, for example the host being vaccinated, such as a human. In another embodiment, the cleavage enzyme may be encoded in the immunogen expression cassette or viral vector. In one embodiment, the cleavage domain comprises a non-HBV sequence, for example, a mammalian sequence. In one embodiment, the cleavage domain comprises a human derived sequence.

In one embodiment, the cleavage domain comprises a ribosome skipping cleavage domain. In one embodiment, the cleavage domain comprises a Furin recognition site comprising or consisting of the sequence RXRR, where X could be any amino acid. In one embodiment, the cleavage domain comprises a Furin recognition site and a 2A peptide sequence. The 2A peptide sequence may comprise FMDV (Foot and Mouth Disease Virus) 2A peptide sequence (APVKQTLNFDLLKLAGDVESNPGP - SEQ ID NO: 43). Alternatively, 2A peptides may be selected from P2A (porcine teschovirus-1 2A), T2A (Thoseaasigna virus 2A), and E2A (equine rhinitis A virus [ERAV] 2A). Any picoma virus 2A peptide sequence may be provided for, and function as, a peptide cleavage site. Therefore, the cleavage domain may comprise a sequence encoding picorna virus 2A peptide sequence.

In one embodiment, the cleavage domain comprises or consists of Furin-2A (F2A) peptide sequence or a functional variant thereof. The cleavage domain may comprise or consists of the sequence of SEQ ID NO: 9 or a variant thereof. A variant of SEQ ID NO: 9 may comprise a sequence having at least 70%, 75%, 80%, 85%, 90%, 95%, 98%, 99%, or 99.5% identity with SEQ ID NO: 9. The variant of SEQ ID NO: 9 may substantially retain the cleavage function of SEQ ID NO: 9.

F2A is advantageously short (only 28 amino acids), which helps to provide appropriate inserts and express more than one protein from vectors that can have a size limit, such as Adenovirus based vectors, including ChAdOx1/2.

In an embodiment where the viral vector is MVA, or otherwise a viral vector is used as a boost vaccination following a prime vaccination, a different cleavage domain may be provided to avoid any potential boosting of T-cell responses to the cleavage domain of the prime vaccination. Alternatively a secondary promoter may be used instead of a cleavage domain in order to avoid any potential boosting of T-cell responses to a cleavage domain of a prime vaccination.

### IRES (Internal Ribosomal Entry Site)

In one embodiment, the immunogen expression cassette encodes an Internal Ribosome Entry Site (IRES). The skilled person will recognise that an IRES is an RNA structures that allows cap independent initiation of translation, and is able to initiate translation in the middle of a messenger RNA.

### Splicing Signal

In one embodiment, the immunogen expression cassette encodes an mRNA splicing signal. The skilled person will be familiar with commonly used splicing signals that are appropriate for this use. One such example of an mRNA splicing signal that may be used is a chimeric intron composed of the 5' donor site from the first intron of the human β-globin gene and the branch and 3' acceptor site from the intron of an immunoglobulin gene heavy chain variable region in the expression vector pCI-neo Mammalian Expression Vector (Promega).

### HBV Pre-Core (PreC)

The HBV PreC may comprise or consist of a full length wild-type HBV PreC sequence, or a variant thereof. The HBV PreC variant may comprise or consist of a truncated HBV PreC sequence.

The HBV PreC may comprise or consist of the sequence of SEQ ID NO: 16 or a variant thereof. A variant of SEQ ID NO: 16 may comprise a sequence having at least 70%, 75%, 80%, 85%, 90%, 95%, 98%, 99%, or 99.5% identity with SEQ ID NO: 16. The variant of SEQ ID NO: 16 may substantially retain the immunogenicity of SEQ ID NO: 16. The variant of SEQ ID NO: 16 may substantially retain the tertiary structure of SEQ ID NO: 16.

### HBV Core

The HBV Core may comprise or consist of a full length wild-type HBV Core sequence, or a variant thereof. The HBV Core variant may comprise or consist of a truncated HBV Core sequence. The HBV Core may not comprise HBV Pre-Core.

The HBV Core may comprise or consist of the sequence of SEQ ID NO: 6 or a variant thereof. A variant of SEQ ID NO: 6 may comprise a sequence having at least 70%, 75%, 80%, 85%, 90%, 95%, 98%, 99%, or 99.5% identity with SEQ ID NO: 6. The variant of SEQ ID NO: 6 may substantially retain the immunogenicity of SEQ ID NO: 6. The variant of SEQ ID NO: 6 may substantially retain the tertiary structure of SEQ ID NO: 6.

### HBV e-Antigen (HBeAg)

The HBV e-Antigen (HBeAg) may comprise or consist of a full length wild-type HBV e-Antigen sequence, or a variant thereof. The HBV e-Antigen variant may comprise or consist of a truncated HBV e-Antigen sequence.

The HBV e-Antigen (HBeAg) may comprise or consist of the sequence:

SKLCLGWLWGMDIDPYKEFGASVELLSFLPSDFFPSIRDLLDTASALYREALESPE
HCSPHHTALRQAILCWGELMNLATWVGSNLEDPASRELVVSYVNVNMGLKIRQ

LLWFHISCLTFGRETVLEYLVSFGVWIRTPPAYRPPNAPILSTLPETTVV (SEQ ID
NO: 17).

The HBV e-Antigen (HBeAg) may comprise or consist of the sequence of SEQ ID NO: 17 or a variant thereof. A variant of SEQ ID NO: 17 may comprise a sequence having at least 70%, 75%, 80%, 85%, 90%, 95%, 98%, 99%, or 99.5% identity with SEQ ID NO: 17. The variant of SEQ ID NO: 17 may substantially retain the immunogenicity of SEQ ID NO: 17. The variant of SEQ ID NO: 17 may substantially retain the tertiary structure of SEQ ID NO: 17.

### Modified HBV polymerase

The modified HBV polymerase (Pₘᵤₜ) may comprise or consist of a truncated HBV polymerase. In particular, the mutation to wild-type HBV polymerase to substantially remove polymerase function may comprise a sequence encoding a truncated HBV polymerase. Alternatively or additionally, the mutation comprises one or more point mutations to the encoded HBV polymerase sequence. The modification may comprise one or more amino acid substitutions, deletions or additions in the encoded HBV polymerase sequence. In one embodiment, the modified HBV polymerase (Pmut) is not a truncated form of HBV polymerase (i.e. it is full length relative to wildtype HBV polymerase).

The modified HBV polymerase (Pₘᵤₜ) may comprise or consist of the sequence of SEQ ID NO: 8 or a variant thereof. A variant of SEQ ID NO: 8 may comprise a sequence having at least 70%, 75%, 80%, 85%, 90%, 95%, 98%, 99%, or 99.5% identity with SEQ ID NO: 8. The variant of SEQ ID NO: 8 may substantially retain the immunogenicity of SEQ ID NO: 8. The variant of SEQ ID NO: 8 may substantially retain the tertiary structure of SEQ ID NO: 8.

The modification may be a mutation that prevents protein priming. Additionally or alternatively mutations may be provided in the reverse transcriptase and/or RNAase domains to prevent their function. Further additionally or alternatively the mutation may be structural such that is disrupts the correct protein folding of the polymerase. The modifications may comprise one or more, or all of Y63, C323, C334, C338, C352, R714, D788, R792, or equivalent residues thereof, by reference to wild-type HBV polymerase consensus sequence herein (i.e. SEQ ID NO: 19). In one embodiment the modifications may comprise one or more, or all of Y63A, C323A, C334A, C338A, C352A, R714A, D788A, R792A, or equivalent residues thereof, by reference to wild-type HBV polymerase consensus sequence herein (i.e. SEQ ID NO: 19). In another embodiment, the modifications may comprise one or more, or all of R714, D788, and R792, or equivalent residues thereof, by reference to wild-type HBV polymerase consensus sequence herein (i.e. SEQ ID NO: 19). In one embodiment the modifications may comprise one or more, or all of R714A, D788A, and R792A, or equivalent residues thereof, by reference to wild-type HBV polymerase consensus sequence herein (i.e. SEQ ID NO: 19). Reference to equivalent residues is understood to mean that the HBV polymerase may be based on an alternative sequence than the HBV polymerase sequence provided herein, and that numbering or identity of amino acid residues may differ between the sequences. Such differences and equivalency may be readily determined by an alignment of the HBV polymerase sequence with the wild-type HBV polymerase consensus sequence herein (i.e. SEQ ID NO: 19).

Mutations R714A, D788A, R792A advantageously stop polymerase function. However, one or more, or all of, additional mutations of Y63 and other cysteine mutations of C323, C334, C338, and C352 may be added as an extra measure in the event of a reversion within those functional mutations. The additional mutations may comprise Y63A, C323A, C334A, C338A, and/or C352A.

The mutations advantageously provide that the HBV polymerase function is disrupted by (a) RNAase H functional mutations, to stop its enzyme activity, and/or (b) Y63A mutation to stop the first step of replication (Priming of DNA synthesis), and/or (c) cysteine mutations to disrupt its native conformation and stop the HBV polymerase from participating in the initial steps of viral replication (protein priming, RNA binding and RNA packaging).

The skilled person will be familiar with various HBV polymerase modifications that can be provided which significantly reduce or ablate function, and can be provided in the modified polymerase according to the description. Such modifications are for example described in WO2016020538, WO2013007772, and WO2011015656.

In one embodiment, the modified polymerase is modified so as to exhibit a reduced reverse-transcriptase (RTase) enzymatic activity with respect to a wild-type HBV polymerase. The reduction of RTase activity may be provided by one or more mutation(s) in the domain responsible for RTase enzymatic activity. Four residues have been found to be involved in RTase activity, forming a motif "YMDD" (for Tyr, Met, Asp and Asp residues) at approximately position 538 to approximately position 541 of a wild-type HBV polymerase. The present description encompasses any mutation(s) in this motif, or elsewhere in the RTase domain, that provides a significant reduction (e.g. at least a 10 fold reduction) or ablation of the RTase activity. Representative examples of suitable RTase-deficient polymerase mutants are described in Radziwill et al. (1990, J. Virol. 64:613), in Bartenschlager et al. (1990, J. Virol. 64:5324) and in Jeong et al. (1996, Biochem Bioph Res Commun. 223(2):264). In one embodiment, the modified polymerase may comprise the substitution of the first Asp residue of the YMDD motif or of the amino acid residue located in an equivalent position in a native HBV polymerase to any amino acid residue other than Asp, with an optional substitution to a His residue (D540H mutation).

Additionally or alternatively, the modified polymerase may be modified to provide a reduced RNase H enzymatic activity with respect to wild-type HBV polymerase. The reduction of RNase H activity may be provided by one or more mutation(s) in the domain responsible for RNase H enzymatic activity. The functional domain involved in RNase H activity is within the C-terminal portion of HBV polymerase, approximately from position 680 to the C-terminal position 832 of wild-type polymerase, and the modified polymerase of the present description may encompasses any mutation(s) in this domain that provides a significant reduction (e.g. at least a 10 fold reduction) or ablation of the RNase H activity. Representative examples of suitable RNase H-deftcient polymerase mutants are described in Radziwill et ai. (1990, J. Virol. 64:613), in Bartenschlager at al. (1990, J. Virol. 64:5324).

### HBV surface antigen (HbsAg)

The skilled person will understand that PreS1 and PreS2 are components of the Large (L) form of HBV surface protein (e.g. L form = PreS1+PreS2+S). The medium (M) form of HBV surface protein has PreS2+S. Having such sequences together means that T-Cell epitopes are included in those sequential order.

The HbsAg may comprise or consist of a full length wild-type HbsAg sequence, or a variant thereof. The HbsAg variant may comprise or consist of a truncated HbsAg sequence.

The HbsAg including the PreS1 and PreS2 sequences may comprise or consist of the sequence of SEQ ID NO: 10 or a variant thereof. A variant of SEQ ID NO: 10 may comprise a sequence having at least 70%, 75%, 80%, 85%, 90%, 95%, 98%, 99%, or 99.5% identity with SEQ ID NO: 10. The variant of SEQ ID NO: 10 may substantially retain the immunogenicity of SEQ ID NO: 10. The variant of SEQ ID NO: 10 may substantially retain the tertiary structure of SEQ ID NO: 10.

In another embodiment, the HbsAg may comprise the surface antigen without PreS1 and/or PreS2. The HbsAg may comprise or consist of the sequence of SEQ ID NO: 18 or a variant thereof. A variant of SEQ ID NO: 18 may comprise a sequence having at least 70%, 75%, 80%, 85%, 90%, 95%, 98%, 99%, or 99.5% identity with SEQ ID NO: 18. The variant of SEQ ID NO: 18 may substantially retain the immunogenicity of SEQ ID NO: 18. The variant of SEQ ID NO: 18 may substantially retain the tertiary structure of SEQ ID NO: 18.

In one embodiment, the HbsAg may comprise or consist of any one or all of the four known transmembrane regions in HbsAg (amino acids 1-226), which are amino acids (8-32) FLGPLLVLQAGFFLLTRILTIPQSL (SEQ ID NO: 54), amino acids (80-98) FIIFLFILLLCLIFLLVLL (SEQ ID NO: 55), amino acids (160-184) RFLWEWASVRFSWLSLLVPFVQWFV (SEQ ID NO: 56), and amino acids (189-210) TVWLSVIWMMWYWGPSLYNILS (SEQ ID NO: 57) respectively. In one embodiment, the HbsAg may at least comprise or consist of the HbsAg transmembrane region of amino acids (8-32) FLGPLLVLQAGFFLLTRILTIPQSL (SEQ ID NO: 54).

### HBV PreS1

The HBV PreS1 may comprise or consist of a full length wild-type HBV PreS1 sequence, or a variant thereof. The HBV PreS1 may comprise or consist of the sequence of SEQ ID NO: 52 or a variant thereof. A variant of SEQ ID NO: 52 may comprise a sequence having at least 70%, 75%, 80%, 85%, 90%, 95%, 98%, 99%, or 99.5% identity with SEQ ID NO: 52. The variant of SEQ ID NO: 52 may substantially retain the immunogenicity of SEQ ID NO: 52. The variant of SEQ ID NO: 52 may substantially retain the tertiary structure of SEQ ID NO: 52.

The HBV PreS1 variant may comprise or consist of a truncated HBV PreS1 sequence, for example CΔPreS1 or NΔPreS1 described herein (CΔPreS1 refers to C-terminal truncated PreS1 and NΔPreS1 refers to N-terminal truncated PreS1). In one embodiment, the immunogen expression cassette may encode both NΔPreS1 and CΔPreS1. In an embodiment wherein the immunogen expression cassette encodes both NΔPreS1 and CΔPreS1, the NΔPreS1 may be encoded upstream (5') of intergenic sequence and the CΔPreS1 may be encoded downstream (3') of intergenic sequence. In an embodiment wherein the immunogen expression cassette encodes NΔPreS1 upstream (5') of intergenic sequence, it may be fused with PreS2 and/or the modified polymerase (Pmut). In an embodiment wherein the immunogen expression cassette encodes NΔPreS1 upstream (5') of intergenic sequence, it may be fused with PreS2 and/or the modified polymerase (Pmut) and the CΔPreS1 encoded downstream (3') of intergenic sequence may be fused with the surface antigen (HbsAg).

### CΔPreS1

In one embodiment, the immunogen expression cassette encodes a truncated form of HBV PreS1. The truncation may comprise a C-terminal truncation. In one embodiment, the truncated HBV PreS1 comprises CΔPreS1 (SEQ ID NO: 21) described herein. In one embodiment, the truncated PreS1, such as CΔPreS1 described herein, is arranged to be expressed a fusion protein with the HBV surface antigen (S / HbsAg). A linker sequence, such as a linker described herein, may be provided between the truncated PreS1 and surface antigen (S / HbsAg). For example, CΔPreS1 + linker + S (described herein as S(sh)). The nucleotide sequence encoding S(sh) may comprise or consist of SEQ ID NO: 61 or SEQ ID NO: 38.

The CΔPreS1 may comprise or consist of the sequence of SEQ ID NO: 21 or a variant thereof. A variant of SEQ ID NO: 21 may comprise a sequence having at least 70%, 75%, 80%, 85%, 90%, 95%, 98%, 99%, or 99.5% identity with SEQ ID NO: 21. The variant of SEQ ID NO: 21 may substantially retain the immunogenicity of SEQ ID NO: 21. The variant of SEQ ID NO: 21 may substantially retain the tertiary structure of SEQ ID NO: 21.

In one embodiment, the truncated PreS1, such as CΔPreS1 described herein, optionally with a fused surface antigen, is encoded downstream (3') of the intergenic sequence.

The PreS1 truncation advantageously favours antibody generation for both T-cell and antibody response for the HBV antigens provided in the immunogen expression cassette. Without being bound by theory, antibody generation is possibly due to proper folding of PreS1 and Surface antigens.

### NΔPreS1

In one embodiment, the truncated PreS1 comprises NΔPreS1 as described herein. The NΔPreS1 and PreS2 fusion may comprise or consist of SEQ ID NO: 15, or a variant thereof. A variant of SEQ ID NO: 15 may comprise a sequence having at least 70%, 75%, 80%, 85%, 90%, 95%, 98%, 99%, or 99.5% identity with SEQ ID NO: 15. The variant of SEQ ID NO: 15 may substantially retain the immunogenicity of SEQ ID NO: 15.

In one embodiment, the truncated PreS1 comprises NΔPreS1 as described herein. The NΔPreS1 and PreS2 fusion may be encoded by nucleic acid comprising or consisting of SEQ ID NO: 38 or 61, or a variant thereof. A variant of SEQ ID NO: 38 or 61 may comprise a sequence having at least 70%, 75%, 80%, 85%, 90%, 95%, 98%, 99%, or 99.5% identity with SEQ ID NO: 38 or 61. The encoded variant of SEQ ID NO: 38 or 61 may substantially retain the immunogenicity of peptide encoded by SEQ ID NO: 38 or 61.

The NΔPreS1 and PreS2 fusion sequence advantageously provides a good T-cell response. NΔPreS1 advantageously includes amino acids in a sequence that would still preserve T-cell epitopes (8-11 amino acids for CD8 T-cell epitopes and slightly longer (12-16) for CD4 T-cell epitopes.

In an embodiment wherein the immunogen expression cassette encodes NΔPreS1 fused with PreS2 upstream (5') of intergenic sequence, it may be further fused with the modified polymerase (Pmut). A linker sequence, such as a linker described herein, may be provided between the PreS2 and modified polymerase (Pmut). For example, NΔPreS1 + PreS2 + linker + Pmut.

### HBV PreS2

The HBV PreS2 may comprise or consist of a full length wild-type HBV PreS2 sequence, or a variant thereof. The HBV PreS2 variant may comprise or consist of a truncated HBV PreS2 sequence.

The HBV PreS2 may comprise or consist of the sequence of SEQ ID NO: 53 or a variant thereof. A variant of SEQ ID NO: 53 may comprise a sequence having at least 70%, 75%, 80%, 85%, 90%, 95%, 98%, 99%, or 99.5% identity with SEQ ID NO: 53. The variant of SEQ ID NO: 53 may substantially retain the immunogenicity of SEQ ID NO: 53. The variant of SEQ ID NO: 53 may substantially retain the tertiary structure of SEQ ID NO: 53.

### Peptide Adjuvant

The immunogen expression cassette may further encode a peptide adjuvant. The peptide adjuvant may comprise TPA (tissue plasminogen activator). In one embodiment, the peptide adjuvant may comprise a human or non-human invariant chain (Ii), or a fragment thereof. A fragment of the long isoform (isoform (b)) of the human CD74 molecule, is also known as the invariant chain (Nucleic Acids Res. 1985 December 20; 13(24): 8827-8841). It is known that N-terminal fragments of the invariant chain (li) which comprise at least the transmembrane domain thereof, provide a surprisingly effective adjuvant function when expressed as a fusion protein with an antigen of interest. Fragments encompassing the transmembrane domain and the cytoplasmic domain, and preferably including the N-terminal 16 amino acids of the long isoform of the protein are particularly efficacious.

The invariant chain may comprise or consist of a shark invariant chain (SIi), or fragment or a functional variant thereof. The variant shark invariant chain (SIi) may comprise a truncated invariant shark invariant chain. Other non-human animal sources of an invariant chain, or fragment thereof, include chicken, quail, trout, zebrafish, carp, frog, grouper, shark, mandarin fish or mallard. The skilled person will be familiar with appropriate invariant chains, or fragments thereof, for use as peptide adjuvants encoded in an expression cassette/vector, for example the invariant chain may be any invariant chain as provided in WO2015082922.

The peptide adjuvant may comprise or consist of the sequence of SEQ ID NO: 4 (SIi) or a variant thereof. A variant of SEQ ID NO: 4 may comprise a sequence having at least 70%, 75%, 80%, 85%, 90%, 95%, 98%, 99%, or 99.5% identity with SEQ ID NO: 4. The variant of SEQ ID NO: 4 may substantially retain the adjuvant function of SEQ ID NO: 4.

The peptide adjuvant may be encoded by a sequence comprising or consists of the sequence of SEQ ID NO: 29 (TPA nucleic acid sequence) or a variant thereof. A variant of SEQ ID NO: 29 may comprise a sequence having at least 70%, 75%, 80%, 85%, 90%, 95%, 98%, 99%, or 99.5% identity with SEQ ID NO: 29. The variant of SEQ ID NO: 29 may encode a peptide adjuvant that substantially retains the adjuvant function of the TPA encoded by SEQ ID NO: 29. The peptide adjuvant may be encoded by a sequence comprising or consists of the sequence of SEQ ID NO: 60 (TPA nucleic acid sequence) or a variant thereof. A variant of SEQ ID NO: 60 may comprise a sequence having at least 70%, 75%, 80%, 85%, 90%, 95%, 98%, 99%, or 99.5% identity with SEQ ID NO: 60. The variant of SEQ ID NO: 60 may encode a peptide adjuvant that substantially retains the adjuvant function of the TPA encoded by SEQ ID NO: 60.

The peptide adjuvant may comprise or consist of the sequence of SEQ ID NO: 30 (TPA) or a variant thereof. A variant of SEQ ID NO: 30 may comprise a sequence having at least 70%, 75%, 80%, 85%, 90%, 95%, 98%, 99%, or 99.5% identity with SEQ ID NO: 30. The variant of SEQ ID NO: 30 may substantially retain the adjuvant function of SEQ ID NO: 30.

The peptide adjuvant may be encoded at an N-terminal position of a protein/antigen to be expressed from the immunogen expression cassette. A first peptide adjuvant may be encoded at an N-terminal position of a first protein/antigen (such as the Core and Polymerase fusion) to be expressed from the immunogen expression cassette, and a second peptide adjuvant may be encoded at an N-terminal position of a second protein/antigen (such as surface antigen) to be expressed from the immunogen expression cassette.

### HBV genotype

The HBV may be HBV genotype C. In another embodiment, the HBV may be of any one of the 10 genotypes (A-J). All of the encoded HBV proteins/antigens may be derived from one genotype, for example HBV genotype C.

### Linkers

In one embodiment, linker residues may be encoded between one or more, or all, of the protein/antigen sequences that are provided in a fusion protein (e.g. providing junctions between the sequences in the protein). In an embodiment comprising a peptide adjuvant, a linker may be encoded between the peptide adjuvant and the downstream encoded protein/antigen. In one embodiment, a linker is encoded between the sequences of the HBV core and modified HBV polymerase.

The linker residues may comprise random amino acid sequences, or amino-acids that have been selected to be non-immunogenic based on epitope prediction computer programs or experiments in animal models. For example, a linker may not be considered if it is predicted or known to be an epitope (i.e. in order to avoid an immune response to epitopes, e.g. artificial epitopes, not found in HBV. The linker may be flexible. The linker may comprise or consist of K, G, P or S amino acid residues, or combinations thereof. In one embodiment, the linker may comprise or consist of G and/or P amino acid residues. The linker residues may be between 1 and 10 amino acids in length. In another embodiment, the linker residues may be between 2 and 8 residues in length. In another embodiment, the linker residues may be between 1 and 6 residues in length.

A linker may comprise or consist of any of the sequences KGGGPGGG (SEQ ID NO: 5), GGGSGGG (SEQ ID NO: 7), KGGS (SEQ ID NO: 14), KSP, GSKGK (SEQ ID NO: 20), LEGGSGG (SEQ ID NO: 22), SKSGPPSGKS (SEQ ID NO: 31), GSKSGSK (SEQ ID NO: 32), SKSPGSGPP (SEQ ID NO: 33), or ASKGGKSG (SEQ ID NO: 34).

In one embodiment, a linker may comprise the sequence KGGGPGGG (SEQ ID NO: 5). In another embodiment, a linker may comprise the sequence GGGSGGG (SEQ ID NO: 7). In another embodiment, a linker may comprise the sequence KGGS (SEQ ID NO: 14). In another embodiment, a linker may comprise the sequence KSP. In another embodiment, a linker may comprise the sequence GSKGK (SEQ ID NO: 20). In another embodiment, a linker may comprise the sequence LEGGSGG (SEQ ID NO: 22). In another embodiment, a linker may comprise the sequence SKSGPPSGKS (SEQ ID NO: 31). In another embodiment, a linker may comprise the sequence GSKSGSK (SEQ ID NO: 32). In another embodiment, a linker may comprise the sequence SKSPGSGPP (SEQ ID NO: 33). In another embodiment, a linker may comprise the sequence ASKGGKSG (SEQ ID NO: 34).

Advantageously, the use of linkers can avoid generation of peptides with homology to human proteome (which could potentially generate immune response to self-antigen) and they avoid immune-dominant artificial epitopes. Additionally, linkers can provide a flexible hinge between segments of protein, so that they can fold into their native conformation. For example, a linker between CΔPreS1 and S allows independent folding of CΔPreS1 and S, so that they could generate antibodies for conformational epitopes for the respective proteins.

Linkers may be varied between different immunogen expression cassettes. Using different linkers avoids boosting of any T-cell response created to any potential artificial epitopes (i.e. by changing linkers or changing junctions). For example, when vaccines are used in prime boost vaccination strategies, changing the linkers or the order of proteins within the immunogen layout, helps to overcome boosting of artificial epitope response.

### Other elements of the immunogen expression cassette(s)

In an embodiment wherein the immunogen expression cassette is for use in an MVA vector, the immunogen expression cassette may further comprise F11 left and right flanking sequences to allow insertion into the MVA F11 locus by homologous recombination.

The F11-left flank sequence may comprise or consist of the sequence of SEQ ID NO: 35, or a variant thereof. The F11-right flank sequence may comprise or consist of the sequence of SEQ ID NO: 36, or a variant thereof. A variant of SEQ ID NO: 35 or 36 may comprise a sequence having at least 70%, 75%, 80%, 85%, 90%, 95%, 98%, 99%, or 99.5% identity with SEQ ID NO: 35 or 36 respectively. The variant of SEQ ID NO: 35 or 36 may substantially retain the homologous recombination function of SEQ ID NO: 35 or 36 respectively. The skilled person will appreciate that 1, 2, 3, 4, 5 or more amino acid residues may be substituted, added or removed without affecting function. For example, conservative substitutions may be considered.

The immunogen expression cassette may further comprise a transcription terminator sequence. The transcription terminator sequence may be provided in embodiments of the immunogen expression cassette comprising a secondary promoter. For example, the transcription terminator sequence may be provided downstream of a protein expressed by the primary promoter, but before (upstream of) the secondary promoter. The transcription terminator sequence may comprise or consist of the sequence TTTTTGT, or variants thereof.

In one embodiment, the immunogen expression cassette described herein may be isolated or provided in a non-viral vector.

Therefore, according to another description there is provided a nucleic acid comprising the immunogen expression cassette described herein, optionally wherein the nucleic acid is isolated nucleic acid.

According to another description there is provided a composition comprising the viral vector according to the description, optionally wherein the composition is a pharmaceutically acceptable composition.

The composition may be immunogenic, for example in a mammal, such as a human. The composition may comprise a pharmaceutically acceptable carrier. The composition may be a pharmaceutical composition comprising a pharmaceutically acceptable carrier. The composition may be for use in the prophylaxis or treatment of HBV infection.

According to another description there is provided a method of treatment or prophylaxis of HBV infection comprising the administration of the viral vector, nucleic acid or composition according to the description.

The method of treatment or prophylaxis of HBV infection may be a method of vaccination.

According to another description there is provided a viral vector, nucleic acid or composition according to the description for use in the treatment or prevention of HBV infection, optionally wherein the use is in a vaccine.

According to another description there is provided a vaccine comprising the viral vector, nucleic acid or composition according to the description.

The vaccine may be a prime vaccine. The vaccine may be a boost vaccine. Where a boost vaccine is provided following a prime vaccine, the viral vector may be a different viral vector according to the description.

Advantageously, the provision of a different viral vector between prime and boost vaccines can avoid the provision of "false" epitopes formed across junctions of one protein/antigen sequence with another. i.e. the same junction may not occur in a reordered protein.

According to another description, there is provided a prime boost vaccination kit comprising
- a prime vaccination according to the description;
- a boost vaccination according to the description.

The prime and boost vaccination may comprise different viral vectors.

The viral vector may be used in a vaccine in combination with another therapeutically or prophylactically active ingredient. The viral vector may be used in a vaccine in combination with an adjuvant.

The viral vector, nucleic acid encoding the viral vector may be provided in a pharmaceutically acceptable carrier.

The viral vector or composition according to the description may not comprise wild-type HBV, or the nucleic acid according to the description may not encode wild-type HBV.

Reference to sequence "identity" used herein may refer to the percentage identity between two aligned sequences using standard NCBI BLASTp parameters (http://blast.ncbi.nlm.nih.gov).

The term "immunogenic", when applied to the viral vector, nucleic acid or composition of the present description means capable of eliciting an immune response in a human or animal body. The immune response may be protective of HBV. The term "protective" means prevention of Hepatitis B disease, a reduced risk of Hepatitis B disease, reduced risk of HBV infection, transmission and/or progression, reduced severity of Hepatitis B disease, a cure of Hepatitis B, an alleviation of symptoms of Hepatitis B, or a reduction in severity of Hepatitis B disease symptoms.

The term "prophylaxis" means prevention of or protective treatment for Hepatitis B. The prophylaxis may include a reduced risk of HBV infection, transmission and/or Hepatitis B disease progression, or reduced severity of Hepatitis B disease.

The term "treatment", means a cure of Hepatitis B, an alleviation of symptoms, or a reduction in severity of Hepatitis B disease or Hepatitis B disease symptoms.

With reference to "variant" nucleic acid sequences, the skilled person will appreciate that 1, 2, 3, 4, 5 or more codons may be substituted, added or removed without affecting function. For example, conservative substitutions may be considered.

With reference to "variant" amino acid sequences, the skilled person will appreciate that 1, 2, 3, 4, 5 or more amino acids may be substituted, added or removed without affecting function. For example, conservative substitutions may be considered.

The skilled person will understand that optional features of one embodiment or aspect of the description may be applicable, where appropriate, to other embodiments or aspects of the description.

Embodiments of the description will now be described in more detail, by way of example only, with reference to the accompanying drawings.

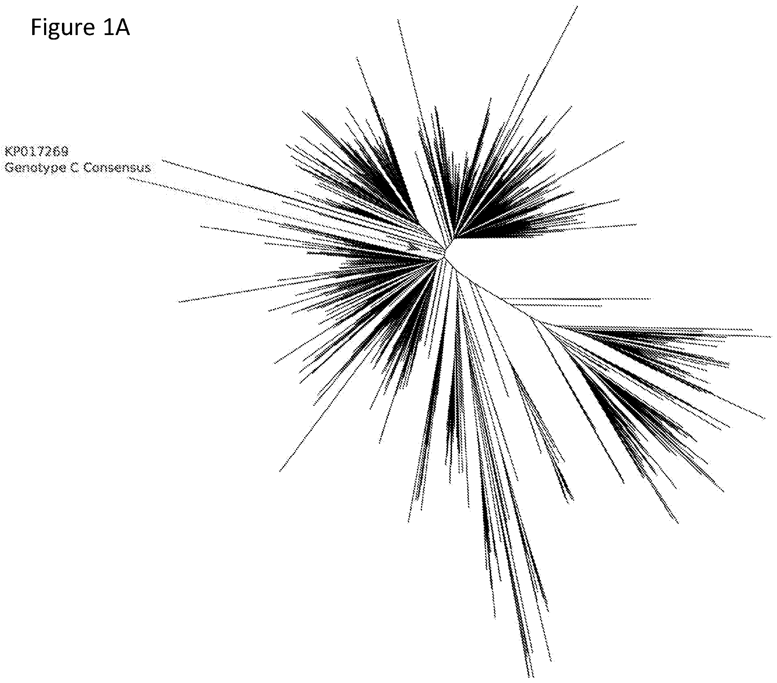

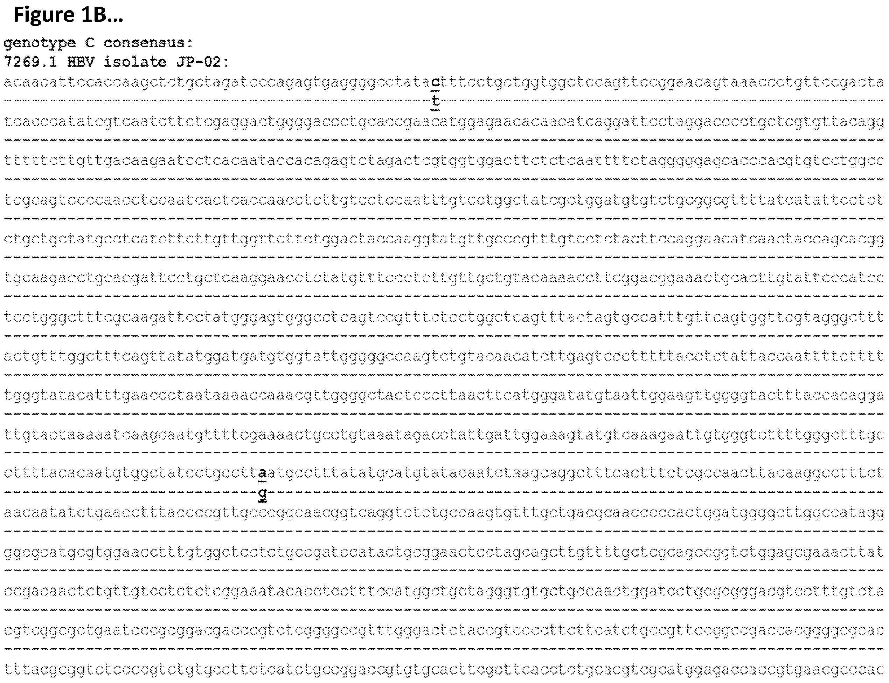

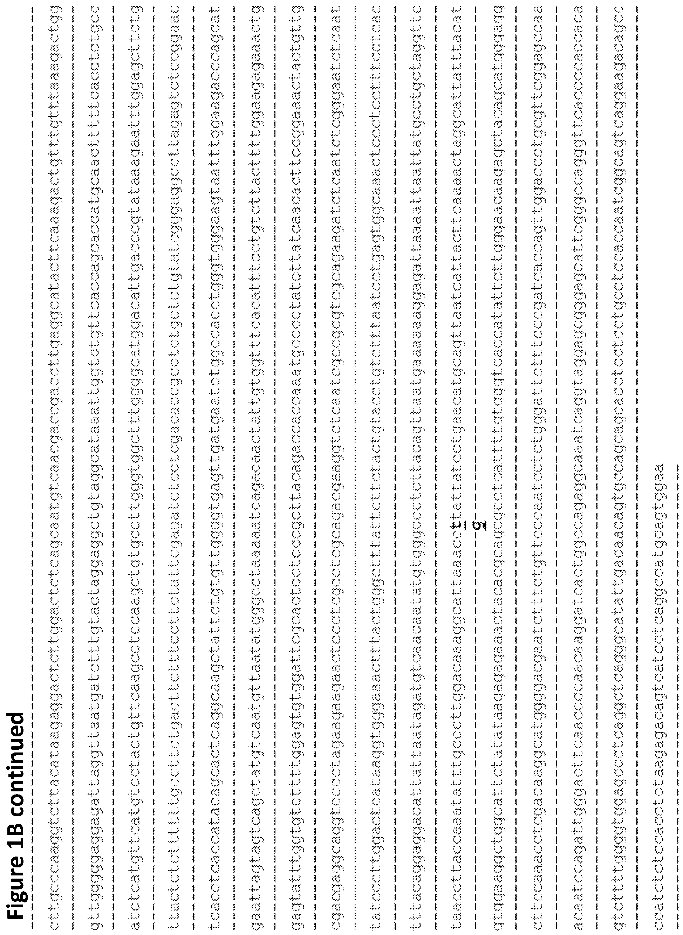

**Figure 1****:** **(A)** **Phlyogenitic relationship of 1447 HBV genotype C sequences used to generate consensus sequence (B) Comparison of HBV genotype C consensus (SEQ ID NO: 1) and KP017269.1 HBV isolate JP-02 (SEQ ID NO: 2).** 1447 HBV genotype C nucleotide sequences, downloaded from HBV data base, HBVdb: https://hbvdb.ibcp.fr/HBVdb/HBVdbIndex, were aligned using MAFFT (a multiple sequence alignment program), to generate the HBV genotype C consensus sequence and a phylogenetic tree and. Alignment of 3215 nucleotide sequences of the consensus and chosen patient's sequence (KP017269.1 HBV isolate JP-02) showed three nucleotide differences (at positions 52, 1053 and 2699), which are highlighted in grey colour. The sequence of the HBV genotype C consensus sequence generated from 1447 genotype c isolate jp-02 is provided below.

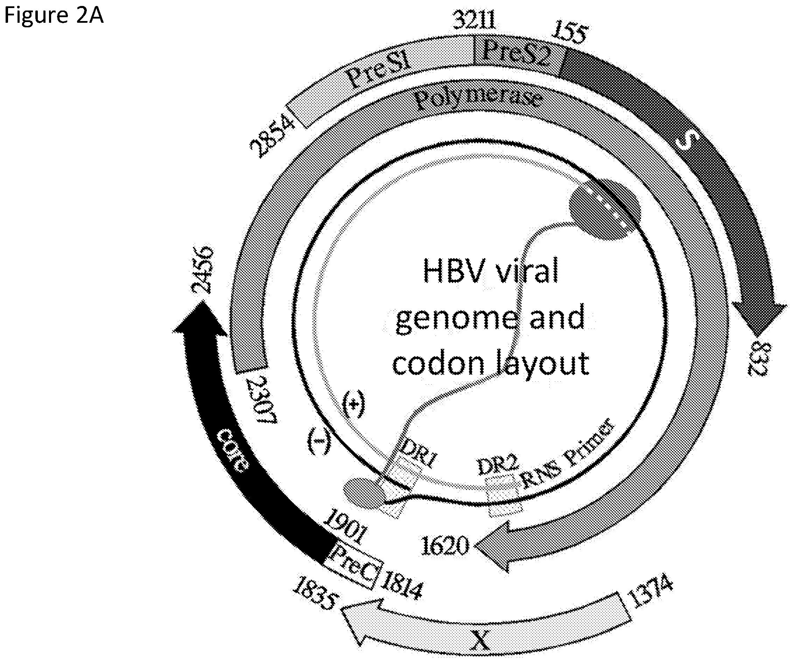

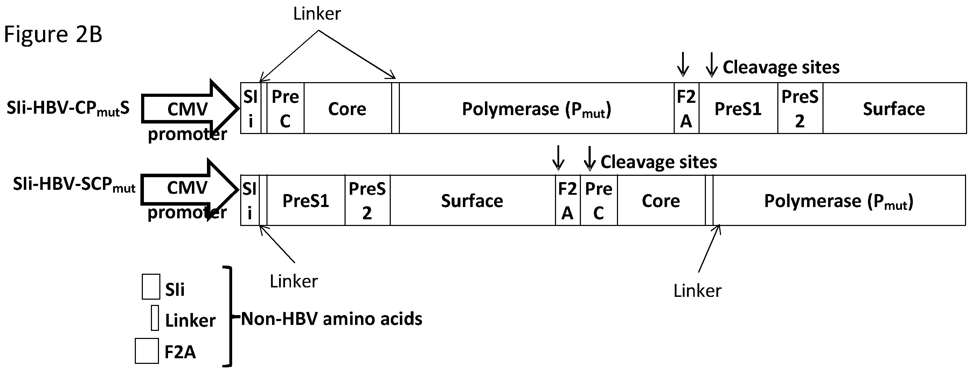

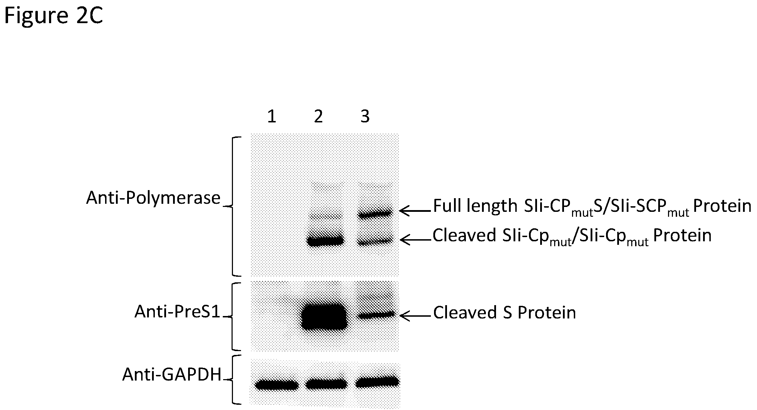

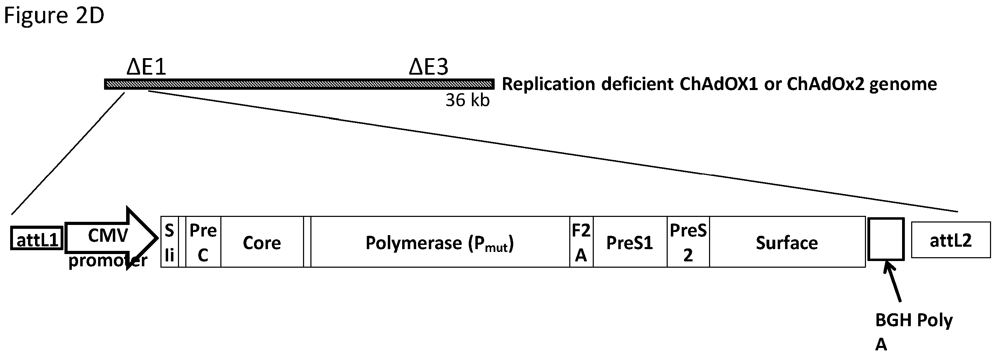

**Figure 2****:** **(A)** **HBV viral genome and codon layout.** The HBV virion has a partial double stranded DNA (having a full length negative strand DNA and a partially synthesised positive strand DNA attached to the polymerase protein). The genome is approximately 3.2 Kb in length and has four major codons: core (including the precore region), polymerase, surface (including the preS1 and preS2 region) and X. **(B) HBV Immunogen Layout.** Two immunogens SIi-HBV-CPmutS and SIi-HBV-SCPmut were designed. Both immunogens encodes HBV codons (encompassing precore, core, polymerase [Pmut], preS1, preS2 and surface proteins) and non HBV codons (comprising of a truncated Shark Invariant chain [SIi], two linkers [represented in Turquoise blue colour] and a Furin 2A [F2A] peptide sequence). Within the mammalian expression cassette, the immunogen codon sequences are placed next to CMV promoter. **(C) Invitro expression analysis.** Plasmids encoding SIi-HBV-CPmutS and SIi-HBV-SCPmut were transfected into HEK293A cells. 24 hours post-transfection, cells were lysed and the lysates were analysed in Western blot experiments using mouse anti-HBV-PreS1 and mouse anti-HBV-Polymerase antibodies. Blots probed with mouse anti-GAPDH served as loading controls. **(D) ChAdOX2-SIi-HBV-CPmutS.** The mammalian expression cassette with SIi-HBV-CPmutS immunogen codons was inserted into the replication deficient ChAdOx2 vector. Recombinant ChAdOX2-SIi-HBV-CPmutS virus was generated by transfecting the ChAdOX2-SIi-HBV-CPmutS vector into T-REx^{™}-293 cells (Thermo Fisher Scientific) using standard methods as previously described.

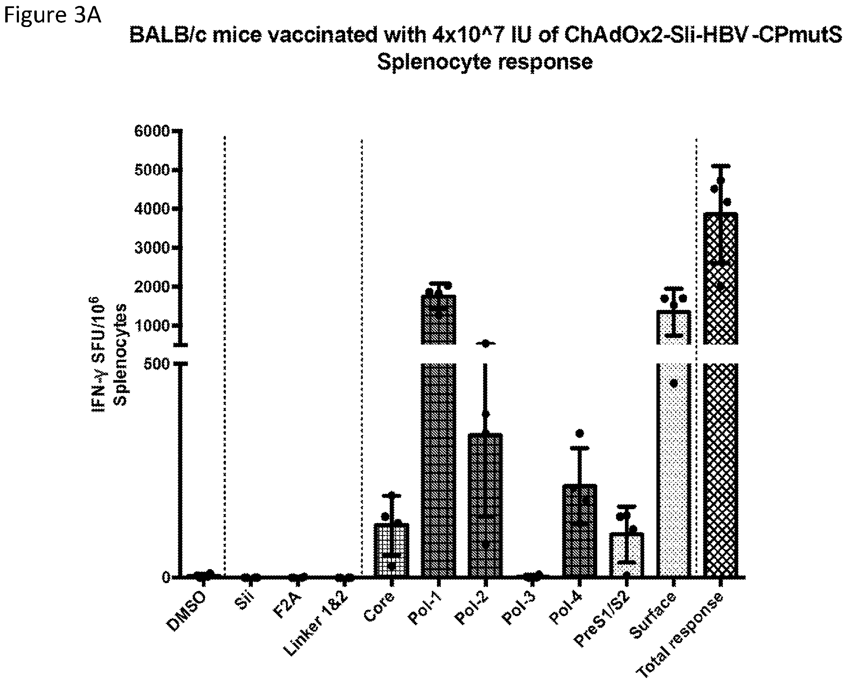

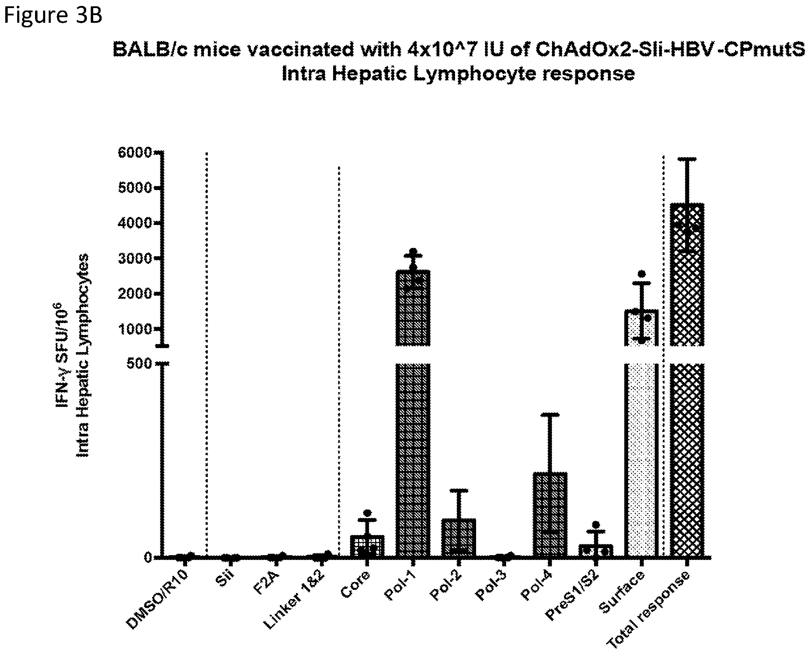

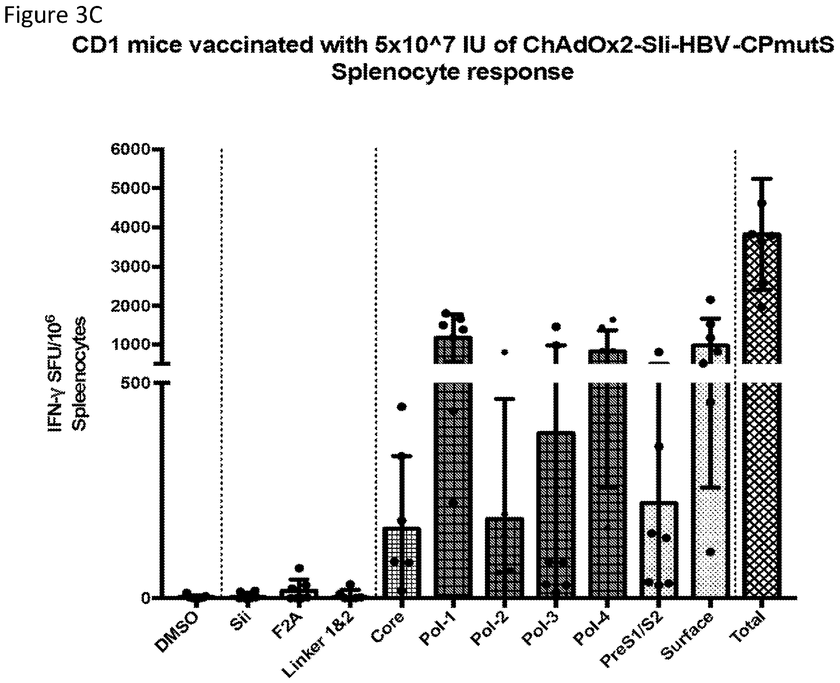

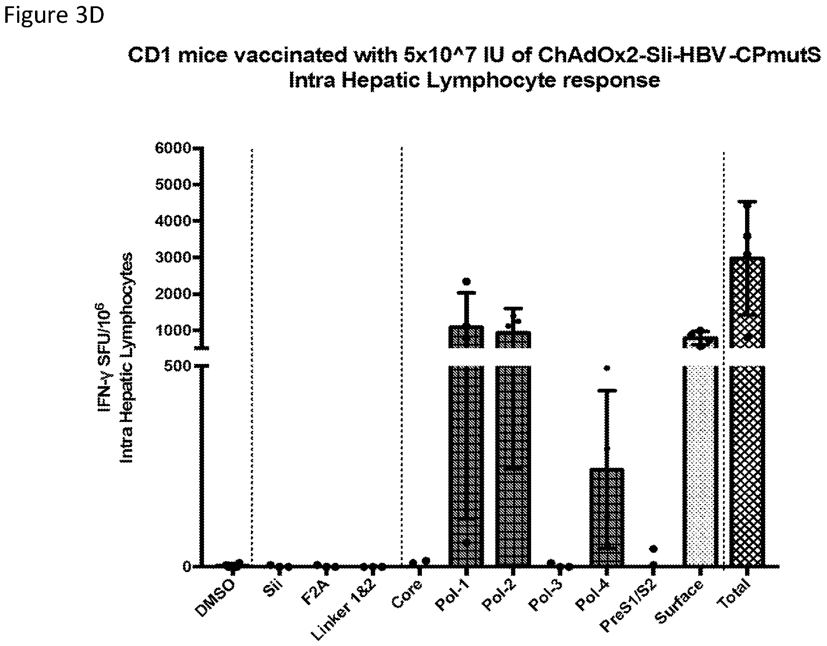

**Figure 3****: Testing ChAdOx2-SIi-HBV-CPmutS vaccine in naive mice models. (A) Spleenocyte responses in BALB/c mice (B) Intra Hepatic Lymphocyte responses in BALB/c mice (C) Spleenocyte responses in CD1 mice (D) Intra Hepatic Lymphocyte responses in CD1 mice.** BALB/c mice (4 mice at the age of 7 weeks) and CD1 (2 mice at age of 18 weeks and 5 mice at age of 13 weeks) were vaccinated by intramuscular injections with 4 x 10⁷ IU and 5 x 10⁷ IU per mice respectively of ChAdOx2-SIi-HBV-CPmutS vaccine. 14 days post-vaccination, mice were sacrificed, spleenocytes and intra hepatic lymphocytes (IHL) were isolated from spleen and liver according to standard protocol. 2x10⁵ spleenocytes and 1x10⁵ IHL's were plated on to ELSIPOT plates (pre-coated with Anti-mouse INFγ monoclonal antibody, by overnight incubation) along with DMSO (1%) or non-HBV peptide pool (A,I, L at a concentration of 3µg/ml) or HBV peptide pool (Core, Pol-1, Pol-2, Pol-3, Pol-4, PreS1/S2 and Surface at a concentration of 3µg/ml) or a positive control mitogen (PHA or Concanavalin A at a concentration of 10µg/ml and 12.5µg/ml respectively). After an overnight 15 hours incubation at 37⁰C, the plates were washed 7x with PBS and incubated with Biotin conjugated mouse anti-INFγ for 2 hours at room temperature, followed by 4x wash with PBS and AP conjugated anti-biotin incubation for 2 hours at room temperature. The plates were then washed 4x with PBS and developed with BCIP/NBT substrate until spots appeared on the wells. After a final wash with water and drying the spot forming units (SFU) per million cells from individual wells were counted on a automated ELISpot plate reader. ELISPOT responses for each peptide pool stimulus with spleenocytes and IHL's from BALB/c and CD1 mice are represented in separate charts. Total responses from HBV peptide stimulus were also calculated by combining ELISPOT responses to all HBV peptide pools (Core, Pol-1, Pol-2, Pol-3, Pol-4, PreS1/S2 and Surface).

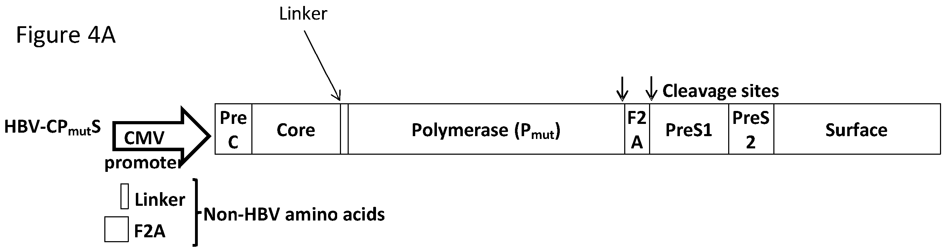

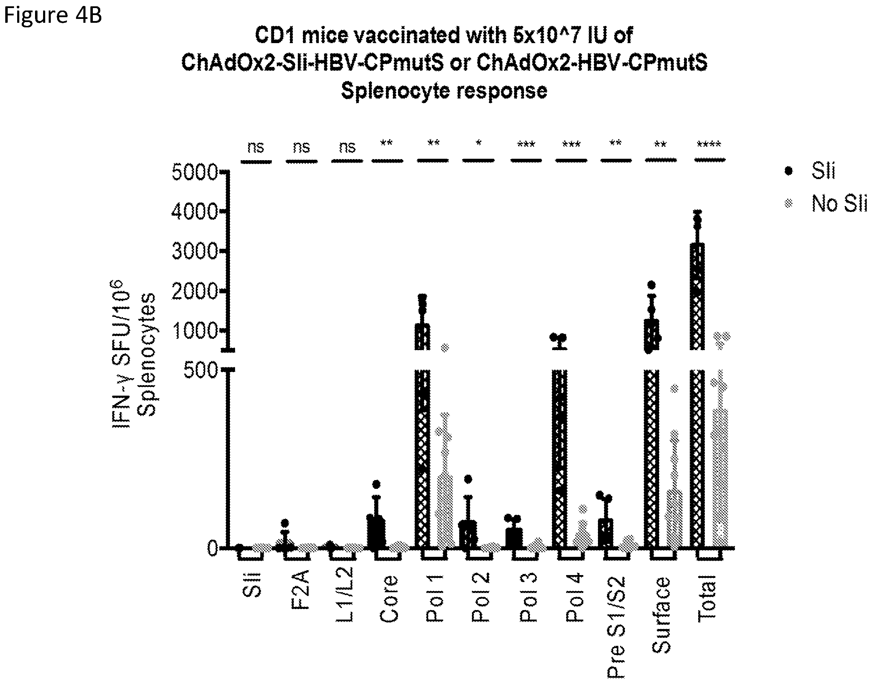

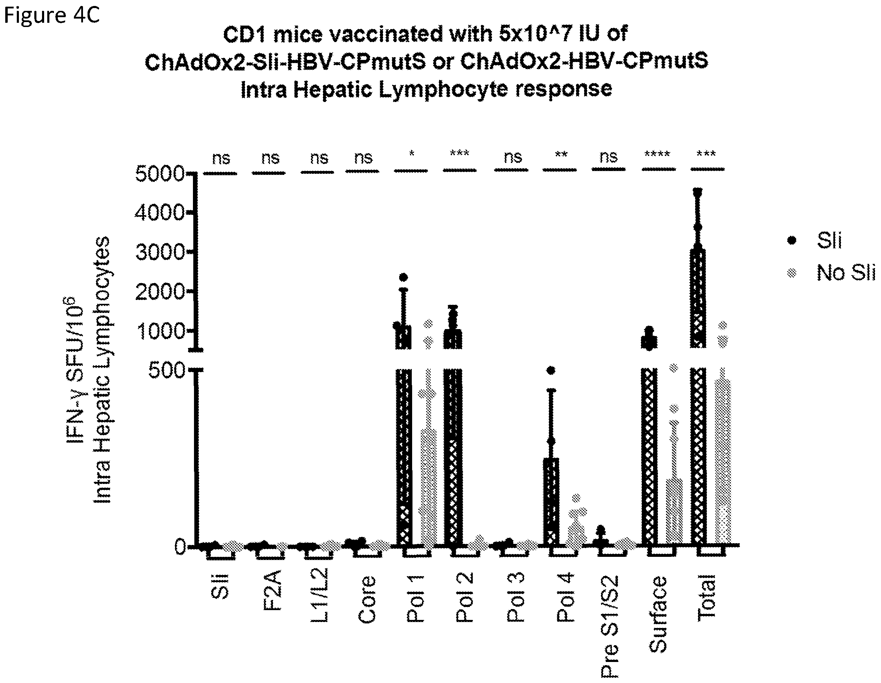

**Figure 4****: Comparison of the immunogenicity of HBV immunogen SIi-HBV-CPmutS with and without Shark Invariant chain (SIi). (A) HBV-CPmutS immunogen layout.** The immunogens HBV-CPmutS was generated by deleting the SIi and first linker present in SIi-HBV-CPmutS. HBV-CPmutS has exactly the same amino acids, except that it lacks the SIi and first linker sequence that is present in the SIi-HBV-CPmutS immunogen. **(B) Spleenocyte responses in CD1 mice. (C) Intra Hepatic Lymphocyte responses in CD1 mice.** ChAdOX2-HBV-CPmutS was generated as described earlier and CD1 mice (10 mice at age of 7 weeks) were vaccinated by intramuscular injections with 5 x 10⁷ IU per mice ChAdOx2-HBV-CPmutS vaccine and 14 days post-vaccination mice were sacrificed and spleenocytes and intra hepatic lymphocytes response data were generated, as described earlier. Data from 5 and 10 CD1 mice vaccinated with ChAdOx2-SIi-HBV-CPmutS and ChAdOx2-HBV-CPmutS respectively were compared and represented in charts for ease of comparision.

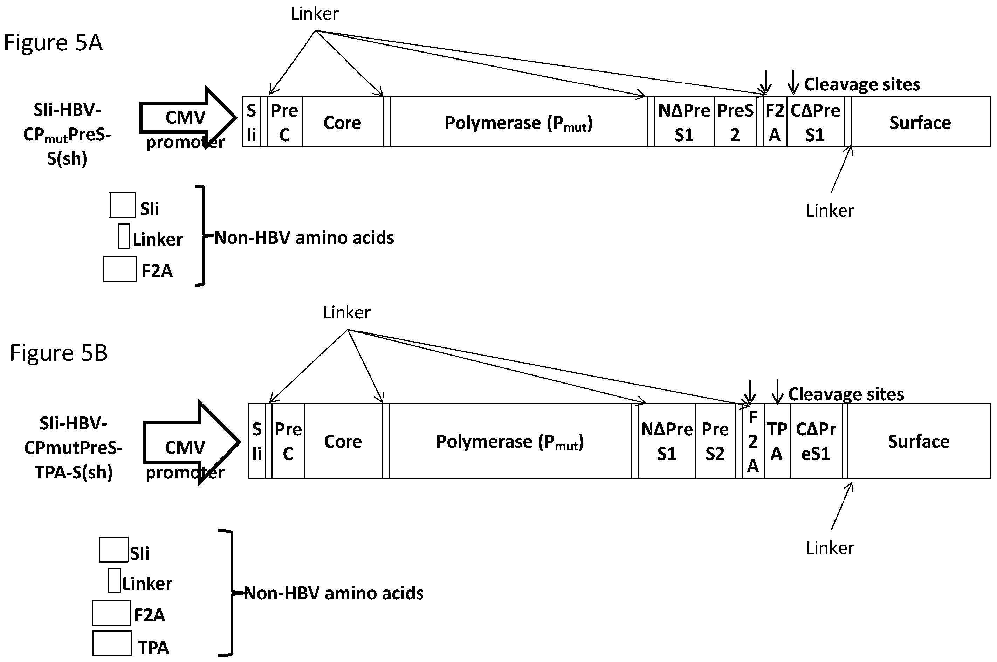

**Figure 5A****: Schematic layout of SIi-HBV-CPmutPreS-S(sh).**
**Figure 5B****: Schematic layout of SIi-HBV-CPmutPreS-TPA-S(sh).**

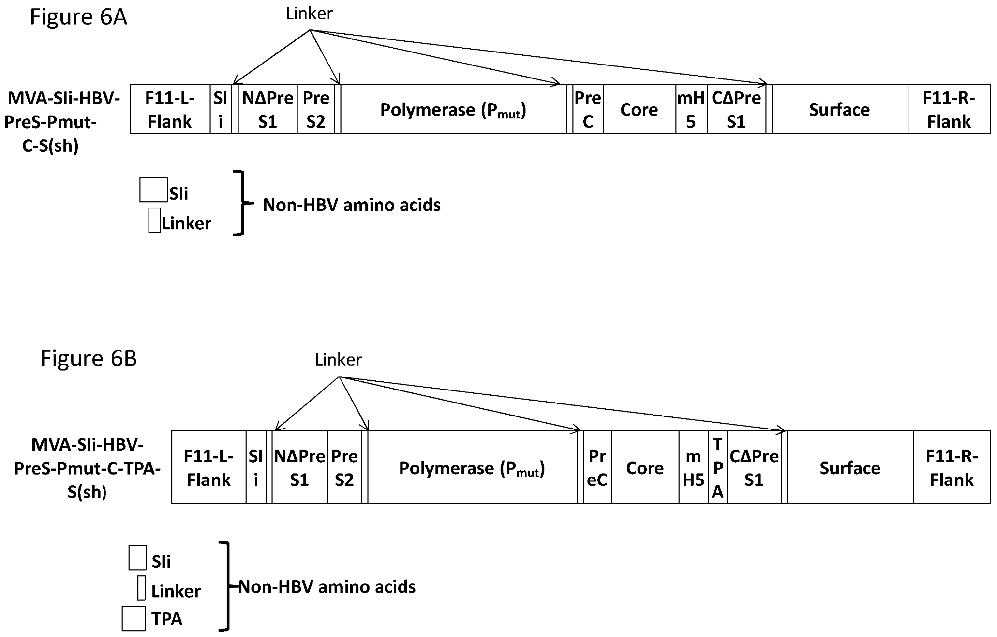

**Figure 6A****: Schematic layout of MVA-SIi-HBV-PreS-Pₘᵤₜ-C-S(sh).**
**Figure 6B****: Schematic layout of MVA-SIi-HBV-PreS-Pmut-C-TPA-S(sh).**

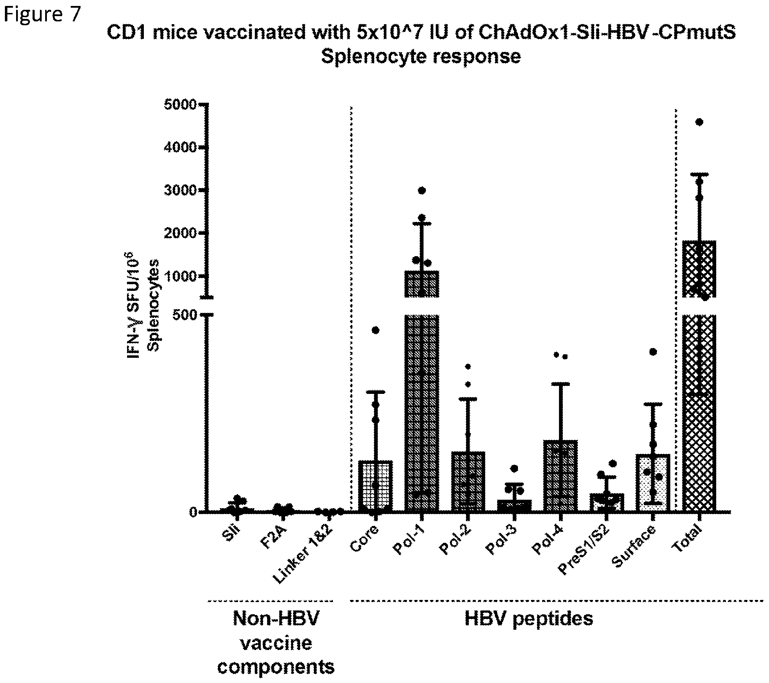

**Figure 7****: Testing ChAdOx1-SIi-HBV-CPmutS vaccine in BALB/c mice, splenocyte response:** BALB/c mice (8 mice at the age of 8 weeks) were vaccinated by intramuscular injections with 5 x 10⁷ IU per mice of ChAdOx1-SIi-HBV-CPmutS vaccine. 14 days post-vaccination, mice were sacrificed; splenocyte response data were generated, as described earlier. Splenocyte T-cell response for each peptide pool stimulus and pooled total response from all HBV-peptide pools are represented in the bar chart.

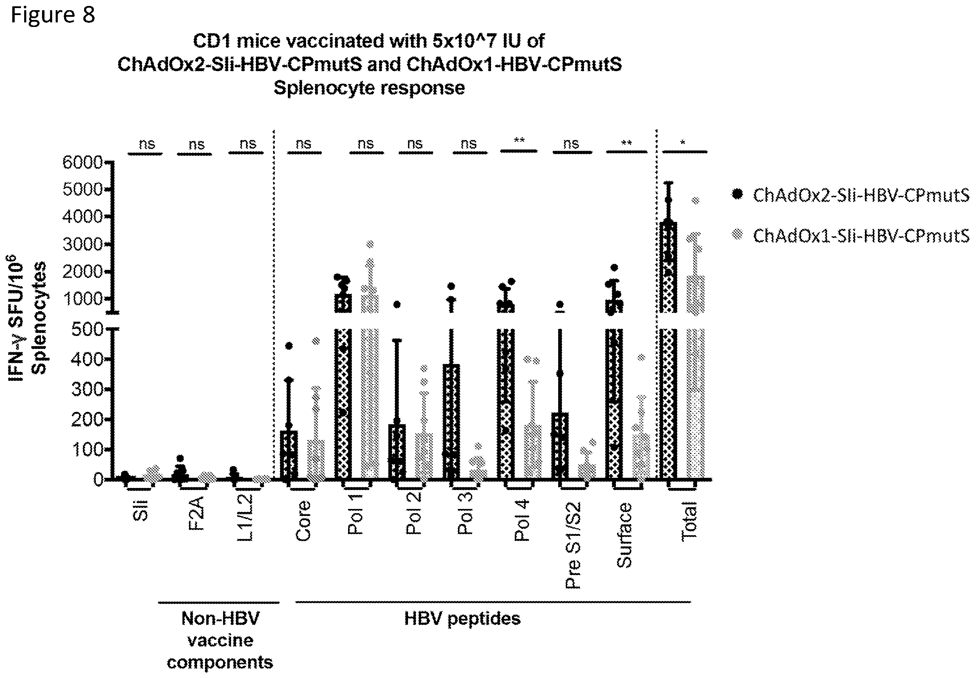

**Figure 8****: Comparison of the immunogenicity of HBV immunogen SIi-HBV-CPmutS encoded via ChAdOx1 and ChAdOx2.** HBV-CPmutS immunogen encoding ChAdOx1 and ChAdOx2 viral vectored vaccines were generated, as previously described (figure 2D). 10 CD1 mice at age of 7 weeks and 8 CD1 mice at age of 8 weeks were vaccinated by intramuscular injections with 5 x 10⁷ IU per mice of ChAdOx2-HBV-CPmutS and ChAdOx1-HBV-CPmutS vaccine, respectively, and 14 days post-vaccination mice were sacrificed and splenocyte response data were generated, as described earlier. Data from 10 and 8 CD1 mice vaccinated with ChAdOx2-SIi-HBV-CPmutS and ChAdOx1-HBV-CPmutS were compared and represented in charts for ease of comparison. A statistically significant higher magnitude of total splenocyte T-cell response was observed in mice vaccinated with ChAdOx2-HBV-CPmutS vaccine, compared to the ChAdOx1-HBV-CPmutS vaccine.

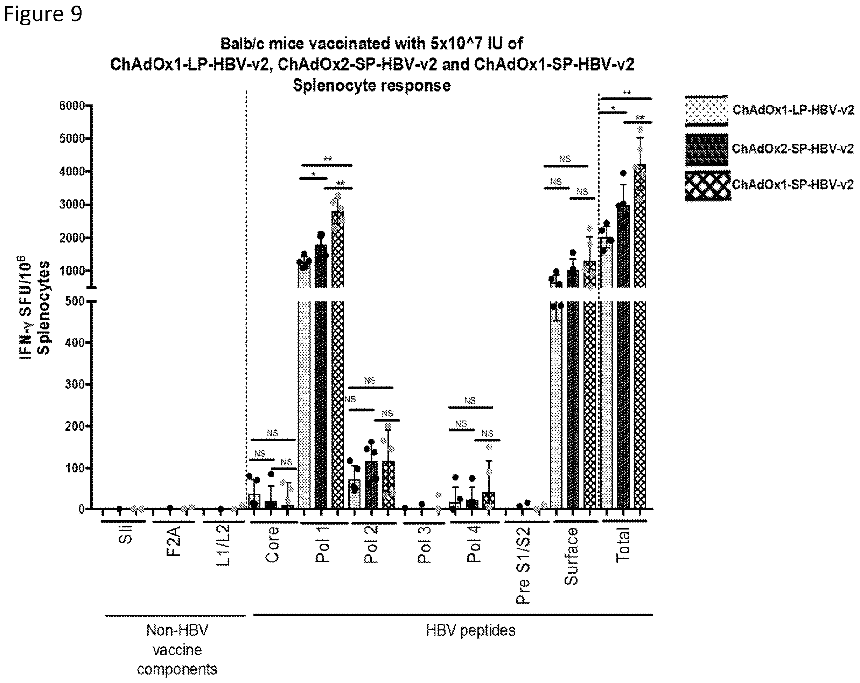

**Figure 9****: Comparison of the immunogenicity of HBV immunogen SIi-HBV-CPmutPreS-S(sh) (termed as HBV-v2) encoded via ChAdOx1 and ChAdOx2 viral vectors using either a short CMV promoter or long CMV promoter.** Two short CMV promoter and one long CMV promoter based SIi-HBV-CPmutPreS-S(sh) immunogen (termed as HBV-v2) encoding ChAdOx1 (ChAdOx1-LP-HBV-v2 and ChAdOx1-SP-HBV-v2) and ChAdOx2 (ChAdOx2-SP-HBV-v2) vaccines were generated as previously described. 15 Balbc mice at age of 8 weeks were split into 3 groups (5 mice per group) and vaccinated by intramuscular injections with 5 x 10⁷ IU of ChAdOx1-SP-HBV-v2 or ChAdOx2-SP-HBV-v2 or ChAdOx1-LP-HBV-v2 vaccine. 14 days post-vaccination mice were sacrificed and splenocyte response data were generated as previously described. Comparative data from all three HBV-v2 vaccines are represented in the same chart for ease of comparison. A statistically significant higher magnitude of total splenocyte T-cell response was observed in mice vaccinated with ChAdOx1-SP-HBV-v2 vaccine, compared to the other two HBV-v2 vaccines. In addition, the ChAdOx2-SP-HBV-v2 vaccine showed a statistically significant higher magnitude of total splenocyte T-cell response compared to the ChAdOx1-LP-HBV-v2 vaccine.

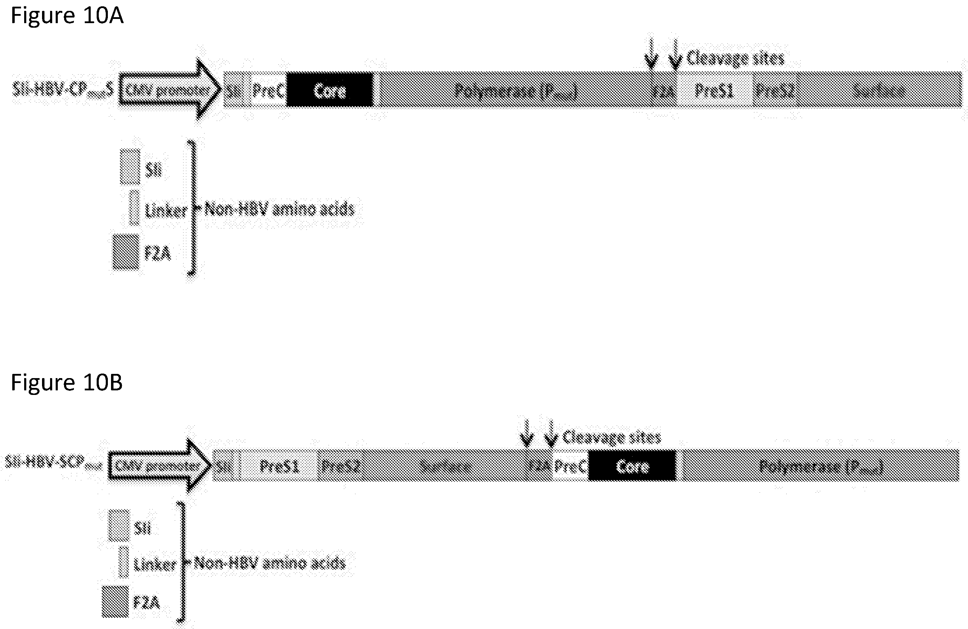

**Figure 10A****-G:** show immunogen layout for (A) SIi-HBV-CPmutS; (B) SIi-HBV-SCPₘᵤₜ (C) HBV-CPₘᵤₜS (D) SIi-HBV-CPₘᵤₜPreS-S(sh) (E) MVA-SIi-HBV-PreS-Pₘᵤₜ-C-S(sh) (F) SIi-HBV-CPₘᵤₜPreS-TPA-S(sh), and (G) MVA-SIi-HBV-PreS-Pₘᵤₜ-C-TPA-S(sh).

A HBV vaccine was generated based on the HBV genotype C, one of the commonest HBV genotypes in South East Asia that has more frequent association with chronic HBV infection. To design the HBV immunogen, a patient's HBV genotype C sequence (GeneBank: KP017269.1) was selected that was closest to the consensus, this was generated by aligning 1447 HBV-genotype-C sequences from HBVdb, a Hepatitis Virus B database (Figure 1A and 1B). The chosen HBV genotype C sequence had only three nucleotide changes compared to the consensus, of which two were silent mutations and one had a mutation in the polymerase protein (Figure 1C).

A strong and multi-antigen specific T cell response against different HBV proteins is believed to play a major role in viral clearance of a resolving HBV infection. Based on this HBV immunogens have been designed that encode all major proteins of the virus, namely the core (including the Pre-core region), a non-functional polymerase Pmut (Pmut: HBV polymerase with functional mutations, aiming to discourage the vaccine encoded polymerase's ability to participate in HBV viral replication) and the surface protein (including its PreS1 and PreS2 regions). Figure 2A and 2B shows the codon layout of HBV genome and a schematic representation of the first two HBV vaccine immunogen designs respectively. The layout has been designed to encode pre-core, core and Pmut as a fusion protein and a separate surface protein using a furin 2A (F2A) peptide cleavage mechanism, which by causing ribosomal skipping events, helps to encode two proteins from a single open reading frame.

Encoding multiple proteins within a single transgene cassette requires careful design, where the proximity of codon sequence to CMV promoter plays an important role in the level of expression of the encoded proteins. To analyse this two immunogens (Figure 2B), SIi-HBV-CPmutS and SIi-HBV-SCPmut, we generated which encodes the surface protein either at the end or the beginning of the immunogen cassette respectively and tested them in Western blot expression studies. Western blot expression analysis showed that layout SIi-HBV-CPmutS has the best F2A cleavage possibility, which generates large amounts of the expected CPmut and S proteins when compared to the SIi-HBV-SCPmut (Figure 2c).

Based on this observation, we decided to take forward SIi-HBV-CPmutS immunogen layout for the generation of chimpanzee adenovirus (ChAdOx2) based T-cell inducing HBV vaccine (Figure 2D). A small scale batch of ChAdOx2-SIi-HBV-CPmutS was manufactured at the viral vector core facility of the Jenner Institute, University of Oxford, following good manufacturing practices, and used in mice immunogenicity studies.

The ability of ChAdOx2-SIi-HBV-CPmutS vaccine to generate a T-cell immune response was tested using naive mice models. Naive BALB/c and CD1 mice were immunized with 4 x 10⁷ IU and 5 x 10⁷ IU per mice respectively of ChAdOx2-SIi-HBV-CPmutS vaccine by intra-muscular injections. 14 days post vaccination mice were sacrificed and IFN-γ ELISPOT assays were performed with spleenocytes and intra hepatic lymphocytes (IHL) isolated from spleen and liver. Synthetic peptides of 15mers, overlapping by 11 amino acids, generated across the whole of SIi-HBV-CPmutS immunogen, were combined into specific pools (representing different regions of the SIi-HBV-CPmutS immunogen) and used as a stimulant in IFN-γ ELISPOT assays. Results showed that the ChAdOx2-SIi-HBV-CPmutS vaccine generated a good IFN-γ ELISPOT response to SIi-HBV-CPmutS immunogen (Figure 3). Both BALB/c and CD1 mice showed stronger responses to the polymerase and surface proteins, a weak response to the core protein and negligible or no response to the non-HBV proteins (Shark Invariant chain [SIi], F2A and linkers) in the immunogen.

Previous studies have shown that the shark invariant chain (SIi), when placed at the N-terminus of the immunogen, functions as a molecular adjuvant and increases the overall T-cell immune response to the immunogen. To test this, we generated a HBV immunogen without SIi (HBV-CPmutS vaccine) (Figure 4A), and then generated a ChAdOx2-HBV-CPmutS vaccine and tested them in similar CD1 mice immunogenicity experiments. Results showed that the SIi vaccine (ChAdOx2-SIi-HBV-CPmutS) generates a higher magnitude of IFN-γ ELISPOT response (both Splenocyte and Intra Hepatic Lymphocyte IFN-γ ELISPOT response, represented in figure 4B, 4C) compared to the Non-SIi vaccine (ChAdOx2-HBV-CPmutS).

The main aim of the viral vectored HBV vaccine design is to generate both T-cell and antibody response to the HBV immunogen. In order to generate a successful antibody response, the immunogen encoded protein has to fold into its native conformation. To provide this, HBV immunogens, SIi-HBV-CPmutPreS-S(sh) and SIi-HBV-CPmutPreS-TPA-S(sh) (Figure 5A and 5B) were designed, which encode the N-terminal half of PreS1 domain fused with a linker to the S domain of surface protein, as the antibody inducing immunogen component, and the remaining peptide sequences of the surface protein (the C-terminal half of PreS1 domain and the whole PreS2 domain) were fused to the PreCore/Core/Pmut, in order to preserve the T-cell epitopes that would be required for generation of T-cell immune response. Figure 5A and 5B shows a schematic layout of SIi-HBV-CPmutPreS-S(sh).

MVA-HBV immunogens were also designed, which followed designs similar to SIi-HBV-CPmutPreS-S(sh) and and SIi-HBV-CPmutPreS-TPA-S(sh). However, the T-cell component of HBV immunogen SIi-HBV-CPmutPreS is encoded by the early promoter F11 and the antibody inducing component S(sh) or TPA- S(sh) is encoded by the early/late promoter mH5. The cloning cassette also has F11-left flank and F11-right flank sequences, to allow insertion into the F11 locus, by homologous recombination. Figure 6A and 6B shows schematic layouts of MVA-SIi-HBV-PreS-Pmut-C-S(sh) and MVA-SIi-HBV-CPmutPreS-TPA-S(sh) respectively.

**Conclusion:** The novel multi-antigen HBV immunogen based ChAdOx2-SIi-HBV-CPmutS are highly immunogenic in naive mice models. These studies pave way for future studies in chronic HBV mice models, to assess their ability to overcome chronic HBV infection through immunotherapeutic approaches.

### Sequences:

HBV genotype C consensus sequence generated from 1447 genotype C sequences (3215 base pairs) is provided as SEQ ID NO: 1. The Sequence of KP017269.1 HBV isolate JP-02 is provided as SEQ ID NO: 2.

### Example Cassettes

**1. SIi-HBV-CPₘᵤₜS**
   **1.1. SIi-HBV-CPₘᵤₜS:** Immunogen layout is shown in Figure 10A
   1.2. **SIi-HBV-CPₘᵤₜS:** Amino acid sequence (SEQ ID NO: 3) 
MSLLWGGVTVLAAMLIAGQVASVVFLVKGGGPGGGMQLFHLCLIISCSCPT
VQASKLCLGWLWGMDIDPYKEFGASVELLSFLPSDFFPSIRDLLDTASALYRE
ALESPEHCSPHHTALRQAILCWGELMNLATWVGSNLEDPASRELVVSYVNV
NMGLKIRQLLWFHISCLTFGRETVLEYLVSFGVWIRTPPAYRPPNAPILSTLPE
TTVVRRRGRSPRRRTPSPRRRRSQSPRRRRSQSRESQCGGGSGGGMPLSYQH
FRKLLLLDDEAGPLEEELPRLADEGLNRRVAEDLNLGNLNVSIPWTHKVGNF
TGLASSTVPVFNPEWQTPSFPHIHLQEDIINRCQQYVGPLTVNEKRRLKLIMPA
RFYPNLTKYLPLDKGIKPYYPEHAVNHYFKTRHYLHTLWKAGILYKRETTRS
ASFCGSPYSWEQELQHGRLVFQTSTRHGDESFCSQSSGILSRSPVGPCVRSQL
KQSRLGLQPQQGSLARGKSGRSGSIRARVHPTTRRSFGVEPSGSGHIDNSASST
SSCLHQSAVRKTAYSHLSTSKRQSSSGHAVELHNIPPSSARSQSEGPIFSAWWL
QFRNSKPASDYALTHIVNLLEDWGPATEHGEHNIRIPRTPARVTGGVFLVDKN
PHNTTESRLVVDFSQFSRGSTHVSWPKFAVPNLQSLTNLLSSNLSWLSLDVSA
AFYHIPLHPAAMPHLLVGSSGLPRYVARLSSTSRNINYQHGTMQDLHDSCSR
NLYVSLLLLYKTFGRKLHLYSHPIILGFRKIPMGVGLSPFLLAQFTSAICSVVR
RAFPHCLAFSYMDDVVLGAKSVQHLESLFTSITNFLLSLGIHLNPNKTKRWGY
SLNFMGYVIGSWGTLPQEHIVLKIKQCFRKLPVNRPIDWKVCQRIVGLLGFAA
PFTQCGYPALMPLYACIQSKQAFTFSPTYKAFLCKQYLNLYPVARQRSGLCQ
VFADATPTGWGLAIGHRAMRGTFVAPLPIHTAELLAACFARSRSGAKLIGTD
NSVVLSRKYTSFPWLLGCAANWILRGTSFVYVPSALNPAADPSAGRLGLYRP
LLHLPFRPTTGRTSLYAVSPSVPSHLPDRVHFASPLHVAWRPPRKRRAPVKQ
TLNFDLLKLAGDVESNPGPMGGWSSKPRQGMGTNLSVPNPLGFFPDHQLDP
AFGANSNNPDWDFNPNKDHWPEANQVGAGAFGPGFTPPHGGLLGWSPQAQ
GILTTVPAAPPPASTNRQSGRQPTPISPPLRDSHPQAMQWNSTTFHQALLDPR
VRGLYFPAGGSSSGTVNPVPTTASPISSIFSRTGDPAPNMENTTSGFLGPLLVL
QAGFFLLTRILTIPQSLDSWWTSLNFLGGAPTCPGQNSQSPTSNHSPTSCPPICP
GYRWMCLRRFIIFLFILLLCLIFLLVLLDYQGMLPVCPLLPGTSTTSTGPCKTCT
IPAQGTSMFPSCCCTKPSDGNCTCIPIPSSWAFARFLWEWASVRFSWLSLLVPF
VQWFVGLSPTVWLSVIWMMWYWGPSLYNILSPFLPLLPIFFCLWVYI
   1.3. **SIi-HBV-CPₘᵤₜS: Description for amino acid sequences of HBV immunogen**
      1.3.1. First amino acid of polypeptide = M
      1.3.2. Shark Invariant chain (SIi) = SLLWGGVTVLAAMLIAGQVASVVFLV (SEQ ID NO: 4)
      1.3.3. Linker = **KGGGPGGG** (SEQ ID NO: 5)
      1.3.4. C =
         1.3.4.1. PreC =
            MQLFHLCLIISCSCPTVQASKLCLGWLWG (SEQ ID NO: 16)
         1.3.4.2. Core = 
MDIDPYKEFGASVELLSFLPSDFFPSIRDLLDTASALYREALESP
EHCSPHHTALRQAILCWGELMNLATWVGSNLEDPASRELVVSY
VNVNMGLKIRQLLWFHISCLTFGRETVLEYLVSFGVWIRTPPAY
RPPNAPILSTLPETTVVRRRGRSPRRRTPSPRRRRSQSPRRRRSQS
RESQC (SEQ ID NO: 6)
      1.3.5. Linker = **GGGSGGG** (SEQ ID NO: 7)
      1.3.6. Pmut = (mutations: Y63A, C323A, C334A, C338A, C352A, R714A, D788A, R792A) 
MPLSYQHFRKLLLLDDEAGPLEEELPRLADEGLNRRVAEDLNLGNL
NVSIPWTHKVGNFTGLASSTVPVFNPEWQTPSFPHIHLQEDIINRCQQ
YVGPLTVNEKRRLKLIMPARFYPNLTKYLPLDKGIKPYYPEHAVNHY
FKTRHYLHTLWKAGILYKRETTRSASFCGSPYSWEQELQHGRLVFQT
STRHGDESFCSQSSGILSRSPVGPCVRSQLKQSRLGLQPQQGSLARGK
SGRSGSIRARVHPTTRRSFGVEPSGSGHIDNSASSTSSCLHQSAVRKT
AYSHLSTSKRQSSSGHAVELHNIPPSSARSQSEGPIFSAWWLQFRNSK
PASDYALTHIVNLLEDWGPATEHGEHNIRIPRTPARVTGGVFLVDKN
PHNTTESRLVVDFSQFSRGSTHVSWPKFAVPNLQSLTNLLSSNLSTFG
RKLHLYSHPIILGFRKIPMGVGLSPFLLAQFTSAICSVVRRAFPHCLAF
SYMDDVVLGAKSVQHLESLFTSITNFLLSLGIHLNPNKTKRWGYSLN
FMGYVIGSWGTLPQEHIVLKIKQCFRKLPVNRPIDWKVCQRIVGLLG
FAAPFTQCGYPALMPLYACIQSKQAFTFSPTYKAFLCKQYLNLYPVA
RQRSGLCQVFADATPTGWGLAIGHRAMRGTFVAPLPIHTAELLAACF
ARSRSGAKLIGTDNSVVLSRKYTSFPWLLGCAANWILRGTSFVYVPS
ALNPAADPSAGRLGLYRPLLHLPFRPTTGRTSLYAVSPSVPSHLPDRV
HFASPLHVAWRPP (SEQ ID NO: 8)
      1.3.7. Furin 2A (F2A) = **RKRRAPVKQTLNFDLLKLAGDVESNPGP** (SEQ ID NO: 9)
      1.3.8. Surface proteins (S) =
         1.3.8.1. PreS1 = 
MGGWSSKPRQGMGTNLSVPNPLGFFPDHQLDPAFGANSNNPDWD
FNPNKDHWPEANQVGAGAFGPGFTPPHGGLLGWSPQAQGILTTVP
AAPPPASTNRQSGRQPTPISPPLRDSHPQA (SEQ ID NO: 52)
         1.3.8.2. PreS2 = 
MQWNSTTFHQALLDPRVRGLYFPAGGSSSGTVNPVPTTASPISSIFS
RTGDPAPN (SEQ ID NO: 53)
         1.3.8.3. Surface (S) =

MENTTSGFLGPLLVLQAGFFLLTRILTIPQSLDSWWTSLNFLGGAPTC
PGQNSQSPTSNHSPTSCPPICPGYRWMCLRRFIIFLFILLLCLIFLLVLL
DYQGMLPVCPLLPGTSTTSTGPCKTCTIPAQGTSMFPSCCCTKPSDGN
CTCIPIPSSWAFARFLWEWASVRFSWLSLLVPFVQWFVGLSPTVWLS
VIWMMWYWGPSLYNILSPFLPLLPIFFCLWVYI (SEQ ID NO: 10)

The SIi-HBV-CPmutS nucleotide sequence is provided as SEQ ID NO: 46.
2. **SIi-HBV-SCPₘᵤₜ**
**2.1. SIi-HBV-SCPₘᵤₜ: Immunogen layout is shown in** **Figure 10B**
**2.2. SIi_HBV-SCPₘᵤₜ: Amino acid sequence** (SEQ ID NO: 11) 
MSLLWGGVTVLAAMLIAGQVASVVFLVKGGSMGGWSSKPRQGMGTNLSVP
NPLGFFPDHQLDPAFGANSNNPDWDFNPNKDHWPEANQVGAGAFGPGFTPP
HGGLLGWSPQAQGILTTVPAAPPPASTNRQSGRQPTPISPPLRDSHPQAMQWN
STTFHQALLDPRVRGLYFPAGGSSSGTVNPVPTTASPISSIFSRTGDPAPNMEN
TTSGFLGPLLVLQAGFFLLTRILTIPQSLDSWWTSLNFLGGAPTCPGQNSQSPT
SNHSPTSCPPICPGYRWMCLRRFIIFLFILLLCLIFLLVLLDYQGMLPVCPLLPG
TSTTSTGPCKTCTIPAQGTSMFPSCCCTKPSDGNCTCIPIPSSWAFARFLWEWA
SVRFSWLSLLVPFVQWFVGLSPTVWLSVIWMMWYWGPSLYNILSPFLPLLPIF
FCLWVYIRKRRAPVKQTLNFDLLKLAGDVESNPGPMQLFHLCLIISCSCPTV
QASKLCLGWLWGMDIDPYKEFGASVELLSFLPSDFFPSIRDLLDTASALYREA
LESPEHCSPHHTALRQAILCWGELMNLATWVGSNLEDPASRELVVSYVNVN
MGLKIRQLLWFHISCLTFGRETVLEYLVSFGVWIRTPPAYRPPNAPILSTLPET
TVVRRRGRSPRRRTPSPRRRRSQSPRRRRSQSRESQCGGGSGGGMPLSYQHF
RKLLLLDDEAGPLEEELPRLADEGLNRRVAEDLNLGNLNVSIPWTHKVGNFT
GLASSTVPVFNPEWQTPSFPHIHLQEDIINRCQQYVGPLTVNEKRRLKLIMPAR
FYPNLTKYLPLDKGIKPYYPEHAVNHYFKTRHYLHTLWKAGILYKRETTRSA
SFCGSPYSWEQELQHGRLVFQTSTRHGDESFCSQSSGILSRSPVGPCVRSQLK
QSRLGLQPQQGSLARGKSGRSGSIRARVHPTTRRSFGVEPSGSGHIDNSASSTS
SCLHQSAVRKTAYSHLSTSKRQSSSGHAVELHNIPPSSARSQSEGPIFSAWWL
QFRNSKPASDYALTHIVNLLEDWGPATEHGEHNIRIPRTPARVTGGVFLVDKN
PHNTTESRLVVDFSQFSRGSTHVSWPKFAVPNLQSLTNLLSSNLSWLSLDVSA
AFYHIPLHPAAMPHLLVGSSGLPRYVARLSSTSRNINYQHGTMQDLHDSCSR
NLYVSLLLLYKTFGRKLHLYSHPIILGFRKIPMGVGLSPFLLAQFTSAICSVVR
RAFPHCLAFSYMDDVVLGAKSVQHLESLFTSITNFLLSLGIHLNPNKTKRWGY
SLNFMGYVIGSWGTLPQEHIVLKIKQCFRKLPVNRPIDWKVCQRIVGLLGFAA
PFTQCGYPALMPLYACIQSKQAFTFSPTYKAFLCKQYLNLYPVARQRSGLCQ
VFADATPTGWGLAIGHRAMRGTFVAPLPIHTAELLAACFARSRSGAKLIGTD
NSVVLSRKYTSFPWLLGCAANWILRGTSFVYVPSALNPAADPSAGRLGLYRP
LLHLPFRPTTGRTSLYAVSPSVPSHLPDRVHFASPLHVAWRPP
2.3. **SIi-HBV-SCPₘᵤₜ: Description for amino acid sequences of HBV immunogen**
   2.3.1. First amino acid of polypeptide = M
   2.3.2. Shark Invariant chain (SIi) = SLLWGGVTVLAAMLIAGQVASVVFLV (SEQ ID NO: 4)
   2.3.3. Linker = **KGGS** (SEQ ID NO: 14)
   2.3.4. Surface proteins (S) =
      2.3.4.1. PreS1 = 
MGGWSSKPRQGMGTNLSVPNPLGFFPDHQLDPAFGANSNNPDWD
FNPNKDHWPEANQVGAGAFGPGFTPPHGGLLGWSPQAQGILTTVP
AAPPPASTNRQSGRQPTPISPPLRDSHPQA (SEQ ID NO: 52)
      2.3.4.2. PreS2 = 
MQWNSTTFHQALLDPRVRGLYFPAGGSSSGTVNPVPTTASPISSIFS
RTGDPAPN (SEQ ID NO: 53)
      2.3.4.3. Surface (S) = 
MENTTSGFLGPLLVLQAGFFLLTRILTIPQSLDSWWTSLNFLGGAPTC
PGQNSQSPTSNHSPTSCPPICPGYRWMCLRRFIIFLFILLLCLIFLLVLL
DYQGMLPVCPLLPGTSTTSTGPCKTCTIPAQGTSMFPSCCCTKPSDGN
CTCIPIPSSWAFARFLWEWASVRFSWLSLLVPFVQWFVGLSPTVWLS
VIWMMWYWGPSLYNILSPFLPLLPIFFCLWVYI (SEQ ID NO: 10)
   2.3.5. Furin 2A (F2A) = **RKRRAPVKQTLNFDLLKLAGDVESNPGP** (SEQ ID NO: 9)
   2.3.6. C =
      2.3.6.1. PreCore =
         MQLFHLCLIISCSCPTVQASKLCLGWLWG (SEQ ID NO: 16)
      2.3.6.2. Core = 
MDIDPYKEFGASVELLSFLPSDFFPSIRDLLDTASALYREALESP
EHCSPHHTALRQAILCWGELMNLATWVGSNLEDPASRELVVSY
VNVNMGLKIRQLLWFHISCLTFGRETVLEYLVSFGVWIRTPPAY
RPPNAPILSTLPETTVVRRRGRSPRRRTPSPRRRRSQSPRRRRSQS
RESQC (SEQ ID NO: 6)
   2.3.7. Linker = **GGGSGGG** (SEQ ID NO: 7)
   2.3.8. Pmut = (mutations: Y63A, C323A, C334A, C338A, C352A, R714A, D788A, R792A)

MPLSYQHFRKLLLLDDEAGPLEEELPRLADEGLNRRVAEDLNLGNLNVSIPWTH
KVGNFTGLASSTVPVFNPEWQTPSFPHIHLQEDIINRCQQYVGPLTVNEKRRLKLI
MPARFYPNLTKYLPLDKGIKPYYPEHAVNHYFKTRHYLHTLWKAGILYKRETTR
SASFCGSPYSWEQELQHGRLVFQTSTRHGDESFCSQSSGILSRSPVGPCVRSQLKQ
SRLGLQPQQGSLARGKSGRSGSIRARVHPTTRRSFGVEPSGSGHIDNSASSTSSCL
HQSAVRKTAYSHLSTSKRQSSSGHAVELHNIPPSSARSQSEGPIFSAWWLQFRNSK
PASDYALTHIVNLLEDWGPATEHGEHNIRIPRTPARVTGGVFLVDKNPHNTTESR
LVVDFSQFSRGSTHVSWPKFAVPNLQSLTNLLSSNLSTFGRKLHLYSHPIILGFRKI
PMGVGLSPFLLAQFTSAICSVVRRAFPHCLAFSYMDDVVLGAKSVQHLESLFTSIT
NFLLSLGIHLNPNKTKRWGYSLNFMGYVIGSWGTLPQEHIVLKIKQCFRKLPVNR
PIDWKVCQRIVGLLGFAAPFTQCGYPALMPLYACIQSKQAFTFSPTYKAFLCKQY
LNLYPVARQRSGLCQVFADATPTGWGLAIGHRAMRGTFVAPLPIHTAELLAACF
ARSRSGAKLIGTDNSVVLSRKYTSFPWLLGCAANWILRGTSFVYVPSALNPAADP
SAGRLGLYRPLLHLPFRPTTGRTSLYAVSPSVPSHLPDRVHFASPLHVAWRPP
(SEQ ID NO: 8)

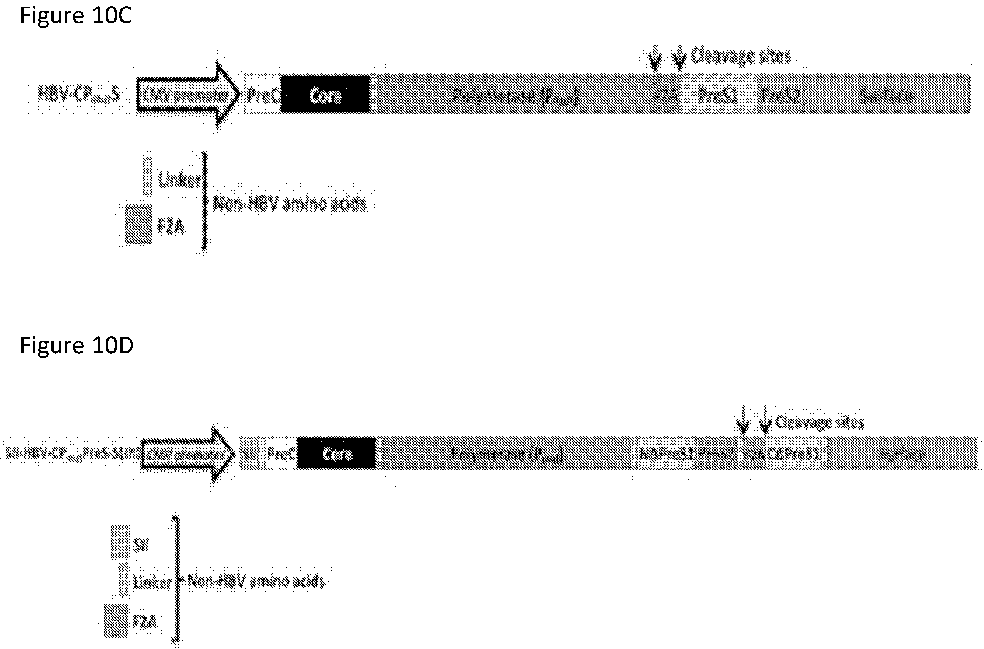

The SIi-HBV-SCPmut nucleotide sequence is provided as SEQ ID NO: 47:
3. **HBV-CPₘᵤₜS**
   3.1. HBV-CPₘᵤₜS: Immunogen layout is shown in Figure 10C
   3.2. HBV-CPₘᵤₜS: Amino acid sequence (SEQ ID NO: 12) 
MQLFHLCLIISCSCPTVQASKLCLGWLWGMDIDPYKEFGASVELLSFLPSDFFP
SIRDLLDTASALYREALESPEHCSPHHTALRQAILCWGELMNLATWVGSNLE
DPASRELVVSYVNVNMGLKIRQLLWFHISCLTFGRETVLEYLVSFGVWIRTPP
AYRPPNAPILSTLPETTVVRRRGRSPRRRTPSPRRRRSQSPRRRRSQSRESQCG
GGSGGGMPLSYQHFRKLLLLDDEAGPLEEELPRLADEGLNRRVAEDLNLGN
LNVSIPWTHKVGNFTGLASSTVPVFNPEWQTPSFPHIHLQEDIINRCQQYVGPL
TVNEKRRLKLIMPARFYPNLTKYLPLDKGIKPYYPEHAVNHYFKTRHYLHTL
WKAGILYKRETTRSASFCGSPYSWEQELQHGRLVFQTSTRHGDESFCSQSSGI
LSRSPVGPCVRSQLKQSRLGLQPQQGSLARGKSGRSGSIRARVHPTTRRSFGV
EPSGSGHIDNSASSTSSCLHQSAVRKTAYSHLSTSKRQSSSGHAVELHNIPPSS
ARSQSEGPIFSAWWLQFRNSKPASDYALTHIVNLLEDWGPATEHGEHNIRIPR
TPARVTGGVFLVDKNPHNTTESRLVVDFSQFSRGSTHVSWPKFAVPNLQSLT
NLLSSNLSWLSLDVSAAFYHIPLHPAAMPHLLVGSSGLPRYVARLSSTSRNIN
YQHGTMQDLHDSCSRNLYVSLLLLYKTFGRKLHLYSHPIILGFRKIPMGVGLS
PFLLAQFTSAICSVVRRAFPHCLAFSYMDDVVLGAKSVQHLESLFTSITNFLLS
LGIHLNPNKTKRWGYSLNFMGYVIGSWGTLPQEHIVLKIKQCFRKLPVNRPID
WKVCQRIVGLLGFAAPFTQCGYPALMPLYACIQSKQAFTFSPTYKAFLCKQY
LNLYPVARQRSGLCQVFADATPTGWGLAIGHRAMRGTFVAPLPIHTAELLAA
CFARSRSGAKLIGTDNSVVLSRKYTSFPWLLGCAANWILRGTSFVYVPSALNP
AADPSAGRLGLYRPLLHLPFRPTTGRTSLYAVSPSVPSHLPDRVHFASPLHVA
WRPPRKRRAPVKQTLNFDLLKLAGDVESNPGPMGGWSSKPRQGMGTNLS
VPNPLGFFPDHQLDPAFGANSNNPDWDFNPNKDHWPEANQVGAGAFGPGFT
PPHGGLLGWSPQAQGILTTVPAAPPPASTNRQSGRQPTPISPPLRDSHPQAMQ
WNSTTFHQALLDPRVRGLYFPAGGSSSGTVNPVPTTASPISSIFSRTGDPAPNM
ENTTSGFLGPLLVLQAGFFLLTRILTIPQSLDSWWTSLNFLGGAPTCPGQNSQS
PTSNHSPTSCPPICPGYRWMCLRRFIIFLFILLLCLIFLLVLLDYQGMLPVCPLLP
GTSTTSTGPCKTCTIPAQGTSMFPSCCCTKPSDGNCTCIPIPSSWAFARFLWEW
ASVRFSWLSLLVPFVQWFVGLSPTVWLSVIWMMWYWGPSLYNILSPFLPLLP
IFFCLWVYI
   3.3. **HBV-CPₘᵤₜS: Description for amino acid sequences of HBV immunogen**
      3.3.1. C =
         3.3.1.1. PreCore =
            MQLFHLCLIISCSCPTVQASKLCLGWLWG (SEQ ID NO: 16)
         3.3.1.2. Core = 
MDIDPYKEFGASVELLSFLPSDFFPSIRDLLDTASALYREALESP
EHCSPHHTALRQAILCWGELMNLATWVGSNLEDPASRELVVSY
VNVNMGLKIRQLLWFHISCLTFGRETVLEYLVSFGVWIRTPPAY
 
RPPNAPILSTLPETTVVRRRGRSPRRRTPSPRRRRSQSPRRRRSQS
RESQC (SEQ ID NO: 6)
      3.3.2. Linker = **GGGSGGG** (SEQ ID NO: 7)
      3.3.3. Pmut = (mutations: Y63A, C323A, C334A, C338A, C352A, R714A, D788A, R792A) 
MPLSYQHFRKLLLLDDEAGPLEEELPRLADEGLNRRVAEDLNLGNLNVSIP
WTHKVGNFTGLASSTVPVFNPEWQTPSFPHIHLQEDIINRCQQYVGPLTVNE
KRRLKLIMPARFYPNLTKYLPLDKGIKPYYPEHAVNHYFKTRHYLHTLWKA
GILYKRETTRSASFCGSPYSWEQELQHGRLVFQTSTRHGDESFCSQSSGILSR
SPVGPCVRSQLKQSRLGLQPQQGSLARGKSGRSGSIRARVHPTTRRSFGVEP
SGSGHIDNSASSTSSCLHQSAVRKTAYSHLSTSKRQSSSGHAVELHNIPPSSA
RSQSEGPIFSAWWLQFRNSKPASDYALTHIVNLLEDWGPATEHGEHNIRIPR
TPARVTGGVFLVDKNPHNTTESRLVVDFSQFSRGSTHVSWPKFAVPNLQSL
TNLLSSNLSTFGRKLHLYSHPIILGFRKIPMGVGLSPFLLAQFTSAICSVVRRA
FPHCLAFSYMDDVVLGAKSVQHLESLFTSITNFLLSLGIHLNPNKTKRWGYS
LNFMGYVIGSWGTLPQEHIVLKIKQCFRKLPVNRPIDWKVCQRIVGLLGFA
APFTQCGYPALMPLYACIQSKQAFTFSPTYKAFLCKQYLNLYPVARQRSGL
CQVFADATPTGWGLAIGHRAMRGTFVAPLPIHTAELLAACFARSRSGAKLI
GTDNSVVLSRKYTSFPWLLGCAANWILRGTSFVYVPSALNPAADPSAGRLG
LYRPLLHLPFRPTTGRTSLYAVSPSVPSHLPDRVHFASPLHVAWRPP (SEQ
ID NO: 8)
      3.3.4. Furin 2A (F2A) = **RKRRAPVKQTLNFDLLKLAGDVESNPGP** (SEQ ID NO: 9)
      3.3.5. Surface proteins (S) =
         3.3.5.1. PreS1 = 
MGGWSSKPRQGMGTNLSVPNPLGFFPDHQLDPAFGANSNNPDWD
FNPNKDHWPEANQVGAGAFGPGFTPPHGGLLGWSPQAQGILTTVP
AAPPPASTNRQSGRQPTPISPPLRDSHPQA (SEQ ID NO: 52)
         3.3.5.2. PreS2 = 
MQWNSTTFHQALLDPRVRGLYFPAGGSSSGTVNPVPTTASPISSIFS
RTGDPAPN (SEQ ID NO: 53)
         3.3.5.3. Surface (S) 
MENTTSGFLGPLLVLQAGFFLLTRILTIPQSLDSWWTSLNFLGGAPTC
PGQNSQSPTSNHSPTSCPPICPGYRWMCLRRFIIFLFILLLCLIFLLVLL
DYQGMLPVCPLLPGTSTTSTGPCKTCTIPAQGTSMFPSCCCTKPSDGN
CTCIPIPSSWAFARFLWEWASVRFSWLSLLVPFVQWFVGLSPTVWLS
VIWMMWYWGPSLYNILSPFLPLLPIFFCLWVYI (SEQ ID NO: 10)
4. **SIi-HBV-CPₘᵤₜPreS-S(sh)**
   4.1. SIi-HBV-CPₘᵤₜPreS-S(sh): Immunogen layout is shown in Figure 10D
   4.2. SIi-HBV-CPₘᵤₜPreS-S(sh): Amino acid sequence (SEQ ID NO: 13) 
MSLLWGGVTVLAAMLIAGQVASVVFLVKGGGPGGGMQLFHLCLIISCSCPT
VQASKLCLGWLWGMDIDPYKEFGASVELLSFLPSDFFPSIRDLLDTASALYRE
 
ALESPEHCSPHHTALRQAILCWGELMNLATWVGSNLEDPASRELVVSYVNV
NMGLKIRQLLWFHISCLTFGRETVLEYLVSFGVWIRTPPAYRPPNAPILSTLPE
TTVVRRRGRSPRRRTPSPRRRRSQSPRRRRSQSRESQCGGGSGGGMPLSYQH
FRKLLLLDDEAGPLEEELPRLADEGLNRRVAEDLNLGNLNVSIPWTHKVGNF
TGLASSTVPVFNPEWQTPSFPHIHLQEDIINRCQQYVGPLTVNEKRRLKLIMPA
RFYPNLTKYLPLDKGIKPYYPEHAVNHYFKTRHYLHTLWKAGILYKRETTRS
ASFCGSPYSWEQELQHGRLVFQTSTRHGDESFCSQSSGILSRSPVGPCVRSQL
KQSRLGLQPQQGSLARGKSGRSGSIRARVHPTTRRSFGVEPSGSGHIDNSASST
SSCLHQSAVRKTAYSHLSTSKRQSSSGHAVELHNIPPSSARSQSEGPIFSAWWL
QFRNSKPASDYALTHIVNLLEDWGPATEHGEHNIRIPRTPARVTGGVFLVDKN
PHNTTESRLVVDFSQFSRGSTHVSWPKFAVPNLQSLTNLLSSNLSTFGRKLHL
YSHPIILGFRKIPMGVGLSPFLLAQFTSAICSVVRRAFPHCLAFSYMDDVVLGA
KSVQHLESLFTSITNFLLSLGIHLNPNKTKRWGYSLNFMGYVIGSWGTLPQEH
IVLKIKQCFRKLPVNRPIDWKVCQRIVGLLGFAAPFTQCGYPALMPLYACIQS
KQAFTFSPTYKAFLCKQYLNLYPVARQRSGLCQVFADATPTGWGLAIGHRA
MRGTFVAPLPIHTAELLAACFARSRSGAKLIGTDNSVVLSRKYTSFPWLLGCA
ANWILRGTSFVYVPSALNPAADPSAGRLGLYRPLLHLPFRPTTGRTSLYAVSP
SVPSHLPDRVHFASPLHVAWRPPKSPNSNNPDWDFNPNKDHWPEANQVGAG
AFGPGFTPPHGGLLGWSPQAQGILTTVPAAPPPASTNRQSGRQPTPISPPLRDS
HPQAMQWNSTTFHQALLDPRVRGLYFPAGGSSSGTVNPVPTTASPISSIFSRT
GDPAPNGSKGKRKRRAPVKQTLNFDLLKLAGDVESNPGPMGGWSSKPRQG
MGTNLSVPNPLGFFPDHQLDPAFGANSNNPDWDFNPNKDHWPEANQVGLEG
GSGGMENTTSGFLGPLLVLQAGFFLLTRILTIPQSLDSWWTSLNFLGGAPTCP
GQNSQSPTSNHSPTSCPPICPGYRWMCLRRFIIFLFILLLCLIFLLVLLDYQGML
PVCPLLPGTSTTSTGPCKTCTIPAQGTSMFPSCCCTKPSDGNCTCIPIPSSWAFA
RFLWEWASVRFSWLSLLVPFVQWFVGLSPTVWLSVIWMMWYWGPSLYNILS
PFLPLLPIFFCLWVYI
   4.3. **SIi-HBV-CPₘᵤₜPreS-S(sh): Description for amino acid sequences of HBV immunogen**
      4.3.1. First amino acid of polypeptide = M
      4.3.2. Shark Invariant chain (SIi) = SLLWGGVTVLAAMLIAGQVASVVFLV (SEQ ID NO: 4)
      4.3.3. Linker = **KGGGPGGG** (SEQ ID NO: 5)
      4.3.4. C =
         4.3.4.1. PreCore =
            MQLFHLCLIISCSCPTVQASKLCLGWLWG (SEQ ID NO: 16)
         4.3.4.2. Core = MDIDPYKEFGASVELLSFLPSDFFPSIRDLLDTASALYREALESP 
EHCSPHHTALRQAILCWGELMNLATWVGSNLEDPASRELVVSY
VNVNMGLKIRQLLWFHISCLTFGRETVLEYLVSFGVWIRTPPAY
RPPNAPILSTLPETTVVRRRGRSPRRRTPSPRRRRSQSPRRRRSQS
RESQC (SEQ ID NO: 6)
      4.3.5. Linker = **GGGSGGG** (SEQ ID NO: 7)
      4.3.6. Pmut = (mutations: Y63A, C323A, C334A, C338A, C352A, R714A, D788A, R792A) 
MPLSYQHFRKLLLLDDEAGPLEEELPRLADEGLNRRVAEDLNLGNLNVSIP
WTHKVGNFTGLASSTVPVFNPEWQTPSFPHIHLQEDIINRCQQYVGPLTVNE
KRRLKLIMPARFYPNLTKYLPLDKGIKPYYPEHAVNHYFKTRHYLHTLWKA
GILYKRETTRSASFCGSPYSWEQELQHGRLVFQTSTRHGDESFCSQSSGILSR
SPVGPCVRSQLKQSRLGLQPQQGSLARGKSGRSGSIRARVHPTTRRSFGVEP
SGSGHIDNSASSTSSCLHQSAVRKTAYSHLSTSKRQSSSGHAVELHNIPPSSA
RSQSEGPIFSAWWLQFRNSKPASDYALTHIVNLLEDWGPATEHGEHNIRIPR
TPARVTGGVFLVDKNPHNTTESRLVVDFSQFSRGSTHVSWPKFAVPNLQSL
TNLLSSNLSTFGRKLHLYSHPIILGFRKIPMGVGLSPFLLAQFTSAICSVVRRA
FPHCLAFSYMDDVVLGAKSVQHLESLFTSITNFLLSLGIHLNPNKTKRWGYS
LNFMGYVIGSWGTLPQEHIVLKIKQCFRKLPVNRPIDWKVCQRIVGLLGFA
APFTQCGYPALMPLYACIQSKQAFTFSPTYKAFLCKQYLNLYPVARQRSGL
CQVFADATPTGWGLAIGHRAMRGTFVAPLPIHTAELLAACFARSRSGAKLI
GTDNSVVLSRKYTSFPWLLGCAANWILRGTSFVYVPSALNPAADPSAGRLG
LYRPLLHLPFRPTTGRTSLYAVSPSVPSHLPDRVHFASPLHVAWRPP (SEQ
ID NO: 8)
      4.3.7. Linker = **KSP**
      4.3.8. NΔPreS1 and PreS2 = 
NSNNPDWDFNPNKDHWPEANQVGAGAFGPGFTPPHGGLLGWSPQA
QGILTTVPAAPPPASTNRQSGRQPTPISPPLRDSHPQAMQWNSTTFHQ
ALLDPRVRGLYFPAGGSSSGTVNPVPTTASPISSIFSRTGDPAPN (SEQ
ID NO: 15)
      4.3.9. Linker = **GSKGK** (SEQ ID NO: 20)
      4.3.10. Furin 2A (F2A) = **RKRRAPVKQTLNFDLLKLAGDVESNPGP** (SEQ ID NO: 9)
      4.3.11. **S(sh)** =
         4.3.11.1. CΔPreS1 = 
MGGWSSKPRQGMGTNLSVPNPLGFFPDHQLDPAFGANSNNPD
WDFNPNKDHWPEANQVG (SEQ ID NO: 21)
         4.3.11.2. Linker = **LEGGSGG** (SEQ ID NO: 22)
         4.3.11.3. Surface (S) =

MENTTSGFLGPLLVLQAGFFLLTRILTIPQSLDSWWTSLNFLGGAPTCPGQN
SQSPTSNHSPTSCPPICPGYRWMCLRRFIIFLFILLLCLIFLLVLLDYQGMLPV
CPLLPGTSTTSTGPCKTCTIPAQGTSMFPSCCCTKPSDGNCTCIPIPSSWAFAR
FLWEWASVRFSWLSLLVPFVQWFVGLSPTVWLSVIWMMWYWGPSLYNIL
SPFLPLLPIFFCLWVYI (SEQ ID NO: 18)

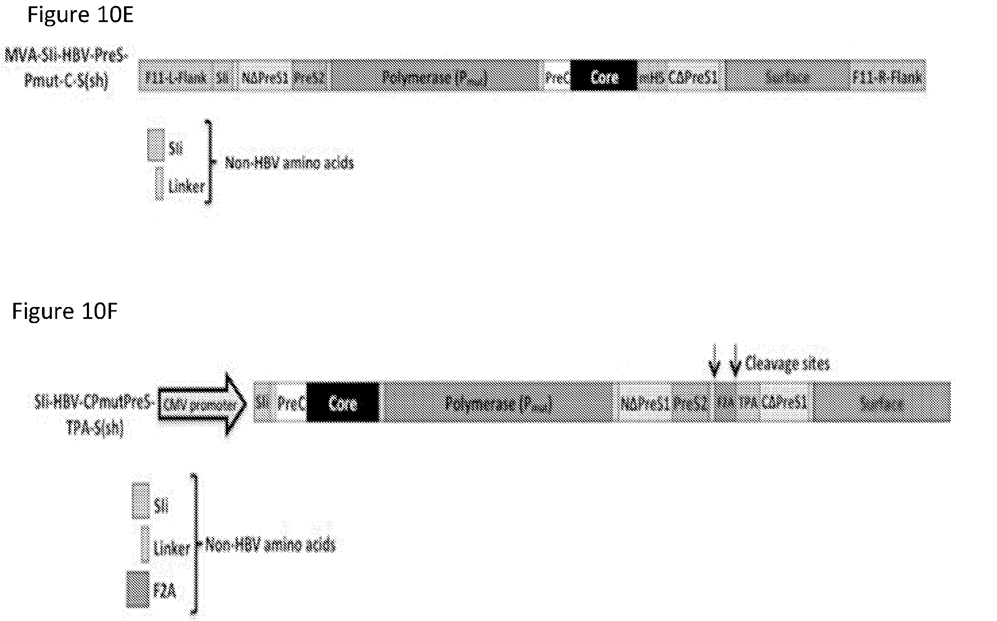

The SIi-HBV-CPmutPreS-S(sh) nucleotide sequence is provided as SEQ ID NO: 48.
5. **MVA-SIi-HBV-PreS-Pₘᵤₜ-C-S(sh)**
   **5.1. MVA-SIi-HBV-PreS-Pₘᵤₜ-C-S(sh): Immunogen layout is shown in** **Figure 10E**
   **5.2. MVA-SIi-HBV-PreS-Pₘᵤₜ-C-S(sh): Amino acid sequence (SEQ ID NO: 23)** 
MSLLWGGVTVLAAMLIAGQVASVVFLVSKSGPPSGKSNSNNPDWDFNPNKDHWPEAN
QVGAGAFGPGFTPPHGGLLGWSPQAQGILTTVPAAPPPASTNRQSGRQPTPISPPLRDSHP
QAMQWNSTTFHQALLDPRVRGLYFPAGGSSSGTVNPVPTTASPISSIFSRTGDPAPNGSKS
GSKMPLSYQHFRKLLLLDDEAGPLEEELPRLADEGLNRRVAEDLNLGNLNVSIPWTHKV
GNFTGLASSTVPVFNPEWQTPSFPHIHLQEDIINRCQQYVGPLTVNEKRRLKLIMPARFYP
NLTKYLPLDKGIKPYYPEHAVNHYFKTRHYLHTLWKAGILYKRETTRSASFCGSPYSWE
QELQHGRLVFQTSTRHGDESFCSQSSGILSRSPVGPCVRSQLKQSRLGLQPQQGSLARGKS
GRSGSIRARVHPTTRRSFGVEPSGSGHIDNSASSTSSCLHQSAVRKTAYSHLSTSKRQSSSG
HAVELHNIPPSSARSQSEGPIFSAWWLQFRNSKPASDYALTHIVNLLEDWGPATEHGEHNI
RIPRTPARVTGGVFLVDKNPHNTTESRLVVDFSQFSRGSTHVSWPKFAVPNLQSLTNLLSS
NLSWLSLDVSAAFYHIPLHPAAMPHLLVGSSGLPRYVARLSSTSRNINYQHGTMQDLHDS
CSRNLYVSLLLLYKTFGRKLHLYSHPIILGFRKIPMGVGLSPFLLAQFTSAICSVVRRAFPH
CLAFSYMDDVVLGAKSVQHLESLFTSITNFLLSLGIHLNPNKTKRWGYSLNFMGYVIGSW
GTLPQEHIVLKIKQCFRKLPVNRPIDWKVCQRIVGLLGFAAPFTQCGYPALMPLYACIQSK
QAFTFSPTYKAFLCKQYLNLYPVARQRSGLCQVFADATPTGWGLAIGHRAMRGTFVAPL
PIHTAELLAACFARSRSGAKLIGTDNSVVLSRKYTSFPWLLGCAANWILRGTSFVYVPSAL
NPAADPSAGRLGLYRPLLHLPFRPTTGRTSLYAVSPSVPSHLPDRVHFASPLHVAWRPPSK
SPGSGPPMQLFHLCLIISCSCPTVQASKLCLGWLWGMDIDPYKEFGASVELLSFLPSDFFP
SIRDLLDTASALYREALESPEHCSPHHTALRQAILCWGELMNLATWVGSNLEDPASRELV
VSYVNVNMGLKIRQLLWFHISCLTFGRETVLEYLVSFGVWIRTPPAYRPPNAPILSTLPET
TVVRRRGRSPRRRTPSPRRRRSQSPRRRRSQSRESQC

MGGWSSKPRQGMGTNLSVPNPLGFFPDHQLDPAFGANSNNPDWDFNPNKDHWPEANQV
GASKGGKSGMENTTSGFLGPLLVLQAGFFLLTRILTIPQSLDSWWTSLNFLGGAPTCPGQ
NSQSPTSNHSPTSCPPICPGYRWMCLRRFIIFLFILLLCLIFLLVLLDYQGMLPVCPLLPGTS
TTSTGPCKTCTIPAQGTSMFPSCCCTKPSDGNCTCIPIPSSWAFARFLWEWASVRFSWLSL
LVPFVQWFVGLSPTVWLSVIWMMWYWGPSLYNILSPFLPLLPIFFCLWVYI
   5.3. **MVA-SIi-HBV-PreS-Pₘᵤₜ-C-S(sh): Description for amino acid sequences of HBV immunogen**
      5.3.1.1. First amino acid of polypeptide = M
      5.3.1.2. Shark Invariant chain (SIi) =
         SLLWGGVTVLAAMLIAGQVASVVFLV (SEQ ID NO: 4)
      5.3.1.3. Linker = **SKSGPPSGKS** (SEQ ID NO: 31)
      5.3.1.4. NΔPreS1 and PreS2 = 
NSNNPDWDFNPNKDHWPEANQVGAGAFGPGFTPPHGGLLGWS
PQAQGILTTVPAAPPPASTNRQSGRQPTPISPPLRDSHPQAMQW
NSTTFHQALLDPRVRGLYFPAGGSSSGTVNPVPTTASPISSIFSRT
GDPAPN (SEQ ID NO: 15)
      5.3.1.5. Linker = **GSKSGSK** (SEQ ID NO: 32)
      5.3.1.6. Pmut = (mutations: Y63A, C323A, C334A, C338A, C352A, R714A, D788A, R792A) 
MPLSYQHFRKLLLLDDEAGPLEEELPRLADEGLNRRVAEDLNL
GNLNVSIPWTHKVGNFTGLASSTVPVFNPEWQTPSFPHIHLQED
IINRCQQYVGPLTVNEKRRLKLIMPARFYPNLTKYLPLDKGIKP
YYPEHAVNHYFKTRHYLHTLWKAGILYKRETTRSASFCGSPYS
WEQELQHGRLVFQTSTRHGDESFCSQSSGILSRSPVGPCVRSQL
KQSRLGLQPQQGSLARGKSGRSGSIRARVHPTTRRSFGVEPSGS
GHIDNSASSTSSCLHQSAVRKTAYSHLSTSKRQSSSGHAVELHN
IPPSSARSQSEGPIFSAWWLQFRNSKPASDYALTHIVNLLEDWG
PATEHGEHNIRIPRTPARVTGGVFLVDKNPHNTTESRLVVDFSQ
FSRGSTHVSWPKFAVPNLQSLTNLLSSNLSTFGRKLHLYSHPIIL
GFRKIPMGVGLSPFLLAQFTSAICSVVRRAFPHCLAFSYMDDVV
LGAKSVQHLESLFTSITNFLLSLGIHLNPNKTKRWGYSLNFMGY
VIGSWGTLPQEHIVLKIKQCFRKLPVNRPIDWKVCQRIVGLLGF
AAPFTQCGYPALMPLYACIQSKQAFTFSPTYKAFLCKQYLNLYP
VARQRSGLCQVFADATPTGWGLAIGHRAMRGTFVAPLPIHTAE
LLAACFARSRSGAKLIGTDNSVVLSRKYTSFPWLLGCAANWILR
GTSFVYVPSALNPAADPSAGRLGLYRPLLHLPFRPTTGRTSLYA
VSPSVPSHLPDRVHFASPLHVAWRPP (SEQ ID NO: 8)
      5.3.1.7. Linker = **SKSPGSGPP** (SEQ ID NO: 33)
      5.3.1.8. C =
         5.3.1.8.1. PreCore
            MQLFHLCLIISCSCPTVQASKLCLGWLWG (SEQ ID NO: 16)
         5.3.1.8.2. Core = 
MDIDPYKEFGASVELLSFLPSDFFPSIRDLLDTASALYREALESP
EHCSPHHTALRQAILCWGELMNLATWVGSNLEDPASRELVVSY
VNVNMGLKIRQLLWFHISCLTFGRETVLEYLVSFGVWIRTPPAY
RPPNAPILSTLPETTVVRRRGRSPRRRTPSPRRRRSQSPRRRRSQS
RESQC (SEQ ID NO: 6)
      5.3.1.9. **S(sh)**
         5.3.1.9.1. CΔPreS1 = 
MGGWSSKPRQGMGTNLSVPNPLGFFPDHQLDPAFGANSNN
PDWDFNPNKDHWPEANQVG
         5.3.1.9.2. Linker =
            **ASKGGKSG** (SEQ ID NO: 34)
         5.3.1.9.3. Surface = 
MENTTSGFLGPLLVLQAGFFLLTRILTIPQSLDSWWTSLNFL
GGAPTCPGQNSQSPTSNHSPTSCPPICPGYRWMCLRRFIIFLF
ILLLCLIFLLVLLDYQGMLPVCPLLPGTSTTSTGPCKTCTIPA
 
QGTSMFPSCCCTKPSDGNCTCIPIPSSWAFARFLWEWASVR
FSWLSLLVPFVQWFVGLSPTVWLSVIWMMWYWGPSLYNI
LSPFLPLLPIFFCLWVYI (SEQ ID NO: 18)
   5.4. Nucleotide sequence of MVA-SIi-HBV-PreS-Pₘᵤₜ-C-S(sh) is provided as SEQ ID NO: 24.
   5.5. Description for nucleotide sequences of MVA-SIi-HBV-PreS-Pₘᵤₜ-C-S(sh):
      5.5.1. F11-L-Flank = bases 1 - 1097 (SEQ ID NO: 35)
      5.5.2. SIi-HBV-PreS-Pₘᵤₜ-C = bases 1098 - 4838 (SEQ ID NO: 37)
      5.5.3. Transcription terminator sequence = bases 4839 - 4845
         TTTTTGT
      5.5.4. mH5 promoter = bases 4846 - 4942 (SEQ ID NO: 28)
      5.5.5. S(sh) = bases 4943 - 5824 (SEQ ID NO: 38)
      5.5.6. F11-R-Flank = bases 5825 - 7143 (SEQ ID NO: 36)
6. **SIi-HBV-CPₘᵤₜPreS-TPA-S(sh)**
   6.1. SIi-HBV-CPₘᵤₜPreS-TPA-S(sh): Immunogen layout is shown in Figure 10F
   6.2. SIi-HBV-CPₘᵤₜPreS-TPA-S(sh): Amino acid sequence (SEQ ID NO: 25) 
MSLLWGGVTVLAAMLIAGQVASVVFLVKGGGPGGGMQLFHLCLIISCSCPT
VQASKLCLGWLWGMDIDPYKEFGASVELLSFLPSDFFPSIRDLLDTASALYRE
ALESPEHCSPHHTALRQAILCWGELMNLATWVGSNLEDPASRELVVSYVNV
NMGLKIRQLLWFHISCLTFGRETVLEYLVSFGVWIRTPPAYRPPNAPILSTLPE
TTVVRRRGRSPRRRTPSPRRRRSQSPRRRRSQSRESQCGGGSGGGMPLSYQH
FRKLLLLDDEAGPLEEELPRLADEGLNRRVAEDLNLGNLNVSIPWTHKVGNF
TGLASSTVPVFNPEWQTPSFPHIHLQEDIINRCQQYVGPLTVNEKRRLKLIMPA
RFYPNLTKYLPLDKGIKPYYPEHAVNHYFKTRHYLHTLWKAGILYKRETTRS
ASFCGSPYSWEQELQHGRLVFQTSTRHGDESFCSQSSGILSRSPVGPCVRSQL
KQSRLGLQPQQGSLARGKSGRSGSIRARVHPTTRRSFGVEPSGSGHIDNSASST
SSCLHQSAVRKTAYSHLSTSKRQSSSGHAVELHNIPPSSARSQSEGPIFSAWWL
QFRNSKPASDYALTHIVNLLEDWGPATEHGEHNIRIPRTPARVTGGVFLVDKN
PHNTTESRLVVDFSQFSRGSTHVSWPKFAVPNLQSLTNLLSSNLSTFGRKLHL
YSHPIILGFRKIPMGVGLSPFLLAQFTSAICSVVRRAFPHCLAFSYMDDVVLGA
KSVQHLESLFTSITNFLLSLGIHLNPNKTKRWGYSLNFMGYVIGSWGTLPQEH
IVLKIKQCFRKLPVNRPIDWKVCQRIVGLLGFAAPFTQCGYPALMPLYACIQS
KQAFTFSPTYKAFLCKQYLNLYPVARQRSGLCQVFADATPTGWGLAIGHRA
MRGTFVAPLPIHTAELLAACFARSRSGAKLIGTDNSVVLSRKYTSFPWLLGCA
 
ANWILRGTSFVYVPSALNPAADPSAGRLGLYRPLLHLPFRPTTGRTSLYAVSP
SVPSHLPDRVHFASPLHVAWRPPKSPNSNNPDWDFNPNKDHWPEANQVGAG
AFGPGFTPPHGGLLGWSPQAQGILTTVPAAPPPASTNRQSGRQPTPISPPLRDS
HPQAMQWNSTTFHQALLDPRVRGLYFPAGGSSSGTVNPVPTTASPISSIFSRT
GDPAPNGSKGKRKRRAPVKQTLNFDLLKLAGDVESNPGPMDAMKRGLCC
VLLLCGAVFVSPSQEIHARFRRMGGWSSKPRQGMGTNLSVPNPLGFFPDHQL
DPAFGANSNNPDWDFNPNKDHWPEANQVGLEGGSGGMENTTSGFLGPLLV
LQAGFFLLTRILTIPQSLDSWWTSLNFLGGAPTCPGQNSQSPTSNHSPTSCPPIC
PGYRWMCLRRFIIFLFILLLCLIFLLVLLDYQGMLPVCPLLPGTSTTSTGPCKTC
TIPAQGTSMFPSCCCTKPSDGNCTCIPIPSSWAFARFLWEWASVRFSWLSLLVP
FVQWFVGLSPTVWLSVIWMMWYWGPSLYNILSPFLPLLPIFFCLWVYI
   6.3. **SIi-HBV-CPₘᵤₜPreS-TPA-S(sh): Description for amino acid sequences of HBV immunogen**
      6.3.1. First amino acid of polypeptide = M
      6.3.2. Shark Invariant chain (SIi) = SLLWGGVTVLAAMLIAGQVASVVFLV (SEQ ID NO: 4)
      6.3.3. Linker = **KGGGPGGG** (SEQ ID NO: 5)
      6.3.4. C =
         6.3.4.1. PreCore
            MQLFHLCLIISCSCPTVQASKLCLGWLWG (SEQ ID NO: 16)
         6.3.4.2. Core = 
MDIDPYKEFGASVELLSFLPSDFFPSIRDLLDTASALYREALESP
EHCSPHHTALRQAILCWGELMNLATWVGSNLEDPASRELVVSY
VNVNMGLKIRQLLWFHISCLTFGRETVLEYLVSFGVWIRTPPAY
RPPNAPILSTLPETTVVRRRGRSPRRRTPSPRRRRSQSPRRRRSQS
RESQC (SEQ ID NO: 6)
      6.3.5. Linker = **GGGSGGG** (SEQ ID NO: 7)
      6.3.6. Pmut = (mutations: Y63A, C323A, C334A, C338A, C352A, R714A, D788A, R792A) 
MPLSYQHFRKLLLLDDEAGPLEEELPRLADEGLNRRVAEDLNLGNLNVSIP
WTHKVGNFTGLASSTVPVFNPEWQTPSFPHIHLQEDIINRCQQYVGPLTVNE
KRRLKLIMPARFYPNLTKYLPLDKGIKPYYPEHAVNHYFKTRHYLHTLWKA
GILYKRETTRSASFCGSPYSWEQELQHGRLVFQTSTRHGDESFCSQSSGILSR
SPVGPCVRSQLKQSRLGLQPQQGSLARGKSGRSGSIRARVHPTTRRSFGVEP
SGSGHIDNSASSTSSCLHQSAVRKTAYSHLSTSKRQSSSGHAVELHNIPPSSA
RSQSEGPIFSAWWLQFRNSKPASDYALTHIVNLLEDWGPATEHGEHNIRIPR
TPARVTGGVFLVDKNPHNTTESRLVVDFSQFSRGSTHVSWPKFAVPNLQSL
TNLLSSNLSTFGRKLHLYSHPIILGFRKIPMGVGLSPFLLAQFTSAICSVVRRA
FPHCLAFSYMDDVVLGAKSVQHLESLFTSITNFLLSLGIHLNPNKTKRWGYS
LNFMGYVIGSWGTLPQEHIVLKIKQCFRKLPVNRPIDWKVCQRIVGLLGFA
APFTQCGYPALMPLYACIQSKQAFTFSPTYKAFLCKQYLNLYPVARQRSGL
CQVFADATPTGWGLAIGHRAMRGTFVAPLPIHTAELLAACFARSRSGAKLI
GTDNSVVLSRKYTSFPWLLGCAANWILRGTSFVYVPSALNPAADPSAGRLG
LYRPLLHLPFRPTTGRTSLYAVSPSVPSHLPDRVHFASPLHVAWRPP (SEQ
ID NO: 8)
      6.3.7. Linker = **KSP**
      6.3.8. NΔPreS1 and PreS2 = 
NSNNPDWDFNPNKDHWPEANQVGAGAFGPGFTPPHGGLLGWSPQA
QGILTTVPAAPPPASTNRQSGRQPTPISPPLRDSHPQAMQWNSTTFHQ
ALLDPRVRGLYFPAGGSSSGTVNPVPTTASPISSIFSRTGDPAPN (SEQ
ID NO: 15)
      6.3.9. Linker = **GSKGK** (SEQ ID NO: 20)
      6.3.10. Furin 2A (F2A) = **RKRRAPVKQTLNFDLLKLAGDVESNPGP** (SEQ ID NO: 9)
      6.3.11. TPA = MDAMKRGLCCVLLLCGAVFVSPSQEIHARFRR (SEQ ID NO: 30)
      6.3.12. S(sh) =
         6.3.12.1. CΔPreS1 = 
MGGWSSKPRQGMGTNLSVPNPLGFFPDHQLDPAFGANSNNPD
WDFNPNKDHWPEANQVG (SEQ ID NO: 21)
         6.3.12.2. Linker = **LEGGSGG** (SEQ ID NO: 22)
         6.3.12.3. Surface (S) =

MENTTSGFLGPLLVLQAGFFLLTRILTIPQSLDSWWTSLNFLGGAPTCPGQN
SQSPTSNHSPTSCPPICPGYRWMCLRRFIIFLFILLLCLIFLLVLLDYQGMLPV
CPLLPGTSTTSTGPCKTCTIPAQGTSMFPSCCCTKPSDGNCTCIPIPSSWAFAR
FLWEWASVRFSWLSLLVPFVQWFVGLSPTVWLSVIWMMWYWGPSLYNIL
SPFLPLLPIFFCLWVYI (SEQ ID NO: 18)

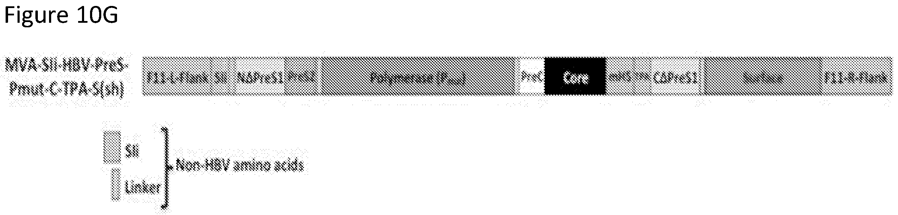

The SIi-HBV-CPmutPreS-TPA-S(sh) nucleotide sequence is provided as SEQ ID NO: 49.
7. **MVA-SIi-HBV-PreS-Pₘᵤₜ-C-TPA-S(sh)**
   **7.1. MVA-SIi-HBV-PreS-Pₘᵤₜ-C-TPA-S(sh): Immunogen layout is shown in** **Figure 10G**
   **7.2. MVA-SIi-HBV-PreS-Pₘᵤₜ-C-TPA-S(sh): Amino acid sequence (SEQ ID NO: 26)** 
MSLLWGGVTVLAAMLIAGQVASVVFLVSKSGPPSGKSNSNNPDWDFNPNKDHWPEAN
QVGAGAFGPGFTPPHGGLLGWSPQAQGILTTVPAAPPASTNRQSGRQPTPISPPLRDSHP
QAMQWNSTTFHQALLDPRVRGLYFPAGGSSSGTVNPVPTTASPISSIFSRTGDPAPNGSKS
GSKMPLSYQHFRKLLLLDDEAGPLEEELPRLADEGLNRRVAEDLNLGNLNVSIPWTHKV
GNFTGLASSTVPVFNPEWQTPSFPHIHLQEDIINRCQQYVGPLTVNEKRRLKLIMPARFYP
NLTKYLPLDKGIKPYYPEHAVNHYFKTRHYLHTLWKAGILYKRETTRSASFCGSPYSWE
QELQHGRLVFQTSTRHGDESFCSQSSGILSRSPVGPCVRSQLKQSRLGLQPQQGSLARGKS
GRSGSIRARVHPTTRRSFGVEPSGSGHIDNSASSTSSCLHQSAVRKTAYSHLSTSKRQSSSG
HAVELHNIPPSSARSQSEGPIFSAWWLQFRNSKPASDYALTHIVNLLEDWGPATEHGEHNI
RIPRTPARVTGGVFLVDKNPHNTTESRLVVDFSQFSRGSTHVSWPKFAVPNLQSLTNLLSS
NLSWLSLDVSAAFYHIPLHPAAMPHLLVGSSGLPRYVARLSSTSRNINYQHGTMQDLHDS
 
CSRNLYVSLLLLYKTFGRKLHLYSHPIILGFRKIPMGVGLSPFLLAQFTSAICSVVRRAFPH
CLAFSYMDDVVLGAKSVQHLESLFTSITNFLLSLGIHLNPNKTKRWGYSLNFMGYVIGSW
GTLPQEHIVLKIKQCFRKLPVNRPIDWKVCQRIVGLLGFAAPFTQCGYPALMPLYACIQSK
QAFTFSPTYKAFLCKQYLNLYPVARQRSGLCQVFADATPTGWGLAIGHRAMRGTFVAPL
PIHTAELLAACFARSRSGAKLIGTDNSVVLSRKYTSFPWLLGCAANWILRGTSFVYVPSAL
NPAADPSAGRLGLYRPLLHLPFRPTTGRTSLYAVSPSVPSHLPDRVHFASPLHVAWRPPSK
SPGSGPPMQLFHLCLIISCSCPTVQASKLCLGWLWGMDIDPYKEFGASVELLSFLPSDFFP
SIRDLLDTASALYREALESPEHCSPHHTALRQAILCWGELMNLATWVGSNLEDPASRELV
VSYVNVNMGLKIRQLLWFHISCLTFGRETVLEYLVSFGVWIRTPPAYRPPNAPILSTLPET
TVVRRRGRSPRRRTPSPRRRRSQSPRRRRSQSRESQCMDAMKRGLCCVLLLCGAVFVSPS
QEIHARFRRMGGWSSKPRQGMGTNLSVPNPLGFFPDHQLDPAFGANSNNPDWDFNPNKD
HWPEANQVGASKGGKSGMENTTSGFLGPLLVLQAGFFLLTRILTIPQSLDSWWTSLNFL
GGAPTCPGQNSQSPTSNHSPTSCPPICPGYRWMCLRRFIIFLFILLLCLIFLLVLLDYQGMLP
VCPLLPGTSTTSTGPCKTCTIPAQGTSMFPSCCCTKPSDGNCTCIPIPSSWAFARFLWEWAS
VRFSWLSLLVPFVQWFVGLSPTVWLSVIWMMWYWGPSLYNILSPFLPLLPIFFCLWVYI
   7.3. **MVA-SIi-HBV-PreS-Pₘᵤₜ-C-TPA-S(sh): Description for amino acid sequences of HBV immunogen**
      7.3.1.1. First amino acid of polypeptide = M
      7.3.1.2. Shark Invariant chain (SIi) =
         SLLWGGVTVLAAMLIAGQVASVVFLV (SEQ ID NO: 4)
      7.3.1.3. Linker = **SKSGPPSGKS** (SEQ ID NO: 31)
      7.3.1.4. NΔPreS1 and PreS2 = 
NSNNPDWDFNPNKDHWPEANQVGAGAFGPGFTPPHGGLLGWS
PQAQGILTTVPAAPPPASTNRQSGRQPTPISPPLRDSHPQAMQW
NSTTFHQALLDPRVRGLYFPAGGSSSGTVNPVPTTASPISSIFSRT
GDPAPN (SEQ ID NO: 15)
      7.3.1.5. Linker = **GSKSGSK** (SEQ ID NO: 32)
      7.3.1.6. Pmut = (mutations: Y63A, C323A, C334A, C338A, C352A, R714A, D788A, R792A) 
MPLSYQHFRKLLLLDDEAGPLEEELPRLADEGLNRRVAEDLNL
GNLNVSIPWTHKVGNFTGLASSTVPVFNPEWQTPSFPHIHLQED
IINRCQQYVGPLTVNEKRRLKLIMPARFYPNLTKYLPLDKGIKP
YYPEHAVNHYFKTRHYLHTLWKAGILYKRETTRSASFCGSPYS
WEQELQHGRLVFQTSTRHGDESFCSQSSGILSRSPVGPCVRSQL
KQSRLGLQPQQGSLARGKSGRSGSIRARVHPTTRRSFGVEPSGS
GHIDNSASSTSSCLHQSAVRKTAYSHLSTSKRQSSSGHAVELHN
IPPSSARSQSEGPIFSAWWLQFRNSKPASDYALTHIVNLLEDWG
PATEHGEHNIRIPRTPARVTGGVFLVDKNPHNTTESRLVVDFSQ
FSRGSTHVSWPKFAVPNLQSLTNLLSSNLSTFGRKLHLYSHPIIL
GFRKIPMGVGLSPFLLAQFTSAICSVVRRAFPHCLAFSYMDDVV
 
LGAKSVQHLESLFTSITNFLLSLGIHLNPNKTKRWGYSLNFMGY
VIGSWGTLPQEHIVLKIKQCFRKLPVNRPIDWKVCQRIVGLLGF
AAPFTQCGYPALMPLYACIQSKQAFTFSPTYKAFLCKQYLNLYP
VARQRSGLCQVFADATPTGWGLAIGHRAMRGTFVAPLPIHTAE
LLAACFARSRSGAKLIGTDNSVVLSRKYTSFPWLLGCAANWILR
GTSFVYVPSALNPAADPSAGRLGLYRPLLHLPFRPTTGRTSLYA
VSPSVPSHLPDRVHFASPLHVAWRPP (SEQ ID NO: 8)
      7.3.1.7. Linker = **SKSPGSGPP** (SEQ ID NO: 33)
      7.3.1.8. C =
         7.3.1.8.1. PreCore =
            MQLFHLCLIISCSCPTVQASKLCLGWLWG (SEQ ID NO: 16)
         7.3.1.8.2. Core = 
MDIDPYKEFGASVELLSFLPSDFFPSIRDLLDTASALYREALESP
EHCSPHHTALRQAILCWGELMNLATWVGSNLEDPASRELVVSY
VNVNMGLKIRQLLWFHISCLTFGRETVLEYLVSFGVWIRTPPAY
RPPNAPILSTLPETTVVRRRGRSPRRRTPSPRRRRSQSPRRRRSQS
RESQC (SEQ ID NO: 6)
      7.3.1.9. TPA = MDAMKRGLCCVLLLCGAVFVSPSQEIHARFRR (SEQ ID NO: 30)
      7.3.1.10. S(sh)_ =
         7.3.1.10.1. CΔPreS1 = 
MGGWSSKPRQGMGTNLSVPNPLGFFPDHQLDPAFGANSNNPD
WDFNPNKDHWPEANQVG (SEQ ID NO: 21)
         7.3.1.10.2. Linker = **ASKGGKSG** (SEQ ID NO: 34)
         7.3.1.10.3. Surface (S) = 
MENTTSGFLGPLLVLQAGFFLLTRILTIPQSLDSWWTSLNF
LGGAPTCPGQNSQSPTSNHSPTSCPPICPGYRWMCLRRFIIF
LFILLLCLIFLLVLLDYQGMLPVCPLLPGTSTTSTGPCKTCTI
PAQGTSMFPSCCCTKPSDGNCTCIPIPSSWAFARFLWEWAS
VRFSWLSLLVPFVQWFVGLSPTVWLSVIWMMWYWGPSL
YNILSPFLPLLPIFFCLWVYI (SEQ ID NO: 18)
   7.4. Nucleotide sequences of MVA-SIi-HBV-PreS-Pₘᵤₜ-C-TPA-S(sh) is provided as SEQ ID NO: 27
   7.5. Description for nucleotide sequences of MVA-SIi-HBV-PreS-Pₘᵤₜ-C-TPA-S(sh):
      7.5.1. F11-L-Flank = bases 1 - 1097 (SEQ ID NO: 35)
      7.5.2. SIi-HBV-PreS-Pₘᵤₜ-C = bases 1098 - 4838 (SEQ ID NO: 37)
      7.5.3. Transcription terminator sequence = bases 4839 - 4845
         TTTTTGT
      7.5.4. mH5 promoter = bases 4846 - 4942 (SEQ ID NO: 28)
      7.5.5. TPA = bases 4943 - 5038 (SEQ ID NO: 29)
      7.5.6. S(sh) = bases 5039 - 5920 (SEQ ID NO: 38)
      7.5.7. F11-R-Flank = bases 5921 - 7239 (SEQ ID NO: 36)
8. Nucleotide sequences of low GC content version MVA-Sli-HBV-PreS-Pmut-C-TPA-S(sh) (SEQ ID NO: 58): 
gtaatctattcgatataccgttgctaacagtatactggcccaataactgtggatggaaaatctataataatacattaatatc
atccgatggtgctagggttatttggatggatgcgtataaattttcttgcggtttatctttacaagactattgttatcattgggg
tagcaaaccagagagccgaccattcgatttaataaaaaaatcagatgctaaacgcaattctaaatcgttggtcaaagaat
ctatggcatccttgaaatccttgtacgaggcattcgagacacaatcaggagcgttagaagttttaatgagtccatgtagga
tgttttcgttttctagaatagaagacatgttcttaactagtgtcattaatagagtatccgagaatactggaatggggatgtat
tatcctaccaacgatataccttctctatttatcgaatcatctatctgtctagattatattatagtaaataatcaggaatccaa
caaatatcgtatcaaatctgttctcgatatcatttcttcaaaacaataccctgcaggacgtcccaactacgttaaaaatggt
acaaaaggaaagttatatatcgcgttgtgtaaagttaccgtacctactaacgaccatattccagtagtttatcacgatgatg
acaatactaccacctttattacagtattgacgtccgtcgatattgaaactgctatcagagcaggatattcgatagtcgaatt
aggggctttacaatgggataataatattccagaacttaaaaacggtttactggatagtatcaagatgatttatgacttgaa
cgcagttacaacaaataatttattggaacagctcatagaaaatattaactttaacaactctagtataatttcgttgttttata
catttgccattagttattgccgagcattcatttactcaattatggaaaccatagatccggtgtatatatctcagttcagttata
aagaattatacgttagtagctcttataaagatattaatgaatccatgagtcagatggtaaaattataaaaagtgaaaaac
aatattatttttatcgttggttgttacactATGTCACTTCTTTGGGGCGGAGTTACAGTTCTTGCTGCTATG
CTTATTGCTGGACAAGTTGCTTCTGTTGTGTTTCTTGTTTCTAAATCTGGACCTCCTTCTGGAAA
ATCTAATTCTAACAATCCTGATTGGGATTTCAATCCTAACAAAGATCATTGGCCTGAAGCTAATC
AAGTTGGAGCTGGTGCTTTTGGACCTGGTTTTACACCTCCTCATGGTGGATTGCTTGGATGGTC
ACCTCAAGCTCAGGGAATTCTTACAACAGTTCCAGCTGCTCCTCCTCCTGCTTCTACAAATAGAC
AATCTGGTAGACAACCTACACCTATTTCTCCACCTCTTAGAGATTCTCATCCTCAAGCTATGCAA
TGGAATTCTACTACATTTCATCAAGCTTTGCTTGATCCTAGAGTTAGAGGACTTTATTTTCCTGC
TGGTGGAAGTTCTTCTGGAACAGTTAATCCTGTTCCTACAACAGCTTCTCCAATTTCTTCTATAT
TTTCTAGAACAGGCGATCCTGCTCCTAATGGATCAAAATCTGGATCAAAAATGCCTCTGTCTTAT
CAACACTTTAGAAAATTGCTGCTTCTTGATGATGAAGCTGGACCTCTTGAAGAAGAATTGCCTA
GACTTGCTGATGAAGGACTTAATAGAAGAGTTGCTGAAGATCTTAATCTGGGAAATCTTAATGT
TTCTATTCCTTGGACACACAAAGTTGGAAATTTCACAGGACTTGCATCTTCTACAGTGCCTGTTT
TTAATCCTGAATGGCAAACACCTTCTTTTCCACATATTCATCTGCAAGAGGATATCATCAATAGA
TGTCAACAATATGTTGGACCACTGACAGTTAATGAGAAGAGAAGGCTTAAACTTATTATGCCTG
CTAGATTCTATCCTAATCTTACAAAGTATTTGCCTCTGGATAAGGGAATCAAACCTTATTATCCT
 
GAACATGCTGTGAATCACTACTTTAAAACAAGACATTATCTGCATACACTGTGGAAAGCTGGTA
TTCTTTACAAAAGAGAAACAACAAGATCTGCTTCATTTTGTGGATCTCCATATTCTTGGGAACAA
GAACTTCAACATGGTAGACTTGTTTTTCAAACATCTACAAGACATGGGGATGAATCATTTTGTT
CTCAAAGTTCTGGAATTCTTTCTAGATCTCCTGTTGGACCTTGTGTTAGATCTCAACTTAAACAA
TCTAGACTTGGACTTCAACCTCAACAAGGATCTCTTGCTAGAGGAAAAAGTGGAAGATCTGGA
TCTATTAGAGCTAGAGTTCATCCTACAACTAGAAGATCTTTTGGAGTTGAACCTTCTGGATCTG
GACATATTGATAATTCTGCCTCTTCTACATCTTCTTGTCTGCATCAATCTGCTGTTAGAAAGACA
GCTTATTCTCATTTGTCTACTTCTAAGAGACAATCATCTTCTGGACATGCTGTTGAACTTCATAAT
ATTCCTCCAAGTAGTGCTAGAAGTCAATCTGAAGGACCAATATTTTCAGCTTGGTGGCTTCAAT
TCAGAAATTCTAAACCTGCTTCTGATTATGCTCTGACACATATAGTTAATTTGCTTGAAGATTGG
GGACCTGCTACAGAACATGGCGAACACAATATTAGAATACCTAGAACTCCTGCTAGAGTTACA
GGCGGAGTCTTTTTGGTTGATAAGAATCCTCATAATACCACAGAATCAAGACTTGTTGTTGATT
TTTCACAGTTTTCTAGAGGATCTACACATGTTTCTTGGCCTAAATTTGCTGTTCCAAATCTTCAAT
CTCTTACAAATTTGCTTTCATCTAATCTTTCTTGGCTGTCTCTTGATGTTTCTGCTGCCTTTTATCA
TATTCCTCTTCATCCTGCTGCAATGCCTCATTTGCTTGTTGGATCATCTGGACTTCCAAGATATGT
TGCTAGACTTAGCTCTACATCTAGAAATATCAATTATCAGCATGGAACAATGCAGGATCTTCAC
GATTCTTGTAGTAGGAATCTGTATGTTTCTTTGCTTCTGCTGTATAAGACATTTGGAAGAAAACT
TCATCTGTATTCTCACCCTATTATTCTGGGTTTTAGAAAGATTCCTATGGGAGTTGGACTTTCTC
CTTTTTTGCTTGCTCAATTCACATCTGCTATTTGTTCTGTTGTTAGAAGGGCTTTTCCTCATTGTC
TTGCATTTTCTTATATGGATGATGTTGTTCTTGGAGCTAAATCTGTTCAACATCTTGAAAGTCTG
TTTACCTCTATTACTAATTTTCTGCTTTCTCTGGGAATTCATCTGAATCCAAACAAAACAAAGAG
ATGGGGATATTCTCTTAATTTCATGGGATATGTTATTGGATCTTGGGGAACACTTCCTCAAGAA
CATATCGTTTTGAAAATCAAGCAATGTTTCAGAAAACTGCCTGTGAATAGACCTATTGATTGGA
AAGTTTGTCAAAGAATTGTGGGACTTCTTGGATTTGCTGCTCCTTTTACACAATGTGGATATCCT
GCTCTTATGCCACTTTATGCTTGTATTCAATCTAAACAGGCTTTTACATTTTCTCCAACATACAAA
GCTTTTCTGTGTAAACAGTATCTGAATCTTTATCCTGTGGCTAGACAAAGATCTGGTCTTTGTCA
AGTTTTTGCTGATGCTACACCAACAGGATGGGGACTTGCTATTGGACATAGAGCTATGAGAGG
AACATTTGTTGCTCCATTGCCTATTCATACAGCTGAATTGCTTGCTGCTTGTTTTGCTAGATCTA
GAAGCGGAGCAAAACTTATTGGTACAGATAATAGTGTTGTCCTGAGTAGAAAGTACACATCTT
TTCCATGGTTGTTGGGATGTGCTGCTAATTGGATTCTTAGAGGAACTTCTTTTGTTTATGTTCCT
TCTGCTCTTAATCCTGCAGCTGATCCATCTGCTGGTAGATTGGGACTGTATAGACCACTTCTTCA
TTTGCCTTTTAGACCAACAACTGGAAGAACATCTCTTTATGCTGTTTCTCCTTCTGTTCCATCTCA
TTTGCCTGATAGAGTTCATTTTGCTTCTCCACTTCATGTTGCTTGGAGGCCACCATCTAAATCTC
 
CAGGTTCTGGACCACCTATGCAACTTTTTCATTTGTGTTTGATCATTAGCTGTTCTTGTCCTACA
GTTCAAGCTTCTAAACTTTGTCTTGGATGGCTTTGGGGAATGGATATTGATCCATACAAAGAAT
TTGGAGCTAGTGTTGAATTGCTGTCATTTCTTCCATCTGATTTTTTCCCTTCTATTCGTGATCTTC
TTGATACAGCATCTGCTCTGTATAGAGAAGCTCTTGAATCTCCTGAACACTGTTCTCCACATCAT
ACAGCACTTAGACAAGCTATTCTTTGTTGGGGAGAACTTATGAATCTTGCTACATGGGTTGGAT
CTAATTTGGAAGATCCAGCTTCTAGAGAATTGGTGGTTTCTTATGTTAATGTGAATATGGGACT
GAAAATTAGACAACTGCTTTGGTTTCATATCTCTTGTCTTACATTTGGTAGAGAAACAGTTTTGG
AATATTTGGTTTCTTTTGGCGTTTGGATTAGAACACCTCCAGCTTATAGACCTCCTAATGCTCCT
ATTTTGTCTACACTTCCTGAAACAACAGTCGTTAGAAGAAGAGGAAGATCTCCAAGAAGAAGA
ACACCAAGTCCTAGAAGAAGAAGATCTCAATCACCAAGAAGAAGAAGAAGTCAATCTAGAGA
ATCTCAATGTTGATTTTTGTTCGACATTAAAAATTGAAAATAAATACAAAGGTTCTTGAGGGTT
GTGTTAAATTGAAAGCGAGAAATAATCATAAATATGATTCAGGTGACGGATCCATGGATGCTA
TGAAGCGAGGACTTTGTTGTGTTTTGCTTCTTTGTGGTGCTGTGTTTGTTTCTCCATCTCAAGAA
ATTCATGCCAGATTCAGAAGAATGGGAGGCTGGTCATCTAAACCTAGACAAGGCATGGGAACA
AATCTTTCTGTTCCTAATCCTTTGGGATTCTTTCCTGATCATCAATTGGATCCAGCATTTGGAGC
AAATAGTAACAATCCAGATTGGGACTTTAACCCAAACAAAGATCATTGGCCAGAAGCTAATCA
AGTTGGAGCATCTAAAGGTGGAAAAAGTGGAATGGAAAACACTACATCTGGATTTCTTGGACC
TTTGCTTGTTCTTCAAGCTGGATTTTTCCTGTTGACAAGAATACTTACAATTCCTCAATCACTGG
ATTCTTGGTGGACAAGTCTTAATTTTCTTGGAGGTGCTCCTACATGTCCTGGACAAAATTCTCAA
TCTCCAACTTCTAATCATTCTCCTACATCTTGTCCTCCAATTTGTCCTGGATATAGATGGATGTGT
CTTAGAAGATTCATTATCTTTCTTTTCATACTGCTGCTGTGTCTGATTTTCCTTCTTGTTTTGTTGG
ATTATCAGGGAATGCTTCCTGTTTGTCCTTTGCTTCCTGGAACTTCTACAACAAGTACAGGACCT
TGTAAAACATGTACAATTCCAGCACAGGGAACATCTATGTTTCCAAGTTGTTGTACAAAAC
CTTCTGATGGAAATTGCACATGTATTCCTATTCCAAGTTCTTGGGCATTTGCTAGATTTCTTTGG
GAATGGGCTTCTGTTAGATTCAGTTGGTTGTCTCTTTTGGTTCCATTTGTTCAGTGGTTTGTTGG
ATTGTCTCCTACAGTTTGGCTTTCTGTTATTTGGATGATGTGGTATTGGGGACCTTCTCTTTACA
ATATTTTGAGTCCTTTTCTCCCTTTGCTGCCAATTTTCTTTTGTCTTTGGGTTTACATTTGAttaacc
gagtttctgcattattgtaattcgtatgctggcaccatcaaagaatcacttctaaaagatatcaatatcacacatacaaata
ttactaccctattgaatgagacagccaaggttatcaagttagtaaaatctctggtagataaagaagatactgatattgtga
ataatttcattaccaaagaaattaaaaacagagacaaaatagttaatagtttgtctctatcaaacctggactttcgtttgta
aattggggctTtttgtacaataaatgggtgttgccaatgattcatcccctgaatatcaatggatgtctccccatagattatca
gatactgttatattaggagactgtttgtattttaacaatataatgtcccaattagatttacaccaaaattgggctccatcagt
tagattgttaaattattttaagaattttaataaggaaacactactaaagatagaagagaatgattacattaattcatcctttt
 
tccaacaaaaggataaacgattttatcctataaacgacgatttttatcacatatctacaggaggatatggtatagtctttaa
gatagataactatgtagtaaaatttgtattcgaggccacaaaattatatagtcccatggaaactacggcggagttcacagt
acccaaatttctatacaacaatctaaagggagatgaaaaaaaattaatcgtgtgtgcgtgggccatgggattaaactata
aattaacatttttacatactctgtataaacgtgttcttcatatgttgctattattgatacaaactatggatggtcaggaactat
cattgagatattcttctaaagtttttttaaaggcgtttaacgagagaaaggacagtatcaaattcgtgaaattactatccca
cttttatccggcagttattaacagtaatattaatgttataaactattttaaccgcatgtttcactttttcgaacatgaaaagag
aactaactacgaatacgaaagaggaaatattataatttttcccctagcactgtattcggcagataaagtagataccgagct
agctatcaaattaggatttaaatctttggtacaatacataaagtttatctttttacagatggctctgttatacattaaaattta
cgaactaccatgctgcgacaactttttacacgcagatcttaaacccgataatatcttactttttgattccaatgaaccaata
ataattcatctaaaggataaaaagtttgtttttaatgaacgtattaaatcggcattaaacgactttgacttttcccaag
8.1 Description for nucleotide sequences of low GC content version of MVA-SIi-HBV-PreS-Pmut-C-S(sh):
   8.1.1 F11-L-Flank = bases 1 - 1097 (SEQ ID NO: 35)
   8.1.2 SIi-HBV-PreS-Pmut-C = bases 1098 - 4838 (SEQ ID NO: 59)
   8.1.3 Transcription terminator sequence = bases 4839 - 4845
      TTTTTGT
   8.1.4 mH5 promoter = bases 4846 - 4942 (SEQ ID NO: 28)
   8.1.5 TPA = bases 4943 - 5038 (SEQ ID NO: 60)
   8.1.6 S(sh) = bases 5039 - 5920 (SEQ ID NO: 61)
   8.1.7 F11-R-Flank = bases 5921 - 7239 (SEQ ID NO: 36)

The Sequence of wild-type HBV Polymerase is provided as SEQ ID NO: 19.
ChAdOx1 sequence
   ChAdOx1 sequence 5' to the immunogen cassette is provided as SEQ ID NO: 39.
   ChAdOx1 sequence 3' to the immunogen cassette is provided as SEQ ID NO: 40.
ChAdOx2 sequence
   ChAdOx2 sequence 5' to the immunogen cassette is provided as SEQ ID NO: 41.
   ChAdOx2 sequence 3' to the immunogen cassette is provided as SEQ ID NO: 42.
MVA sequence
   MVA sequence 5' to the immunogen cassette is provided as SEQ ID NO: 44. MVA sequence 3' to the immunogen cassette is provided as SEQ ID NO: 45

The CMV long promoter with Tetron Operator sequence is provided as SEQ ID NO: 50. The CMV short promoter with Tetron Operator sequence is provided as SEQ ID NO: 51.
PreS1 sequence (SEQ ID NO: 52): 
MGGWSSKPRQGMGTNLSVPNPLGFFPDHQLDPAFGANSNNPDWDFNPNKDHWP
EANQVGAGAFGPGFTPPHGGLLGWSPQAQGILTTVPAAPPPASTNRQSGRQPTPI
SPPLRDSHPQA
PreS2 sequence (SEQ ID NO: 53):
   MQWNSTTFHQALLDPRVRGLYFPAGGSSSGTVNPVPTTASPISSIFSRTGDPAPN

## Claims

1. A multi-HBV immunogen viral vector vaccine comprising:
a viral vector comprising an immunogen expression cassette, wherein the expression of a protein encoded by the expression cassette is arranged to be driven by a promoter, wherein the immunogen expression cassette encodes:
a) HBV Core;
b) a modified HBV polymerase (Pₘᵤₜ), wherein the modification is a mutation to wild-type HBV polymerase to substantially remove polymerase function;
c) HBV surface antigen (HbsAg); and
d) an intergenic sequence that is arranged to cause expression of at least the HBV surface antigen (HbsAg) as a separate protein from the HBV core and the modified HBV polymerase (Pₘᵤₜ),
wherein the intergenic sequence is downstream (3') of the sequences encoding the HBV core and the modified HBV polymerase (Pₘᵤₜ) and upstream (5') of the sequence encoding the HBV surface antigen (HbsAg).

2. The multi-HBV immunogen viral vector vaccine according to claim 1, wherein the intergenic sequence comprises a cleavage domain, an IRES (Internal Ribosomal Entry Site), a splicing signal, or a secondary promoter; or wherein the intergenic sequence comprises a cleavage domain;
optionally wherein the cleavage domain comprises a ribosome skipping cleavage domain;
further optionally, wherein the cleavage domain comprises or consists of Furin-2A (F2A) peptide sequence or a functional variant thereof; or
wherein the intergenic sequence comprises a secondary promoter to promote expression of at least the surface antigen (HbsAg).

3. The multi-HBV immunogen viral vector vaccine according to any preceding claim, wherein the immunogen expression cassette further encodes HBV Pre-Core (PreC); and/or wherein the immunogen expression cassette further encodes HBV PreS1, and/or a truncated form thereof.

4. The multi-HBV immunogen viral vector vaccine according to any preceding claim, wherein the immunogen expression cassette further encodes HBV PreS2.

5. The multi-HBV immunogen viral vector vaccine according to any preceding claim, wherein the immunogen expression cassette encodes HBV Pre-Core (PreC) and HBV PreS1, and a truncated form of PreS1.

6. The multi-HBV immunogen viral vector vaccine according to any preceding claim, wherein the HBV Core and modified polymerase (Pmut) are arranged to be expressed as a fusion protein.

7. The multi-HBV immunogen viral vector vaccine according to any preceding claim, wherein the immunogen expression cassette comprises nucleic acid comprising the sequence of:
SEQ ID NO: 46 (SIi-HBV-CPmutS) or a variant thereof;
SEQ ID NO: 47 (SIi-HBV-SCPmut) or a variant thereof;
SEQ ID NO: 48 (SIi-HBV-CPmutPreS-S(sh)) or a variant thereof;
SEQ ID NO: 49 (SIi-HBV-CPmutPreS-TPA-S(sh)) or a variant thereof; SEQ ID NO: 24 (MVA-SIi-HBV-PreS-Pmut-C-S(sh)) or a variant thereof; or
SEQ ID NO: 27 or SEQ ID NO: 58 (MVA-SIi-HBV-PreS-Pmut-C-TPA-S(sh)) or a variant thereof; and/or
wherein the viral vector encodes the amino acid sequence of:
SEQ ID NO: 3 (SIi-HBV-CPmutS) or a variant thereof.
SEQ ID NO: 11 (SIi-HBV-SCPmut) or a variant thereof.
SEQ ID NO: 13 (SIi-HBV-CPmutPreS-S(sh)) or a variant thereof.
SEQ ID NO: 25 (SIi-HBV-CPmutPreS-TPA-S(sh)) or a variant thereof.
SEQ ID NO: 23 (MVA-SIi-HBV-PreS-Pmut-C-S(sh)) or a variant thereof.
SEQ ID NO: 26 (MVA-SIi-HBV-PreS-Pmut-C-TPA-S(sh)) or a variant thereof.

8. The multi-HBV immunogen viral vector vaccine according to any preceding claim, wherein the modified HBV polymerase (Pₘᵤₜ) is not a truncated form of HBV polymerase; and/or
wherein the modified HBV polymerase (Pₘᵤₜ) comprises or consists of the sequence of SEQ ID NO: 8 or a variant thereof; and/or wherein the HbsAg comprises or consists of a full length wild-type HbsAg sequence, or a variant thereof.

9. The multi-HBV immunogen viral vector vaccine according to any preceding claim, wherein the immunogen expression cassette encodes a truncated form of HBV PreS1 and the truncated PreS1 is arranged to be expressed a fusion protein with the HBV surface antigen (S / HbsAg); and optionally further comprising a linker sequence provided between the truncated PreS1 and surface antigen (S / HbsAg); and/or wherein the truncated PreS1 with a fused surface antigen is encoded downstream (3') of the intergenic sequence.

10. The multi-HBV immunogen viral vector vaccine according to any preceding claim, wherein the expression cassette encodes a NΔPreS1 and PreS2 fusion sequence; and optionally wherein the encoded NΔPreS1 and PreS2 fusion sequence is encoded upstream (5') of the intergenic sequence; and further optionally wherein the encoded NΔPreS1 and PreS2 fusion sequence is further fused with the modified polymerase (Pmut); and further optionally wherein a linker sequence is provided between the PreS2 and modified polymerase (Pmut).

11. The multi-HBV immunogen viral vector vaccine according to any preceding claim, wherein the immunogen expression cassette further encodes a peptide adjuvant; optionally wherein the peptide adjuvant comprises TPA (tissue plasminogen activator) or a human or non-human invariant chain (Ii), or a fragment thereof.

12. A nucleic acid comprising or consisting of an HBV immunogen expression cassette, wherein the immunogen expression cassette encodes:
a) HBV Core;
b) a modified HBV polymerase (Pₘᵤₜ), wherein the modification is a mutation to wild-type HBV polymerase to substantially remove polymerase function;
c) HBV surface antigen (HbsAg); and
d) an intergenic sequence that is arranged to cause expression of at least the HBV surface antigen (HbsAg) as a separate protein from the HBV core and the modified HBV polymerase (Pₘᵤₜ),
wherein the intergenic sequence is downstream (3') of the sequences encoding the HBV core and the modified HBV polymerase (Pₘᵤₜ) and upstream (5') of the sequence encoding the HBV surface antigen (HbsAg).

13. A composition comprising the viral vector according to any of claims 1 to 11 or the nucleic acid according to claim 12, optionally wherein the composition is a pharmaceutically acceptable composition.

14. The composition according to claim 13, the viral vector according to any of claims 1 to 11 or the nucleic acid according to any of claim 12, for use in the prophylaxis or treatment of HBV infection in a subject; optionally wherein the use is as a vaccine.

15. A prime boost vaccination kit comprising
- a prime vaccination comprising the composition according to claim 13, the viral vector according to any of claims 1 to 11 or the nucleic acid according to claim 12; and
- a boost vaccination comprising the composition according to claim 13, the viral vector according to any of claims 1 to 11 or the nucleic acid according to claim 12.

## Patentansprüche

1. Multi-HBV-Immunogenimpfstoff aus viralem Vektor, umfassend:
einen viralen Vektor, der eine Immunogenexpressionskassette umfasst, wobei die Expression eines Proteins, das durch die Expressionskassette codiert wird, ausgelegt ist, um von einem Promotor angesteuert zu werden, wobei die Immunogenexpressionskassette codiert für:
a)HBV-Core;
b) eine modifizierte HBV-Polymerase (Pₘᵤₜ), wobei die Modifikation eine Mutation zur HBV-Wildtyp-Polymerase ist, um die Polymerasefunktion im Wesentlichen zu entfernen;
c)HBV-Oberflächenantigen (HbsAg); und
d) eine intergene Sequenz, die ausgelegt ist, um zumindest eine Expression von zumindest des HBV-Oberflächenantigens (HbsAg) als separates Protein aus dem HBV-Core und der modifizierten HBV-Polymerase (Pₘᵤₜ) zu veranlassen;
wobei die intergene Sequenz den Sequenzen, die für den HBV-Core und die modifizierte HBV-Polymerase (Pₘᵤₜ) codieren, vorgeschaltet (3') und der Sequenz, die für das HBV-Oberflächenantigen (HbsAg) codiert, nachgeschaltet ist.

2. Multi-HBV-Immunogenimpfstoff aus viralem Vektor nach Anspruch 1, wobei die intergene Sequenz eine Spaltdomäne, eine IRES (interne ribosomale Eintrittsstelle), ein Spleißsignal oder einen sekundären Promotor umfasst; oder wobei die intergene Sequenz eine Spaltdomäne umfasst;
wobei wahlweise die Spaltdomäne eine Ribosom-Skipping-Spaltdomäne umfasst,
wobei ferner wahlweise die Spaltdomäne Furin-2A-Peptidsequenz (F2A-Peptidsequenz) oder eine funktionelle Variante davon umfasst oder daraus besteht; oder
wobei die intergene Sequenz einen sekundären Promotor umfasst, um die Expression zumindest des Oberflächenantigens (HbsAg) zu fördern.

3. Multi-HBV-Immunogenimpfstoff aus viralem Vektor nach einem vorstehenden Anspruch, wobei die Immunogenexpressionskassette ferner für HBV-Pre-Core (PreC) codiert; und/oder wobei die Immunogenexpressionskassette ferner für HBV-Pres1 und/oder eine gekürzte Form davon codiert.

4. Multi-HBV-Immunogenimpfstoff aus viralem Vektor nach einem vorstehenden Anspruch, wobei die Immunogenexpressionskassette ferner für HBV PreS2 codiert.

5. Multi-HBV-Immunogenimpfstoff aus viralem Vektor nach einem vorstehenden Anspruch, wobei die Immunogenexpressionskassette für HBV-Pre-Core (PreC) und HBV-PreS1 und eine gekürzte Form von PreS1 codiert.

6. Multi-HBV-Immunogenimpfstoff aus viralem Vektor nach einem vorstehenden Anspruch, wobei der HBV-Core und die modifizierte Polymerase (Pmut) ausgelegt sind, um als Fusionsprotein exprimiert zu werden.

7. Multi-HBV-Immunogenimpfstoff aus viralem Vektor nach einem vorstehenden Anspruch, wobei die Immunogenexpressionskassette eine Nukleinsäure umfasst, die die Sequenz umfasst von:
SEQ ID NO: 46 (SIi-HBV-CPmutS) oder eine Variante davon;
SEQ ID NO: 47 (SIi-HBV-SCPmut) oder eine Variante davon;
SEQ ID NO: 48 (SIi-HBV-CPmutPreS-S(sh)) oder eine Variante davon;
SEQ ID NO: 49 (SIi-HBV-CPmutPreS-TPA-S(sh)) oder eine Variante davon; SEQ ID NO: 24 (MVA-SIi-HBV-PreS-Pmut-C-S(sh)) oder eine Variante davon; oder
SEQ ID NO: 27 oder SEQ ID NO: 58 (MVA-SIi-HBV-PreS-Pmut-C-TPA-S(sh)) oder eine Variante davon; oder
wobei der virale Vektor für die Aminosäuresequenz codiert von:
SEQ ID NO: 3 (SIi-HBV-CPmutS) oder eine Variante davon.
SEQ ID NO: 11 (SIi-HBV-SCPmut) oder eine Variante davon.
SEQ ID NO: 13 (SIi-HBV-CPmutPreS-S(sh)) oder eine Variante davon.
SEQ ID NO: 25 (SIi-HBV-CPmutPreS-TPA-S(sh)) oder eine Variante davon.
SEQ ID NO: 23 (MVA-SIi-HBV-PreS-Pmut-C-S(sh)) oder eine Variante davon.
SEQ ID NO: 26 (MVA-SIi-HBV-PreS-Pmut-C-TPA-S(sh)) oder eine Variante davon.

8. Multi-HBV-Immunogenimpfstoff aus viralem Vektor nach einem vorstehenden Anspruch, wobei die modifizierte HBV-Polymerase (Pₘᵤₜ) keine gekürzte Form der HBV-Polymerase ist; und/oder
wobei die modifizierte HBV-Polymerase (Pₘᵤₜ) die Sequenz von SEQ ID NO: 8 oder eine Variante davon umfasst oder daraus besteht; und/oder wobei das HbsAg eine Wildtyp-HbsAg-Sequenz voller Länge oder eine Variante davon umfasst oder daraus besteht.

9. Multi-HBV-Immunogenimpfstoff aus viralem Vektor nach einem der vorstehenden Anspruch, wobei die Immunogenexpressionskassette für eine gekürzte Form von HBV-Pres1 codiert und das gekürzte PreS1 ausgelegt ist, um als Fusionsprotein mit dem HBV-Oberflächenantigen (S/HbsAg) exprimiert zu werden; und ferner wahlweise umfassend eine Linkersequenz zwischen dem gekürzten PreS1 und dem Oberflächenantigen (S/HbsAg); und/oder wobei das gekürzte PreS1 mit einem fusionierten Oberflächenantigen der intergenen Sequenz nachgeschaltet (3') codiert wird.

10. Multi-HBV-Immunogenimpfstoff aus viralem Vektor nach einem vorstehenden Anspruch, wobei die Expressionskassette für eine NΔPreS1- und PreS2-Fusionssequenz codiert; und wahlweise wobei die codierte NΔPreS1- und PreS2-Fusionssequenz der intergenen Sequenz vorgeschaltet (5') codiert wird; und ferner wahlweise wobei die codierte NΔPreS1- und PreS2-Fusionssequenz ferner mit der modifizierten Polymerase (Pmut) fusioniert ist; und ferner wahlweise wobei eine Linkersequenz zwischen dem PreS2 und der modifizierten Polymerase (Pmut) bereitgestellt ist.

11. Multi-HBV-Immunogenimpfstoff aus viralem Vektor nach einem vorstehenden Anspruch, wobei die Immunogenexpressionskassette ferner für ein Peptidadjuvans codiert; wahlweise wobei das Peptidadjuvans TPA (Gewebeplasminogenaktivator) oder eine humane oder nicht humane invariante Kette (Ii) oder ein Fragment davon umfasst.

12. Nukleinsäure, die eine HBV-Immunogenexpressionskassette umfasst oder daraus besteht, wobei die Immunogenexpressionskassette codiert für:
a)HBV-Core;
b) eine modifizierte HBV-Polymerase (Pₘᵤₜ), wobei die Modifikation eine Mutation zur HBV-Wildtyp-Polymerase ist, um die Polymerasefunktion im Wesentlichen zu entfernen;
c)HBV-Oberflächenantigen (HbsAg); und
d)eine intergene Sequenz, die ausgelegt ist, um zumindest eine Expression von zumindest des HBV-Oberflächenantigens (HbsAg) als separates Protein aus dem HBV-Core und der modifizierten HBV-Polymerase (Pₘᵤₜ) zu veranlassen;
wobei die intergene Sequenz den Sequenzen, die für den HBV-Core und die modifizierte HBV-Polymerase (Pₘᵤₜ) codieren, vorgeschaltet (3') und der Sequenz, die für das HBV-Oberflächenantigen (HbsAg) codiert, nachgeschaltet ist.

13. Zusammensetzung, die den viralen Vektor nach einem der Ansprüche 1 bis 11 oder die Nukleinsäure nach Anspruch 12 umfasst, wahlweise wobei die Zusammensetzung eine pharmazeutisch unbedenkliche Zusammensetzung ist.

14. Zusammensetzung nach Anspruch 13, viraler Vektor nach einem der Ansprüche 1 bis 11 oder Nukleinsäure nach einem der Ansprüche 12 zur Verwendung bei der Prophylaxe oder Behandlung einer HBV-Infektion bei einem Individuum; wahlweise wobei die Verwendung als Impfstoff erfolgt.

15. Kit für Grundimmunisierungs- und Auffrischimpfung, das umfasst:
- eine Grundimmunisierungsimpfung, die die Zusammensetzung nach Anspruch 13, den viralen Vektor nach einem der Ansprüche 1 bis 11 oder die Nukleinsäure nach Anspruch 12 umfasst; und
- eine Auffrischungsimpfung, die die Zusammensetzung nach Anspruch 13, den viralen Vektor nach einem der Ansprüche 1 bis 11 oder die Nukleinsäure nach Anspruch 12 umfasst.

## Revendications

1. Vaccin à vecteur viral multi-immunogène contre le VHB comprenant :
un vecteur viral comprenant une cassette d'expression d'immunogène, dans lequel l'expression d'une protéine codée par la cassette d'expression est conçue pour être pilotée par un promoteur, dans lequel la cassette d'expression d'immunogène code :
a) un noyau du VHB ;
b) une polymérase du VHB modifiée (Pₘᵤₜ), dans lequel la modification est une mutation de la polymérase du VHB de type sauvage pour supprimer substantiellement la fonction polymérase ;
c) un antigène de surface du VHB (HbsAg) ; et
d) une séquence intergénique conçue pour provoquer l'expression d'au moins l'antigène de surface du VHB (HbsAg) en tant que protéine distincte du noyau du VHB et de la polymérase du VHB modifiée (Pₘᵤₜ),
dans lequel la séquence intergénique est en aval (3') des séquences codant pour le noyau du VHB et la polymérase du VHB modifiée (Pₘᵤₜ) et en amont (5') de la séquence codant pour l'antigène de surface du VHB (HbsAg).

2. Vaccin à vecteur viral multi-immunogène contre le VHB selon la revendication 1, dans lequel la séquence intergénique comprend un domaine de clivage, un IRES (site d'entrée ribosomique interne), un signal d'épissage ou un promoteur secondaire ; ou dans lequel la séquence intergénique comprend un domaine de clivage ;
éventuellement dans lequel le domaine de clivage comprend un domaine de clivage sautant le ribosome ;
en outre, éventuellement, dans lequel le domaine de clivage comprend ou est constitué d'une séquence peptidique de la furine-2A (F2A) ou d'une variante fonctionnelle de celle-ci ; ou
dans lequel la séquence intergénique comprend un promoteur secondaire pour favoriser l'expression d'au moins l'antigène de surface (HbsAg).

3. Vaccin à vecteur viral multi-immunogène contre le VHB selon l'une quelconque des revendications précédentes, dans lequel la cassette d'expression d'immunogène code en outre le pré-noyau du VHB (PreC) ; et/ou dans lequel la cassette d'expression d'immunogène code en outre le pré-noyau du VHB, et/ou une forme tronquée de celui-ci.

4. Vaccin à vecteur viral multi-immunogène contre le VHB selon l'une quelconque des revendications précédentes, dans lequel la cassette d'expression immunogène code en outre pour HBV PreS2.

5. Vaccin à vecteur viral multi-immunogène contre le VHB selon l'une quelconque des revendications précédentes, dans lequel la cassette d'expression immunogène code pour le pré-noyau du HBV (PreC) et HBV PreS1, et une forme tronquée de PreS1.

6. Vaccin à vecteur viral multi-immunogène contre le VHB selon l'une quelconque des revendications précédentes, dans lequel le noyau HBV et la polymérase modifiée (Pₘᵤₜ) sont disposés pour être exprimés sous la forme d'une protéine de fusion.

7. Vaccin à vecteur viral multi-immunogène contre le VHB selon l'une quelconque des revendications précédentes, dans lequel la cassette d'expression de l'immunogène comprend un acide nucléique comprenant la séquence de :
SEQ ID NO : 46 (SIi-HBV-CPmutS) ou une variante de celle-ci ;
SEQ ID NO : 47 (SIi-HBV-SCPmut) ou une variante de celle-ci ;
SEQ ID NO : 48 (SIi-HBV-CPmutPreS-S(sh)) ou une variante de celle-ci ;
SEQ ID NO : 49 (Sh -HBV-CPmutPreS-TPA-S(sh)) ou une variante de celle-ci ;
SEQ ID NO : 24 (MVA-SIi-HBV-PreS-Pmut-CS(sh)) ou une variante de celle-ci ; ou
SEQ ID NO : 27 ou SEQ ID NO : 58 (MVA-SIi-HB V-PreS-Pmut-C-TPA-S(sh)) ou une variante de celle-ci ; et/ou
dans lequel le vecteur viral code la séquence d'acides aminés de :
SEQ ID NO : 3 (SIi-HBV-CPmutS) ou une variante de celle-ci ;
SEQ ID NO : 11 (SIi-HBV-SCPmut) ou une variante de celle-ci ;
SEQ ID NO : 13 (SIi-HBV-CPmutPreS-S(sh)) ou une variante de celle-ci ;
SEQ ID NO : 25 (SIi-HBV-CPmutPreS-TPA-S(sh)) ou une variante de celle-ci ;
SEQ ID NO : 23 (MVA-SIi-HBV-PreS-Pmut-C-S(sh)) ou une variante de celle-ci ;
SEQ ID NO : 26 (MVA-SIi-HBV- PreS-Pmut-C-TPA-S(sh)) ou une variante de celle-ci.

8. Vaccin à vecteur viral multi-immunogène contre le VHB selon l'une quelconque des revendications précédentes, dans lequel la polymérase du VHB modifiée (Pₘᵤₜ) **n'est** pas une forme tronquée de la polymérase du VHB ; et/ou
dans lequel la polymérase du VHB modifiée (Pₘᵤₜ) comprend ou est constituée de la séquence de SEQ ID NO : 8 ou d'une variante de celle-ci ; et/ou dans lequel le HbsAg comprend ou est constitué d'une séquence HbsAg de type sauvage pleine longueur, ou d'une variante de celle-ci.

9. Vaccin à vecteur viral multi-immunogène contre le VHB selon l'une quelconque des revendications précédentes, dans lequel la cassette d'expression immunogène code une forme tronquée de PreS1 du VHB et la PreS1 tronquée est agencée pour être exprimée en tant que protéine de fusion avec l'antigène de surface du VHB (S/HbsAg) ; et comprenant éventuellement en outre une séquence de liaison prévue entre la PreSl tronquée et l'antigène de surface (S/HbsAg) ; et/ou dans lequel la PreS1 tronquée avec un antigène de surface fusionné est codée en aval (3') de la séquence intergénique.

10. Vaccin à vecteur viral multi-immunogène contre le VHB selon l'une quelconque des revendications précédentes, dans lequel la cassette d'expression code une séquence de fusion NΔPreS1 et PreS2 ; et éventuellement dans lequel la séquence de fusion NΔPreS1 et PreS2 codée est codée en amont (5') de la séquence intergénique ; et éventuellement dans lequel la séquence de fusion NΔPreS1 et PreS2 codée est en outre fusionnée avec la polymérase modifiée (Pₘᵤₜ) ; et éventuellement dans lequel une séquence de liaison est fournie entre la PreS2 et la polymérase modifiée (Pₘᵤₜ).

11. Vaccin à vecteur viral multi-immunogène contre le VHB selon l'une quelconque des revendications précédentes, dans lequel la cassette d'expression immunogène code en outre un adjuvant peptidique ; éventuellement dans lequel l'adjuvant peptidique comprend un TPA (activateur tissulaire du plasminogène) ou une chaîne invariante humaine ou non humaine (Ii), ou un fragment de celle-ci.

12. Acide nucléique comprenant ou constitué d'une cassette d'expression d'immunogène du VHB, dans lequel la cassette d'expression d'immunogène code :
a) le noyau du VHB ;
b) une polymérase du VHB modifiée (Pₘᵤₜ), dans laquelle la modification est une mutation de la polymérase du VHB de type sauvage pour supprimer sensiblement la fonction polymérase ;
c) l'antigène de surface du VHB (HbsAg) ; et
d) une séquence intergénique qui est agencée pour provoquer l'expression d'au moins l'antigène de surface du VHB (HbsAg) en tant que protéine distincte du noyau du VHB et de la polymérase du VHB modifiée (Pₘᵤₜ),
dans lequel la séquence intergénique est en aval (3') des séquences codant pour le noyau du VHB et la polymérase du VHB modifiée (Pₘᵤₜ) et en amont (5') de la séquence codant pour l'antigène de surface du VHB (HbsAg).

13. Composition comprenant le vecteur viral selon l'une quelconque des revendications 1 à 11 ou l'acide nucléique selon la revendication 12, éventuellement dans laquelle la composition est une composition pharmaceutiquement acceptable.

14. Composition selon la revendication 13, vecteur viral selon l'une quelconque des revendications 1 à 11 ou acide nucléique selon l'une quelconque des revendications 12, destinés à être utilisés dans la prophylaxie ou le traitement d'une infection par le VHB chez un sujet ; éventuellement dans lequel l'utilisation est en tant que vaccin.

15. Kit de vaccination Prime Boost comprenant
- une primovaccination comprenant la composition selon la revendication 13, le vecteur viral selon l'une quelconque des revendications 1 à 11 ou l'acide nucléique selon la revendication 12 ; et
- une vaccination de rappel comprenant la composition selon la revendication 13, le vecteur viral selon l'une quelconque des revendications 1 à 11 ou l'acide nucléique selon la revendication 12.
